# EUROPEAN PATENT SPECIFICATION

(11) **EP 1 581 249 B1**
(45) Date of publication and mention of the grant of the patent: **02.12.2009**
(21) Application number: 03799727.7
(22) Date of filing: 31.12.2003
(51) Int. Cl.: A61K 38/19, G01N 33/50, C12N 5/06, A61P 25/28

(54) **G-CSF FOR USE IN THE TREATMENT OF AMYOTROPHIC LATERAL SCLEROSIS**
G-CSF ZUR BEHANDLUNG VON AMYOTROPHER LATERALSKLEROSE
G-CSF POUR SON UTILISATION DANS LE TRAITEMENT DE LA SCLEROSE LATERALE AMYOTROPHIQUE

(30) Priority: 31.12.2002 US 331755; 11.09.2003 US 659295
(43) Date of publication of application: 05.10.2005
(60) Divisional application: 09011942.1
(73) Proprietor: Sygnis Bioscience GmbH & Co. KG, 69120 Heidelberg (DE)
(72) Inventor: SCHAEBITZ, Wolf-Ruediger, 69221 Dossenheim (DE); SCHNEIDER, Armin, 69118 Heidelberg (DE); KRUEGER, Carola, 67346 Speyer (DE); SOMMER, Clemens, 89312 Guenzburg (DE); SCHWAB, Stefan, 69121 Heidelberg (DE); KOLLMAR, Rainer, 69121 Heidelberg (DE); MAURER, Martin, 69121 Heildelberg (DE); WEBER, Daniela, 68159 Mannheim (DE); GASSLER, Nikolaus, 69120 Heidelberg (DE)
(74) Representative: Isenbruck, Günter
(86) International application number: PCT/IB2003/006446
(87) International publication number: WO 2004/058287

(56) References cited:
- EP-A- 1 374 898
- WO-A-02/099081
- WO-A-03/061685
- DE-A- 10 033 219
- US-A1- 2002 151 488
- SCHAEBITZ W R ET AL: "RECOMBINANT GRANULOCYTE-COLONY STIMULATING FACTOR (RG-CSF) IS NEUROPROTECTIVE FOLLOWING FOCAL TRANSIENT CEREBRAL ISCHEMIA AND EXCITOTOXITY" SOCIETY FOR NEUROSCIENCE ABSTRACTS, SOCIETY FOR NEUROSCIENCE, US, vol. 27, no. PART 2, 10 November 2001 (2001-11-10), page 2027,AN76411, XP008009334 ISSN: 0190-5295
- KONISHI Y ET AL: "Trophic effect of erythropoietin and other hematopoietic factors on central cholinergic neurons in vitro and in vivo." BRAIN RESEARCH. 23 APR 1993, vol. 609, no. 1-2, 23 April 1993 (1993-04-23), pages 29-35, XP001183386 ISSN: 0006-8993
- HIERHOLZER C ET AL: "Activation of STAT proteins following ischemia reperfusion injury demonstrates a distinct IL-6 and G-CSF mediated profile." TRANSPLANTATION PROCEEDINGS. SEP 1998, vol. 30, no. 6, September 1998 (1998-09), page 2647, XP001182894 ISSN: 0041-1345
- TIAN SHIN-SHAY ET AL: "Multiple signaling pathways induced by granulocyte colony-stimulating factor involving activation of JAKs, STAT5, and/or STAT3 are required for regulation of three distinct classes of immediate early genes" BLOOD, vol. 88, no. 12, 1996, pages 4435-4444, XP001183387 ISSN: 0006-4971
- WARD ALISTER C ET AL: "Tyrosine-dependent and -independent mechanisms of STAT3 activation by the human granulocyte colony-stimulating factor (G-CSF) receptor are differentially utilized depending on G-CSF concentration" BLOOD, vol. 93, no. 1, 1 January 1999 (1999-01-01), pages 113-124, XP001183113 ISSN: 0006-4971
- SCHAEBITZ W -R ET AL: "Neuroprotective effect of granulocyte colony-stimulating factor after focal cerebral ischemia." STROKE, vol. 34, no. 3, March 2003 (2003-03), pages 745-751, XP002294803 ISSN: 0039-2499

## Description

### BACKGROUND OF THE INVENTION

### Field of the Invention

The present invention relates to the treatment of Amyotrophic lateral sclerosis by means of G-CSF of certain variants thereof.

### Discussion of the Related Art

Growth factors are proteins that are essentially involved in regulating survival, proliferation, maturation, and outgrowth of developing neuronal cells. For example, the expression of a large number of growth factors increases in response to various brain insults. Many factors display endogenous neuroprotective and neurotrophic effects (see Arvidsson A et al., Neuroscience 2001;106:27-41; Larsson E, et al., J Cereb Blood Flow Metab 1999;19:1220-8; Mattson MP, et al., J Neurotrauma 1994;11:3-33; Semkova I, et al., Brain Res Brain Res Rev 1999;30:176-88). These effects were also reported after exogenous administration *in vitro* and *in vivo* after brain trauma and stroke (see Semkova I., et al., Brain Res. Rev. 1999; 30:176-88; Fisher M, et al., J. Cereb. Blood Flow Metab. 1995; 15:953-9; Schäbitz WR, et al., Stroke 2001; 32:1226-33; Schäbitz WR, et al., Stroke 2000; 31:2212-7). After binding to high-affinity membrane receptors the effects of growth factors are mediated by a cascade of intracellular signal-transduction events (Kernie SG, et al., Arch Neurol 2000; 57:654-7), which induces cells to grow and differentiate; or provides trophic support for cell survival.

Granulocyte-colony stimulating factor (GCSF), a 20 kDa protein, together with tumor necrosis factor-α (TNF-α) and the interleukins is a member of the cytokine family of growth factors. GCSF is the major growth factor involved in the production of neutrophilic granulocytes.

GCSF exerts its function via the activation of a membrane receptor (GCSF receptor) that belongs to the super-family of hematopoietin receptors, also being referred to as class I cytokine receptors (de Koning and Touw, Curr. Opin. Hematol., 1996, 3, 180-4).

A number of receptors for lymphokines, hematopoetic growth factors, and growth hormone-related molecules have been found to share a common binding domain. These receptors are referred to as hematopoietin receptors and the corresponding ligands as hematopoietins. Further, hematopoietins have been subdivided into two major structural groups: Large/long and small/short hematopoietins. One subset of individual receptor chains that are part of receptor complexes for large hematopoietins contain common structural elements in their extracellular parts: an immunoglobin-like domain, a hematopoietin-receptor domain, and 3 fibronectin type-III domains (2 in the leptin receptor). This subgroup was designated the "gp130 family of receptors" *(*Mosley, et al., J. Biol Chem.1996, 271, 32635-43) and include Leptin receptor (LPTR), Granulocyte colony stimulating factor receptor (GCSFR), Interleukin-6/-11/LIF/OSM/CNTF common beta chain (GP130), Leukemia inhibiting factor receptor (LIFR), Oncostatin-M receptor beta chain (OSMR), Interleukin-12 receptor beta-1 chain (IL12RB1), Interleukin-12 receptor beta-2 chain (IL12RB2). These receptor chains homodimerize (GCSFR, GP130, LPTR) or heterodimerize (GP130 with LIFR or OSMR, IL12RB1 with IL12RB2) upon binding the cognate cytokine. In addition, a prosite consensus pattern is characteristic of this receptor family, which is:
N-x(4)-S-x(28,35)-[LVIM]-x-W-x(0,3)-P-x(5,9)-[YF]-x(1,2)-[VILM]-x-W (SEQ ID NO:1)

GCSF stimulates proliferation, survival, and maturation of cells committed to the neutrophilic granulocyte lineage through binding to the specific GCSF receptor (GCSFR) (see Hartung T., et al., Curr. Opin. Hematol. 1998;5:221-5). GCSFR mediated signaling activates the family of Signal Transducer and Activator of Transcription (STAT) proteins which translocate to the nucleus and regulate transcription (Darnell JE Jr., Science 1997; 277:1630-5). GCSF is typically used for the treatment of different kinds of neutropenia in humans. It is one of the few growth factors approved for clinical use. In particular, it is used to reduce chemotherapy (CT)-induced cytopenia (Viens et al., J. of Clin. Oncology, Vol. 20, No.1, 2002:24-36). GCSF has also been implicated for therapeutic use in infectious diseases as potential adjunctive agent (Hübel et al., J. of Infectious Diseases, Vol. 185:1490-501, 2002). GCSF has reportedly been crystallized to some extent (EP 344 796), and the overall structure of GCSF has been surmised, but only on a gross level (Bazan, Immunology Today 11: 350-354 (1990); Parry et al. J. Molecular Recognition 8: 107-110 (1988)).

In recent years a number of growth factors such as bFGF and pharmaceutically promising substances such as thrombocyte adhesion blockers like anti-GP IIb/IIa and Abcizimab have been tested for neuroprotective efficacy in clinical studies. Unfortunately, none of these prevailed to provide neuroprotective efficacy. In particular, NMDA antagonists, free radical scavengers and glutamate antagonists failed or demonstrated severe side-effects. The list of substances such as anti-ICAM or inhibitors of the glutamate-mediated NO-synthetase that have failed growing (De Keyser, et al. (1999), Trends Neurosci, 22, 535-40).

Most studies on cerebral ischemia and testing of pharmacological substances *in vivo* have only been concerned with the immediate effects of the drug or paradigm under investigation (i.e. infarct size 24h after induction of the stroke). However, a more valid parameter of true efficacy of a particular substance is the long-term effect on functional recovery, which is also reflected in human stroke studies, where clinical scales (e.g., scandinavian stroke scale, NIH scale, Barthel index) also reflect the ability to perform daily life activities. Recovery in the first few days after focal lesions may be due to resolution of edema or reperfusion of the ischemic penumbra. Much of the functional recovery after the acute phase is likely due to brain plasticity, with adjacent cortical areas of the brain taking over functions previously performed by the damaged regions (Chen R, Cohen LG, Hallett M. Neuroscience 2002;111(4):761-73). The two main mechanisms proposed to explain reorganization are unmasking of previously present but functionally inactive connections and growth of new connections such as collateral sprouting (Chen R, Cohen LG, Hallett M. 2002 Neuroscience 2002;111(4):761-73). Short term plastic changes are mediated by removing inhibition to excitatory synapses, which is likely due to reduced GABAergic inhibition (Kaas JH. Annu Rev Neurosci. 1991;14:137-67; Jones EG. Cereb Cortex. 1993 Sep-Oct;3(5):361-72.). Plasticity changes that occur over a longer time involve mechanisms in addition to the unmasking of latent synapses such as long-term potentiation (LTP), which requires NMDA receptor activation and increased intracellular calcium concentration (Hess and Donoghue, J Neurophysiol. 1994 71(6):2543-7). Long term changes also involve axonal regeneration and sprouting with alterations in synapse shape, number, size and type (Kaas JH. Annu Rev Neurosci. 1991;14:137-67., 3:).

Stroke is the third-leading cause of death, and the main cause of disability in the western world. It presents a large socioeconomic burden. The etiology can be either ischemic (in the majority of cases) or hemorraghic. The cause of ischemic stroke is often embolic, or thrombotic. So far, there is no effective treatment for the majority of stroke patients. The only clinically proven drugs so far are tissue plasminogen activator (TPA) and Aspirin. After massive cell death in the immediate infarct core due to lack of glucose and oxygen, the infarct area expands for days, owing to secondary mechanisms such as glutamate excitotoxicity, apoptotic mechanisms, and generation of free radicals.

Amyotrophic lateral sclerosis (ALS; Lou-Gehrig's disease; Charcot's disease) is a neurodegenerative disorder with an annual incidence of 0.4 to 1.76 per 100.000 population (Adams et al., Principles of Neurology, 6th ed., New York, pp 1090-1095). It is the most common form of motor neuron disease with typical manifestations of generalized fasciculations, progressive atrophy and weakness of the skeletal muscles, spasticity and pyramidal tract signs, dysarthria, dysphagia, and dyspnea. The pathology consists principally in loss of nerve cells in the anterior horn of the spinal cord and motor nuclei of the lower brainstem, but can also include the first order motor neurons in the cortex. Pathogenesis of this devastating disease is still largely unknown, although the role of superoxide-dismutase (SOD1) mutants in familial cases has been worked out quite well, which invokes an oxidative stress hypothesis. So far, more than 90 mutations in the SOD1 protein have been described, that can cause ALS (Cleveland and Rothstein (2001), Nat Rev Neurosci, 2, 806-19). Also, a role for neurofilaments in this disease was shown. Excitotoxicity, a mechanism evoked by an excess glutamate stimulation is also an important factor, exemplified by the beneficial role of Riluzole in human patients. Most convincingly shown in the SOD1 mutants, activation of caspases and apoptosis seems to be the common final pathway in ALS (Ishigaki, et al. (2002), J Neurochem, 82, 576-84., Li, et al. (2000), Science, 288, 335-9). Therefore, it seems that ALS also falls into the same general pathogenetic pattern that is also operative in other neurodegenerative diseases and stroke, e.g. glutamate involvement, oxidative stress, and programmed cell death.

Parkinson's disease is the most frequent movement disorder, with approximately 1 million patients in North America; about 1 percent of the population over the age of 65 years is affected. The core symptoms of the disease are rigor, tremor and akinesia (Adams et al., Principles of Neurology, 6th ed., New York, pp 1090-1095). The etiology of Parkinson's disease is not known. Nevertheless, a significant body of biochemical data from human brain autopsy studies and from animal models points to an ongoing process of oxidative stress in the substantia nigra, which could initiate dopaminergic neurodegeneration. Oxidative stress, as induced by the neurotoxins 6-hydroxydopamine and MPTP (N-methyl-4-phenyl-1,2,3,6-tetrahydropyridine), has been used in animal models to investigate the process of neurodegeneration. Although a symptomatic therapy exists (e.g. L-DOPA plus a decarboxylase inhibitor; bromocriptine, pergolide as dopamin agonists; and anticholinergic agents such as trihexyphenidyl (artane)), there is a clear need for a causative therapy, e.g. a neuroprotective therapy, that really halts the disease progress. These animal models have been used to test the efficacy of radical scavengers, iron chelators, dopamine agonists, nitric oxide synthase inhibitors and certain calcium channel antagonists. Apoptotic mechanisms are clearly operative in the animal models as well as in the patient (Mochizuki, et al. (2001), Proc. Natl. Acad Sci. USA, 98, 10918-23, Xu et al. (2002), Nat. Med, 8, 600-6, Viswanath, et al. (2001), J. Neurosci., 21, 9519-28, Hartmann, et al. (2002), Neurology, 58, 308-10). This pathophysiology with involvement of oxidative stress and apoptosis also places Parkinson's disease amongst the other neurodegenerative disorders and stroke.

Cerebral ischemia may result from a variety of causes that impair cerebral blood flow (CBF) and lead to deprivation of both oxygen and glucose. Traumatic brain injury (TBI), on the other hand, involves a primary mechanical impact that usually causes skull fracture and abruptly disrupts the brain parenchyma with shearing and tearing of blood vessels and brain tissue. This, in turn, triggers a cascade of events characterized by activation of molecular and cellular responses that lead to secondary injury. The evolution of such secondary damage is an active process in which many biochemical pathways are involved (Leker and Shohami (2002), Brain Res. Rev., 39, 55-73). Many similarities between the harmful pathways that lead to secondary cellular death in the penumbral ischemic zone and in the area exposed to secondary post-traumatic injury have been identified (e.g. excitotoxity by excess glutamate release, nitric oxide, reactive oxygen species, inflammation, and apoptosis *(*Leker and Shohami (2002), Brain Res. Rev., 39, 55-73)). In addition, early ischemic episodes are reported to occur after traumatic brain injury, adding a component of ischemia to the primary mechanical damage.

Cardiovascular disease is the major cause of death in western industrialized nations. In the United States, there are approximately 1 million deaths each year with nearly 50% of them being sudden and occurring outside the hospital (Zheng, et al. (2001), Circulation, 104, 2158-63). Cardio-pulmonary resuscitation (CPR) is attempted in 40-90 of 100,000 inhabitants annually, and restoration of spontaneous circulation (ROSC) is achieved in 25-50% of these patients. However, the hospital discharge rate following successful ROSC is only 2-10% (Bottiger, et al. (1999), Heart, 82, 674-9). Therefore, the vast majority of the cardiac arrest victims annually in the United States is not treated successfully. The major reason for the low survival rates after successful CPR, i.e., for postarrest in-hospital mortality, is persistent brain damage. Brain damage following cardiocirculatory arrest is related both to the short period of tolerance to hypoxic stress and to specific reperfusion disorders (Safar (1986), Circulation, 74, IV138-53, Hossmann (1993), Resuscitation, 26, 225-35). Initially, a higher number of patients can be stabilized hemodynamically after cardiocirculatory arrest; many of them, however, die due to central nervous system injury. The personal, social, and economic consequences of brain damage following cardiac arrest are devastating. One of the most important issues in cardiac arrest and resuscitation ("whole body ischemia and reperfusion") research, therefore, is cerebral resuscitation and postarrest cerebral damage (Safar (1986), Circulation, 74, IV138-53, Safar, et al. (2002), Crit Care Med, 30, p. 140-4). Presently, it is not possible to decrease the primary damage to neurons that is caused by hypoxia during cardiac arrest by any post-arrest therapeutic measures. Major pathophysiological issues include hypoxia and subsequent necrosis, reperfusion injury with free radical formation and cellular calcium influx, release of excitatory amino acids, cerebral microcirculatory reperfusion disorders, and programmed neuronal death or apoptosis (Safar (1986), Circulation, 74, IV138-53, Safar et al. (2002), Crit Care Med, 30, 140-4).

Several clinical trials have attempted to improve neurological outcome after cardiac arrest without success. The therapeutic use of barbiturates (to enhance neuroprotection) or the use of calcium channel blockers (to reduce ischemia reperfusion damage) was tested (Group (1986), Am. J. Emerg. Med, 4, 72-86, Group (1986), N. Engl. J. Med, 314, 397-403, Group (1991), Control Clin. Trials, 12, 525-45, Group (1991), N. Engl. J. Med, 324, 1225-31). To date no specific post-arrest treatment options are available to improve neurological outcome following cardiocirculatory arrest in the clinical setting (with the possible exception of mild hypothermia and thrombolysis where the results of large, randomized, and controlled clinical trials are eagerly awaited (Safar et al (2002), Crit. Care Med, 30, 140-4)). Therefore, an innovative therapy to improve neurological outcome after cardiac arrest is crucial.

Multiple sclerosis is the prototype inflammatory autoimmune disorder of the central nervous system and, with a lifetime risk of one in 400, potentially the most common cause of neurological disability in young adults. Worldwide, there are about 2 - 5 million patients suffering from this disease (Compston and Coles (2002), Lancet, 359, 1221-31. ). As with all complex traits, the disorder results from interplay between as yet unidentified environmental factors and susceptibility genes. Together, these factors trigger a cascade of events, involving engagement of the immune system, acute inflammatory injury of axons and glia, recovery of function and structural repair, post-inflammatory gliosis, and neurodegeneration. The sequential involvement of these processes underlies the clinical course characterized by episodes with recovery, episodes leaving persistent deficits, and secondary progression. The aim of treatment is to reduce the frequency, and limit the lasting effects of relapses, relieve symptoms, prevent disability arising from disease progression, and promote tissue repair.

Depression is a common mental disorder characterized by sadness, loss of interest in activities and by decreased energy. Depression is differentiated from normal mood changes by the extent of its severity, the symptoms and the duration of the disorder. Suicide remains one of the common and often unavoidable outcomes of depression. If depressive episodes alternate with exaggerated elation or irritability they are known as bipolar disorder. Depressive disorders and schizophrenia are responsible for 60% of all suicides. The causes of depression can vary. Psychosocial factors, such as adverse living conditions, can influence the onset and persistence of depressive episodes. Genetic and biological factors can also play a part.

An estimated 121 million people currently suffer from depression. Depression is the leading cause of disability as measured by YLDs (Years Lived with Disability) and the 4th leading contributor to the global burden of disease (DALYs = Disability Adjusted Life Years; The sum of years of potential life lost due to premature mortality and the years of productive life lost due to disability) in 2000. An estimated 5.8% of men and 9.5% of women will experience a depressive episode in any given year. By the year 2020, depression is projected to reach second place of the ranking of DALYs calculated for all ages, both sexes. In the developed regions, depression will then be the highest ranking cause of burden of disease.

Today the first-line treatment for most people with depression consists of antidepressant medication, psychotherapy or a combination of both. Anti-depressants are effective across the full range of severity of major depressive episodes. Currently, effective antidepressive therapy is closely related to modulation or fine-tuning of serotonergic neurotransmission. Drugs that increase the levels of serotonin in the brain are the most potent known antidepressants (such as fluoxetine, Prozac® or Fluctin®). Treatments, which have antidepressive effects in patients, too, are e.g. pharmacological antidepressants such as lithium, electro-convulsive therapy and physical exercise. Other interventions include setting up supportive network systems for vulnerable individuals, families and groups. The evidence regarding prevention of depression is less conclusive, only a few isolated studies show that interventions proposed for the prevention of depression are effective. It is important to have in mind that the existing drugs are aimed at alleviating symptoms of the disease, but not primarily to address basic pathophysiological mechanisms causative to this disease. Therefore, a new treatment is needed that specifically addresses the newly discovered causal aspects in depression

Schizophrenia is one of the most common mental illnesses. About 1 of every 100 people (1% of the population) is affected by schizophrenia. This disorder is found throughout the world and in all races and cultures. Schizophrenia affects men and women in equal numbers, although on average, men appear to develop schizophrenia earlier than women. Generally, men show the first signs of schizophrenia in their mid 20s and women show the first signs in their late 20s. Schizophrenia has a tremendous cost to society, estimated at $32.5 billion per year in the US. Schizophrenia is characterized by several of the following symptoms: delusions, hallucinations, disorganized thinking and speech, negative symptoms (social withdrawal, absence of emotion and expression, reduced energy, motivation and activity), catatonia. The main therapy for schizophrenia is based on neuropleptics, such as chlorpromazine, haloperidol, olanzapine, clozapine, thioridazine, and others. However, neuroleptic treatment often does not reduce all of the symptoms of schizophrenia. Moreover, antipsychotic treatment can have severe side effects, such as tardive dyskinesias. The etiology of schizophrenia is not clear, although there seems to be a strong genetic influence. Recently, it has become clear that schizophrenia has at least some aspects of a neurodegenerative disease. In particular, MR studies have revealed rapid cortical grey matter loss in schizophrenic patients (Thompson, et al. (2001), Proc Natl Acad Sci U S A, 98, 11650-5; Cannon, et al. (2002), Proc Natl Acad Sci U S A, 99, 3228-33). Therefore, treatment of schizophrenics with neuroprotective medication such as GCSF or GMCSF or other hematopoetic factors is warranted.

In humans there is a need for ways to increase cognitive capacities, and boost intelligence. "Intelligence" in modem understanding is not limited to purely logical or semantic capabilities. For example, the theory of multiple intelligences by Howard Gardner evaluates intelligence from evolutionary and anthropological perspectives and yields a broader view that includes athletic, musical, artistic, and empathetic capacities as well as the linguistic/logical abilities that are more commonly associated with intelligence and measured by IQ tests. This broader sense of intelligence also extends into the area of creativity.
In addition, there is a non-pathological condition known in the human as ARML (age-related memory loss) or MCI (mild cognitive impairment) or ARCD (age-related cognitive decline) that usually commences at about age 40, and is different from early signs of Alzheimer's disease.

There is a physiological loss of nerve cells throughout adulthood, estimated to as many as 100,000 neurons a day. Throughout adulthood, there is a gradual reduction in the weight and volume of the brain. This decline is about 2% per decade. Contrary to previously held beliefs, the decline does not accelerate after the age of 50, but continues at about the same pace from early adulthood on. The accumulative effects of this are generally not noticed until older age.

While the brain does shrink in size, it does not do so uniformly. Certain structures are more prone to shrinkage. For example, the hippocampus and the frontal lobes, two structures involved in memory, often become smaller. This is partly due to a loss of neurons and partly due to the atrophy of some neurons. Many other brain structures suffer no loss in size.
The slowing of mental processing may be caused by the deterioration of neurons, whether they are lost, shrink, or lose connections. This depletion of fully functioning neurons makes it necessary to recruit additional networks of neurons to manage mental tasks that would otherwise be simple or automatic. Thus, the process is slowed down.
A portion of the frontal lobe, called the prefrontal cortex, is involved in monitoring and controlling thoughts and actions. The atrophy that occurs in this brain region may account for the word finding difficulties many older adults experience. It may also account for forgetting where the car keys were put or general absentmindedness. The shrinkage of both the frontal lobe and the hippocampus are thought to be responsible for memory difficulties. Therefore, there also remains a need for improving or enhancing the cognitive ability of an individual.

In view of the above, there is a need for treating neurological and/or psychiatric conditions, such as neurological diseases that relate to the enhancement of plasticity and functional recovery, or cell-death in the nervous system. In particular, there is a need for treating neurological diseases by providing neuroprotection to the neural cells involved.

### SUMMARY OF THE INVENTION

The present invention describes means for treating a neurological or a psychiatric condition in a mammal by administering to the mammal a hematopoietic factor such as GMCSF, a GMCSF derivative, GCSF, a GCSF derivative, and combinations thereof, or cells secreting GCSF or GMCSF or derivatives, to treat the condition.

As claimed, the present invention is directed to G-CSF or a functional active variant thereof having at least 90 % sequence identity to human G-CSF as shown in SEQ ID NO:28 for use in a method for the treatment of amyotrophic lateral sclerosis.

### BRIEF DESCRIPTION OF THE DRAWINGS

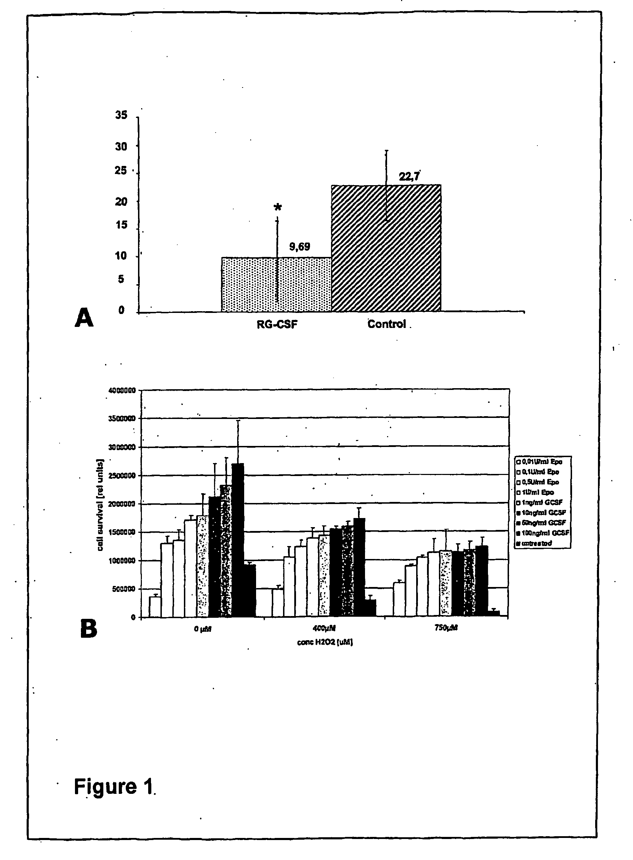

Figure 1 demonstrates effective neuroprotection by GCSF in vivo and in vitro. A, the extent of neuroprotection of GCSF after focal cerebral ischemia (filament model; middle cerebral artery occlusion, MCAO) as measured by TTC-staining. The values of the y-axis relate to percent of infarction of the total hemisphere (data are mean ± SD; T-test; p<0.05). B, cell survival assay in NGF-treated PC12 cells under increasing oxidative stress by H₂O₂ (O uM, 400 uM, 750 uM). GCSF treatment produces dramatic increases in cell survival. In comparison, cell survival after treatment of the cells with Erythropoetin (EPO), a known neuroprotective substance, is given. Y-axis: Relative (Rel.) cell survival (light units of luciferase activity).

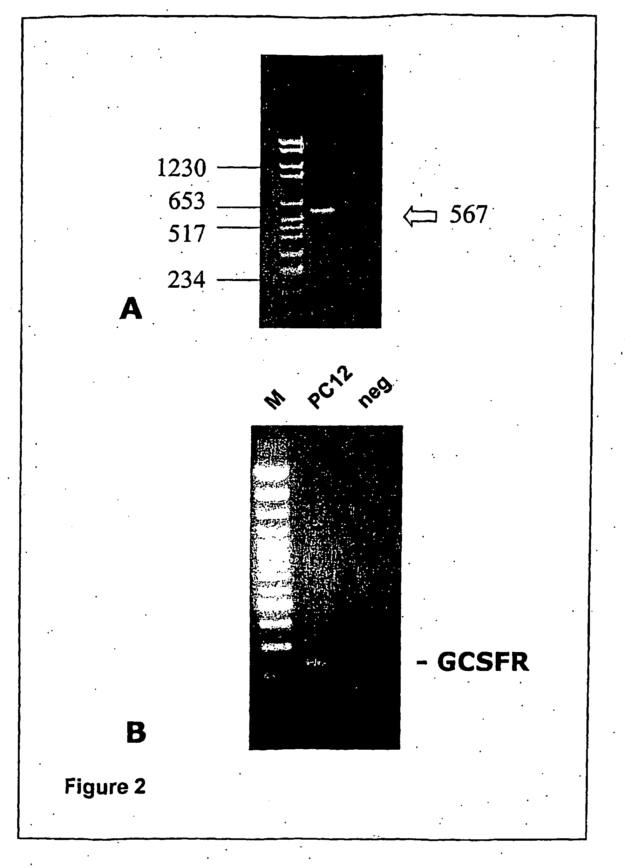

Figure 2A shows a RT-PCR specific for the mouse GCSFR. GCSF-R RNA was detected in the brain tissue with the expected size of 567 bp. The identity was verified by sequencing the PCR product.

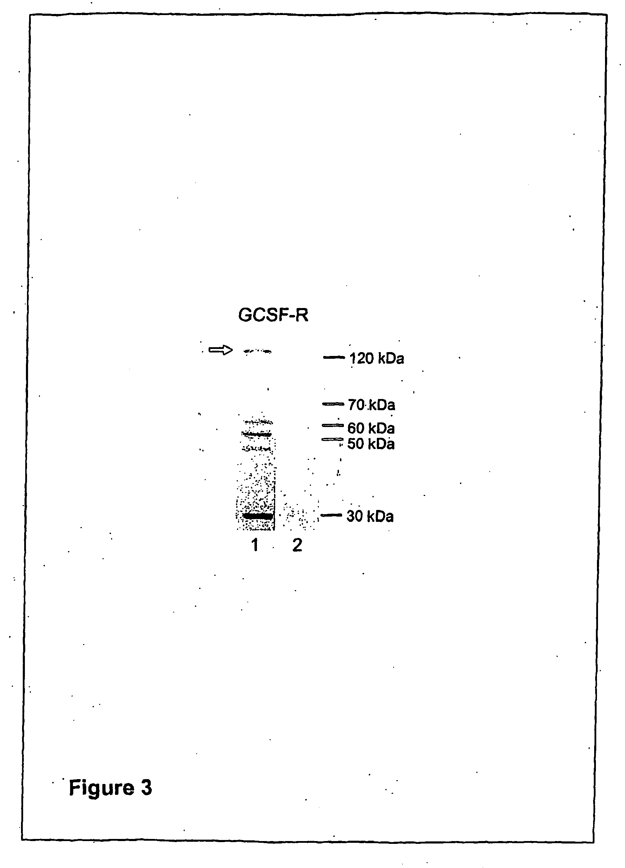

Figure 3 Representative immunoblots of GCSF-R. Using the GCSF-R antiserum a band at about 130 kDa is detectable in tissue of the rat cortex (lane 1). Additional bands of lower molecular weight most probably reflect break down products. After preabsorption of the antiserum with the respective peptide, no bands are yet detectable (lane 2).

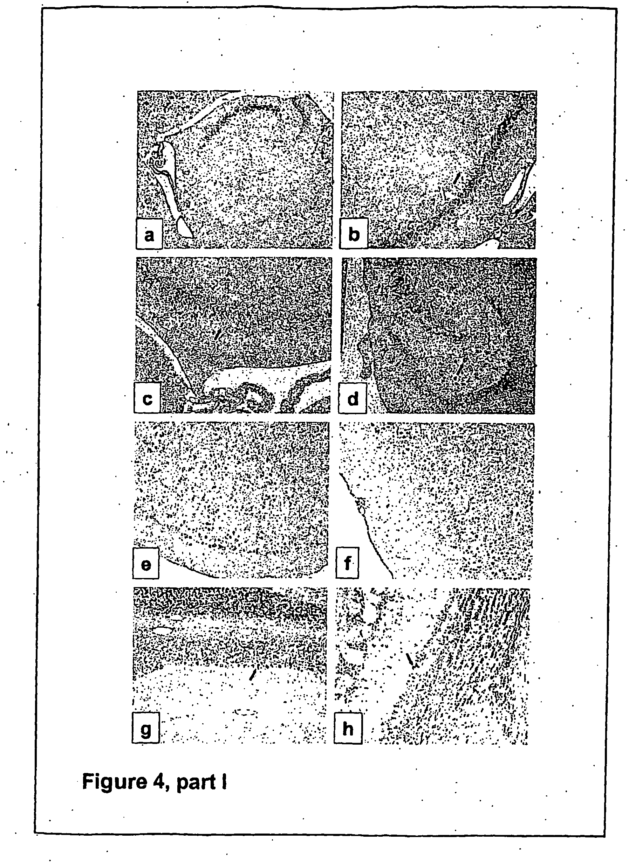

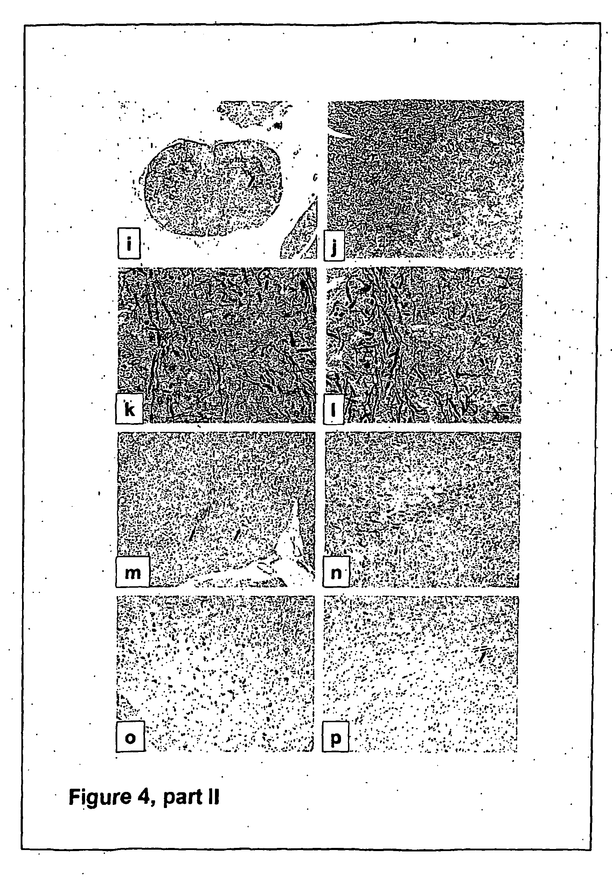

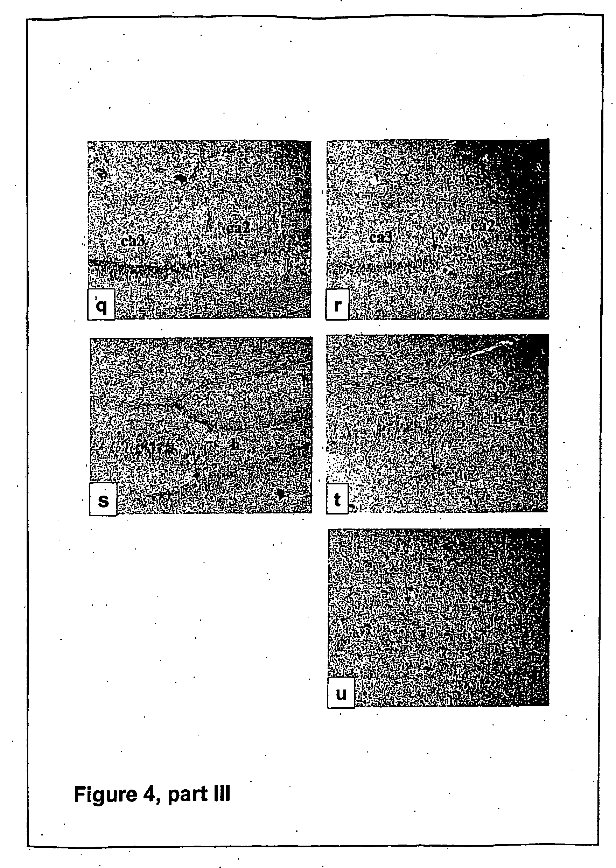

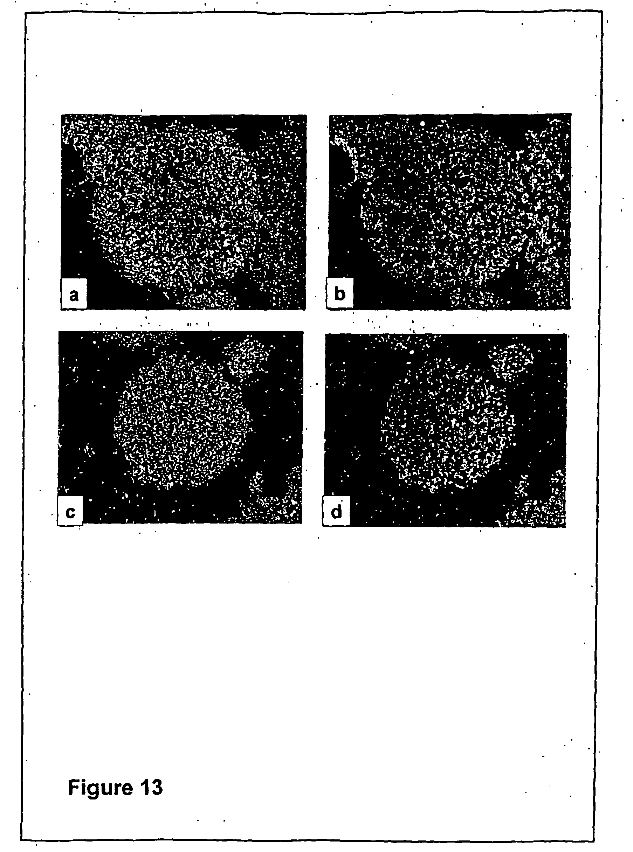

Figure 4 Immunohistochemistry showing the distribution of GCSF-receptor in different brain regions in the mouse (paraffin sections, 2 µm). a-d: localization of GCSF-R in the hippocampus. Note that the antibody predominantly stains neurons in the CA3 area (a,b), with a sharp boundary between the CA3 and CA2 region (c, arrow). GCSF-R is distributed over the soma, as well as processes of neurons (b, arrow). Note the presence of the receptor in the hilus and the basal cell layers of the dentate gyrus (d, arrow). GCSF-Receptor was also detected in cortical areas: piriform cortex (e), and perirhinal cortex (f) as examples. In the cerebellum, Purkinje cells are labeled (g, arrow). Also, some of the large mitral cells in the olfactory bulb are GCSF-R positive (h, arrow). Strong staining is exhibited by the anterior columns in the spinal cord (i, j), and higher magnification identifies the large motoneurons as GCSF-R positive (k,l). Note that the neuronal processes are strongly labeled. In the midbrain, neurons in the substantia nigra show GCSF-R positivity (m). Especially, all neurons in the pars compacta (SNC) are labeled (arrow in m, and n). Also, in the pars reticulata, several neurons express GCSF-R (o). Apart from neurons, oligodendrocytes in white matter tracts are stained, for example, in the anterior commissure (p, arrow). Surprisingly, the staining of the GCSF ligand (antibody sc13102, Santa Cruz) colocalizes with the expression of its receptor (antibody sc694, Santa Cruz) (Figures 4 q-u). This argues for a autocrine mechanism as a protective measure of neurons against noxious stimuli. In the hippocampus, the same subfield specificity is observed for the GCSF ligand (q: GCSFR, r: GCSF, arrows point to the border between subfields CA 2-3, ca3 and ca2 labels indicate the subfield). This specificity coincides with known differences in susceptibilities of these regions against ischemic damage, and argues again for a neuroprotective function of the GCSF system. Also, in the dentate gyrus, the same interesting pattern of expression in the hilus and the subgranular zone is observed (Figure 4s, GCSF receptor; Figure 4t, GCSF; arrows point to one neuron in the subgranular zone, labels: s: subgranular zone, h: hilus of the dentate gyrus), which underlines the importnace of the GCSF system for neurogenesis, and nicely parallels the expression of receptor and ligand on neurospheres (see Figure 13). Interestingly, GCSF is also expressed in the large motoneurons of the spinal cord (Figure 4 u) where its receptor is also expressed (Figure 4 i-l).

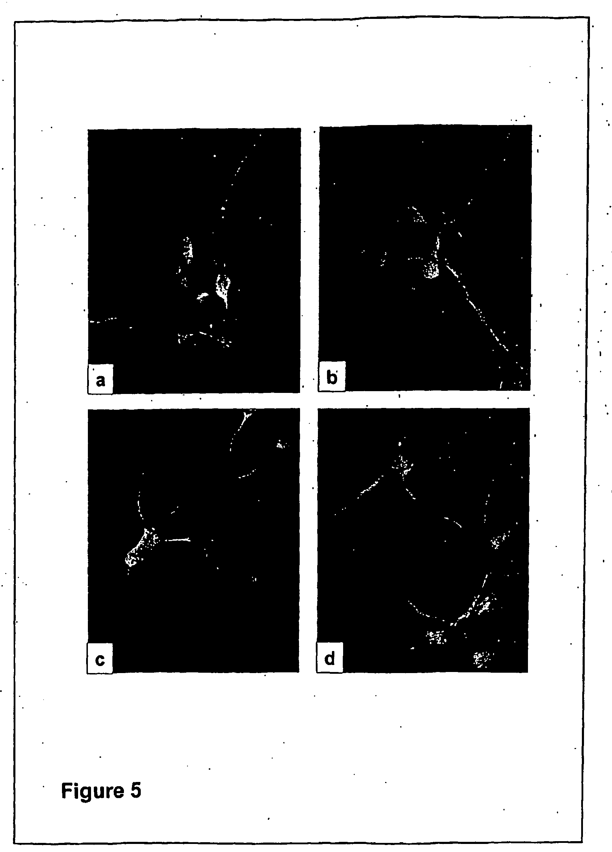

Figure 5 shows a staining for the GCSFR on cortical (a, b) and hippocampal cultured neurons (c,d). The receptor is both present on the somata and processes of the neurons. Not all neurons on the slide were labelled. Preincubation of the antibody with the respective peptide, or omission of the primary antibody did not result in specific staining (not shown).

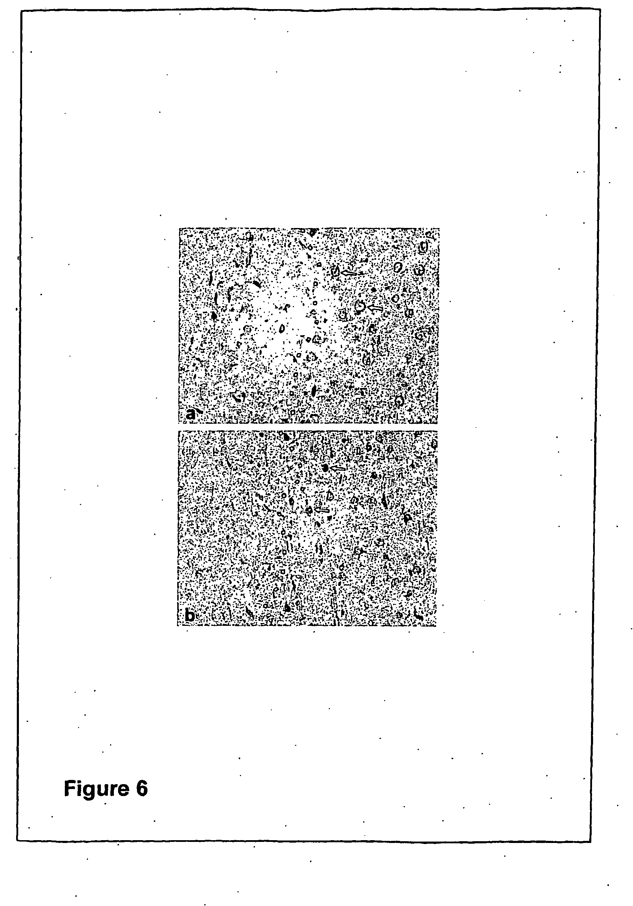

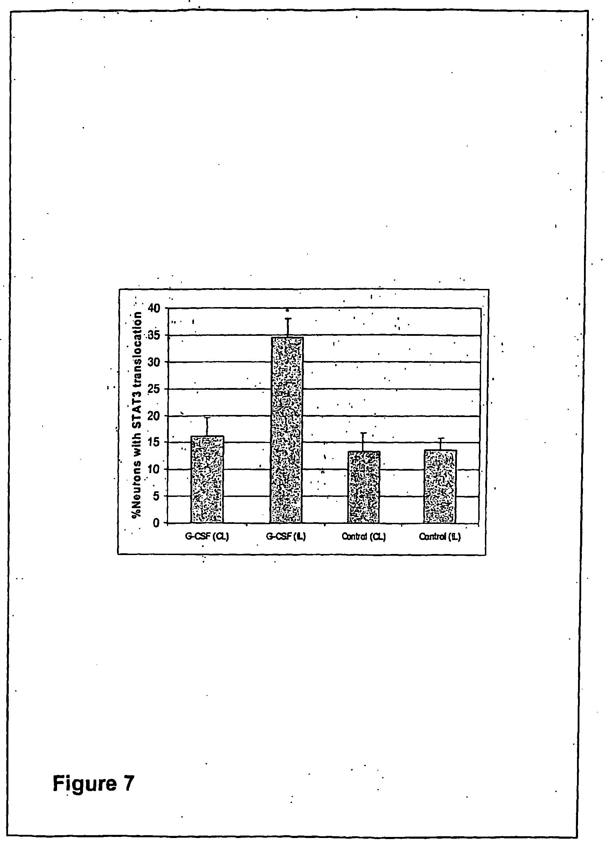

Figure 6 and 7 show the immunohistochemical results obtained with an antibody against STAT3. Figure 6: STAT3 immunohistochemistry. Note, that numerous neuronal nuclei are positively stained in the cortical penumbra of GCSF treated rats (b, arrows) compared to the cortical periinfarct area of a placebo treated animal (a, arrows; left: infarct; right: penumbra; original magnification x200). Figure 7: Cortical neurons in the unaffected contralateral side (CL) and ipsilateral in the vicinity of the infarction (IL) were quantified in GCSF treated animals and controls. There was a significant activation of STAT3 in neurons adjacent to the infarction in the GCSF-treated group as quantified by counting neurons with nuclear translocation of STAT3 (*p<0.05, t-test).

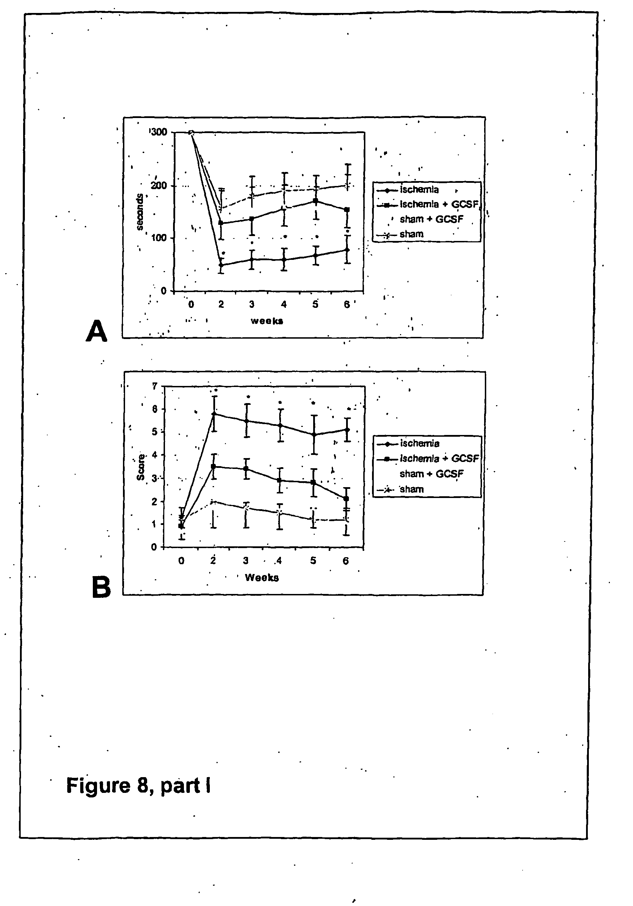

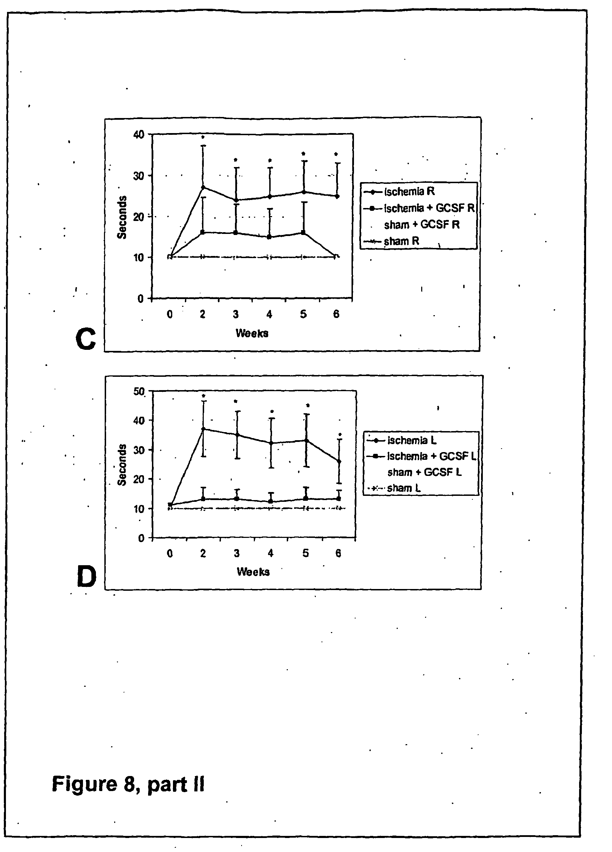

Figure 8 shows the effect of GCSF treatment in the photothrombotic bengal rose model of cerebral ischemia. A, rotarod performance; B, neurological score, including beam balance; C, D: adhesive tape removal test, measured on the ipsi- (C), as well as contralateral side (D). Legend: *ischemia*: group of ischemic rats, non-treated; *ischemia + GCSF*: group of ischemic rats treated with GCSF; *sham + GCSF*: sham-operated animals, treated with GCSF; *sham*: sham-operated animals, untreated. *L*: tape-removal test on the left paw; *R*: tape-removal test on the right paw. Note that there is an effect on both sides in the tape-removal test (C,D), probably caused by a predominant motor deficit when the tape is on the ipsilateral side, and a predominant sensor deficit, when the tape is on the contralateral side.

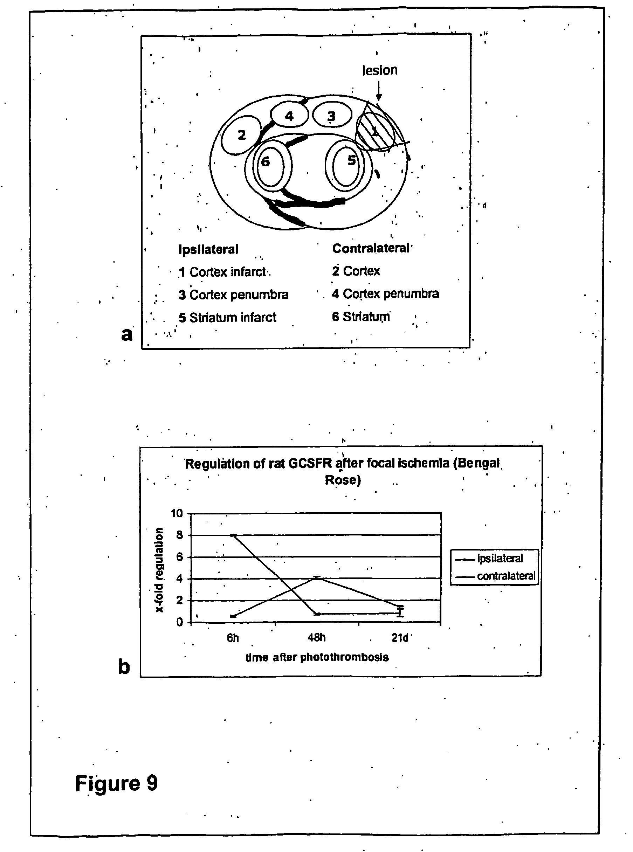

Figure 9 shows the upregulation of the GCSF-Receptor in the bengal-rose model 48h after induction of photothrombosis on the contralateral side to the ischemia. A, scheme of a coronal section of a rat brain, tissue samples 3 and 4 were used for the quantification of the GCSF receptor mRNA compared to the same tissue samples from sham-operated rats. B, quantification of GCSF receptor mRNA in the cortical penumbral samples (samples 3 and 4 from A). An initial upregulation at 6h after ischemia induction on the ipsilateral side is followed by an upregulation on the contralateral side at 48h after the infarct. This contralateral upregulation was also seen with the GMCSF receptor (see below).

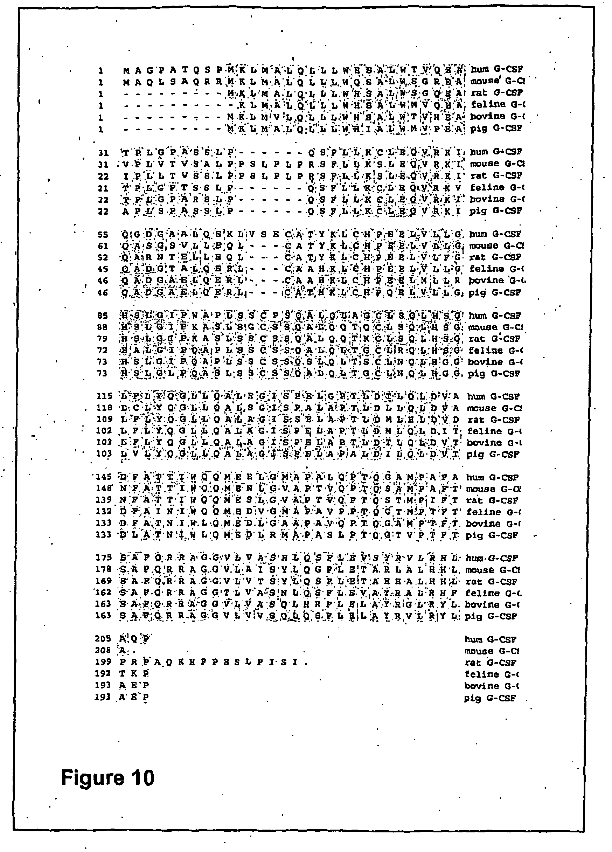

Figure 10 shows an alignment of the GCSF from different species using the ClustalW algorithm (SEQ ID NOS:28-33) (MEGALIGN™, Lasergene, Wisconsin).

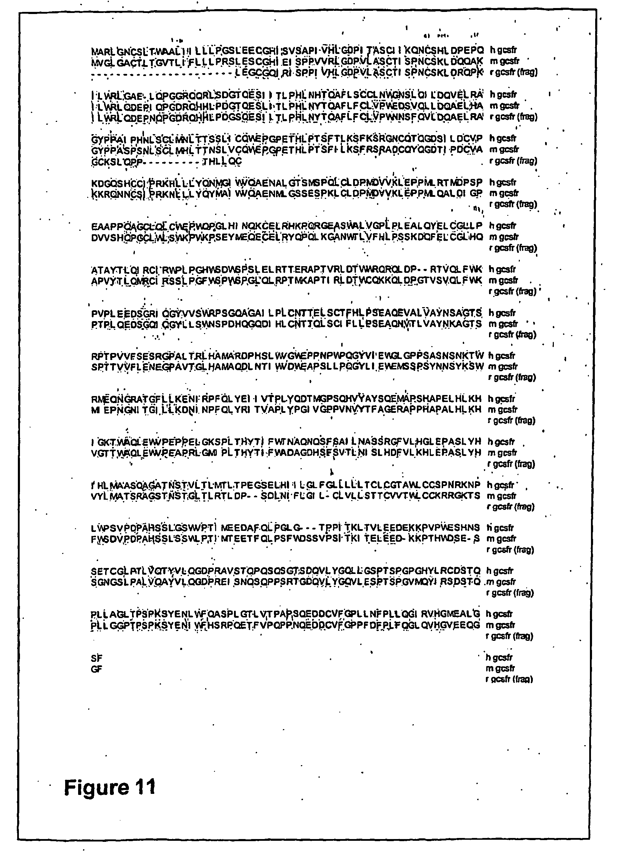

Figure 11 shows an alignment of GCSF receptors from mouse and human, and a fragment of rat using the ClustalW algorithm (SEQ ID NOS: 34-36) (MEGALIGN™, Lasergene, Wisconsin).

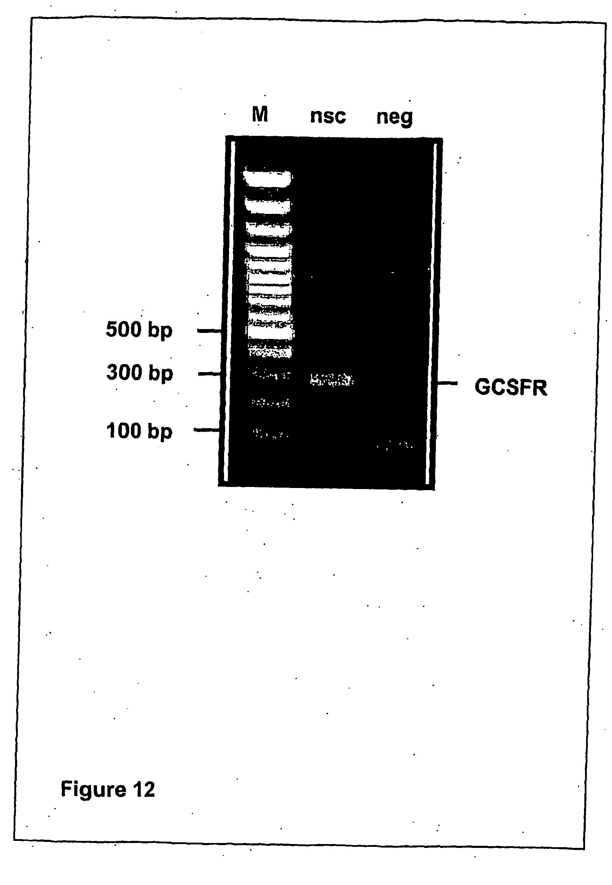

Figure 12 demonstrates the presence of the GCSF receptor on adult neuronal stem cells (nsc) by RT-PCR (reverse transcription PCR). Shown is an agarose gel. Lane 1: size marker; lane 2: PCR products from neuronal stem cells (nsc), visible is the rat GCSFR - specific band (279 bp); lane 3: negative control.
Figure 13 demonstrates the presence of the GCSF-Receptor and GCSF on neural stem cells. A. DAPI stain of a neurosphere for visualization of all cells, B, the same neurosphere stained with an antibody directed against the GCSF receptor. C,D neurosphere stained with DAPI (C) and an antibody for GCSF (D).

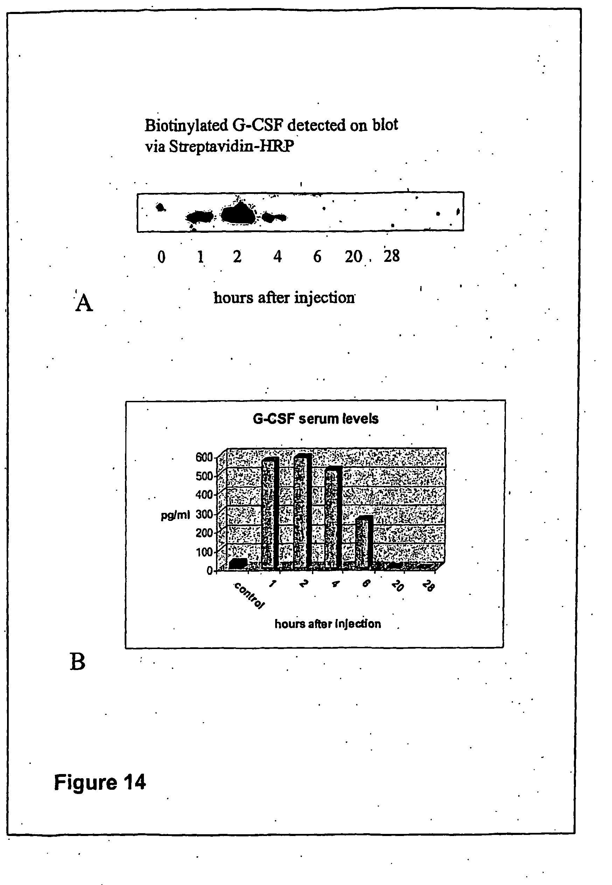

Figure 14 demonstrates the rapid uptake of biotinylated GCSF after intraperitoneal injection into mice. A, Western blot of serum from mice sacrificed at 1,2,4,6,20,28 h post injection of 7.5 ug GCSF/mouse. B, ELISA of Serum GCSF after i.p. injection. There is rapid uptake of GCSF from the peritoneum with serum peak levels at 2 hrs., demonstrating applicability of this administration route.

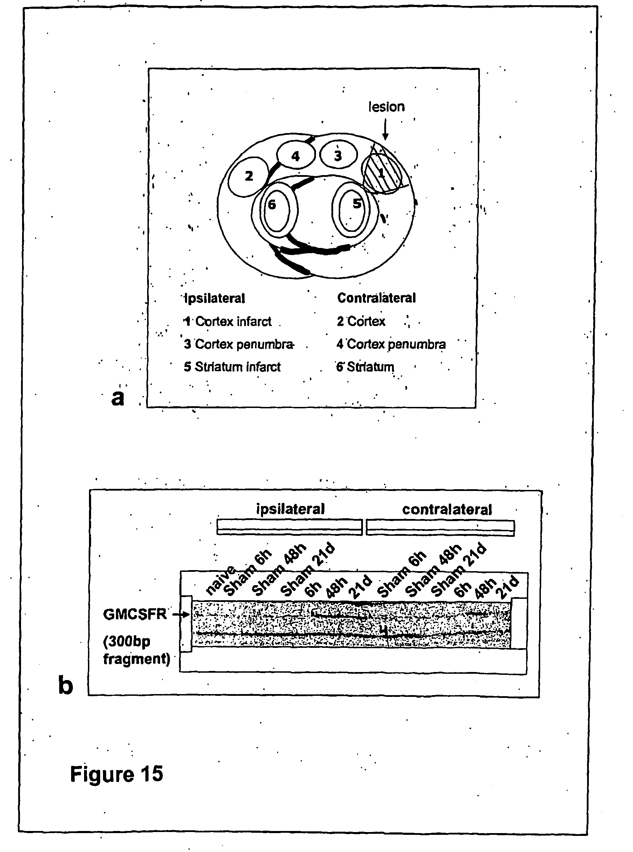

Figure 15 shows the identification of the GMCSF-Receptor as an upregulated mRNA after induction of photothrombosis (bengal rose model) on the ipsilateral and contralateral side to the ischemia. A shows a schematic coronal section of a mouse brain and areas of interest are marked in grey; B shows a section of an RMDD-Gel, on which the transcript of the GMCSF-Receptor was identified as being regulated. The lanes represent RT-PCR-products on RNA samples of mouse brain. Samples were taken from cortex penumbra at different timepoints after the stroke (3 and 4 in A, respectively).

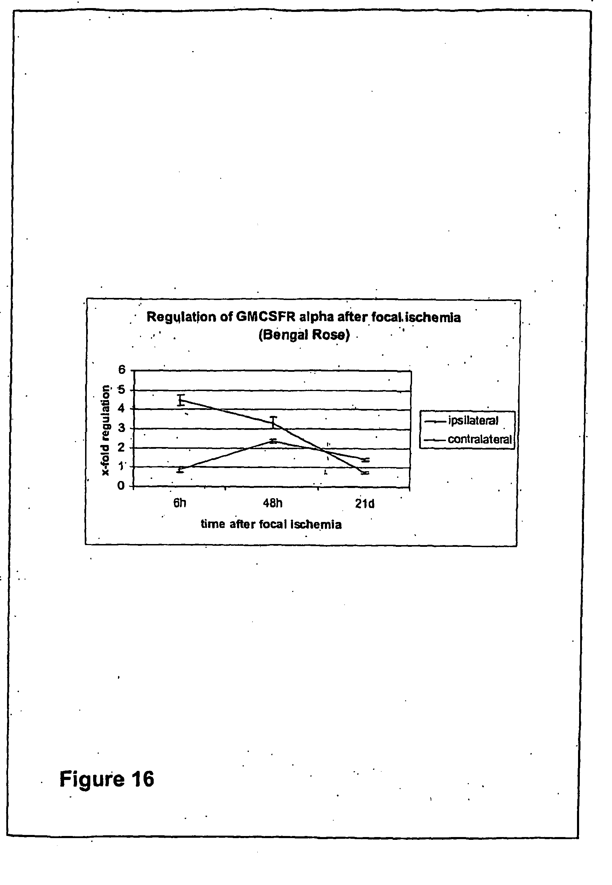

Figure 16 shows the verification of the upregulation of the GMCSF-Receptor in the bengal-rose model at 48h by quantitative RT-PCR by applying the LightCycler-System. Samples were taken at 6h, 48h and 21d after induction of photothrombosis and induction levels were compared to sham-operated animals. On the ipsilateral hemisphere the upregulation of the GMCSF receptor is maximal early after the cortical ischemia and drops steadily until day 21. On the corresponding contralateral cortex-sample, the upregulation is seen most clearly at 2 days after the infarct, and is still moderately upregulated at day 21. This regulation pattern on the contralateral side is reminiscent of the GCSF receptor (see above).

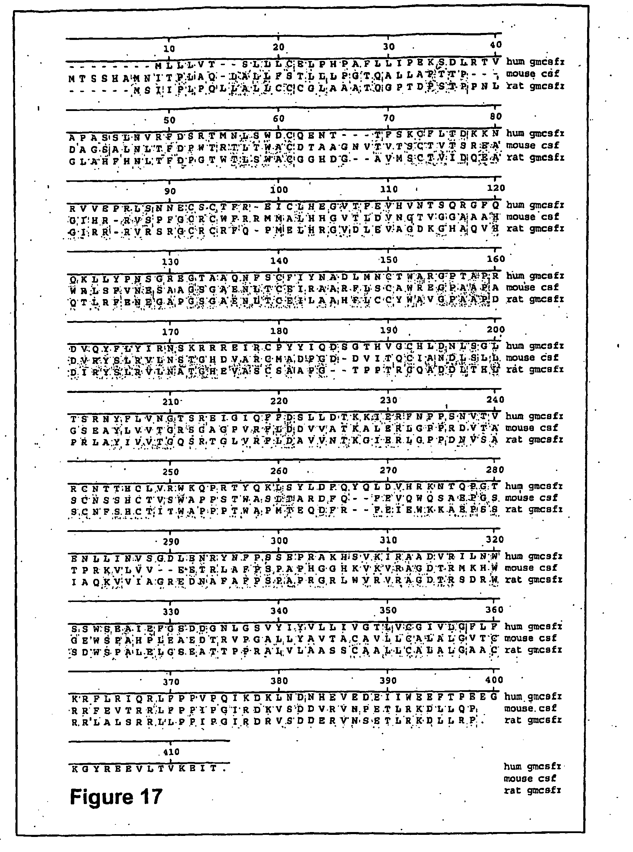

Figure 17 shows an alignment of GMCSF receptors from human, mouse, and the sequence from rat identified as an upregulated transcript (SEQ ID NOS:22, 23 and 24) (ClustalW algorithm, MEGALIGN™, Lasergene, Wisconsin). It is concluded from this homology that the identified sequence is the rat GMCSF receptor.

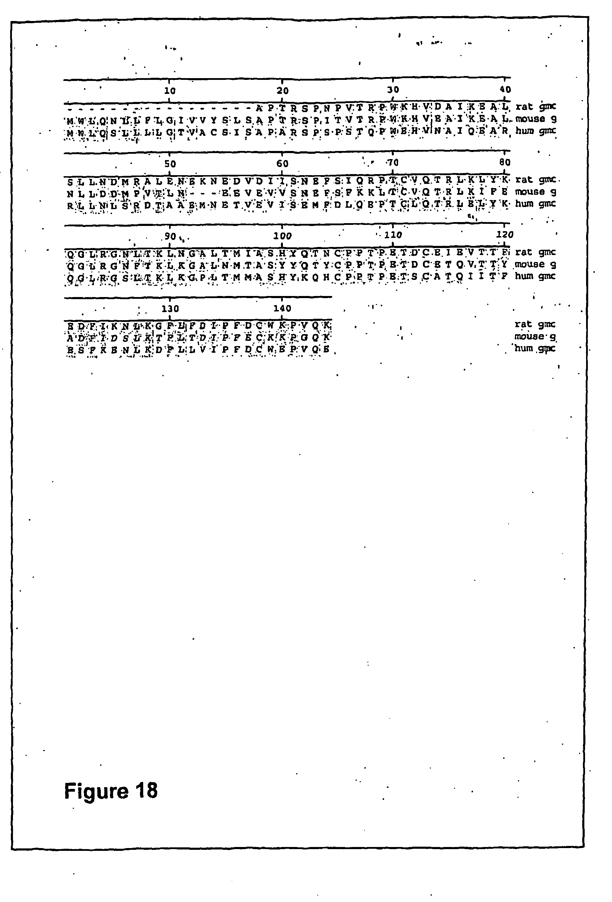

Figure 18 shows an alignment of GMCSF from human, mouse, and rat (SEQ ID NOS: 25, 26, and 27) (ClustalW algorithm, MEGALIGN™, Lasergene, Wisconsin).

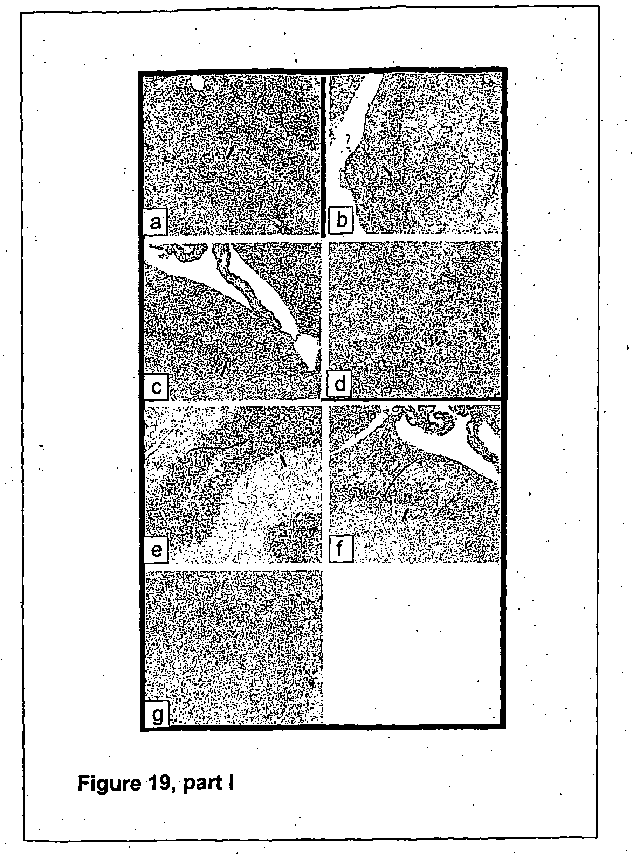

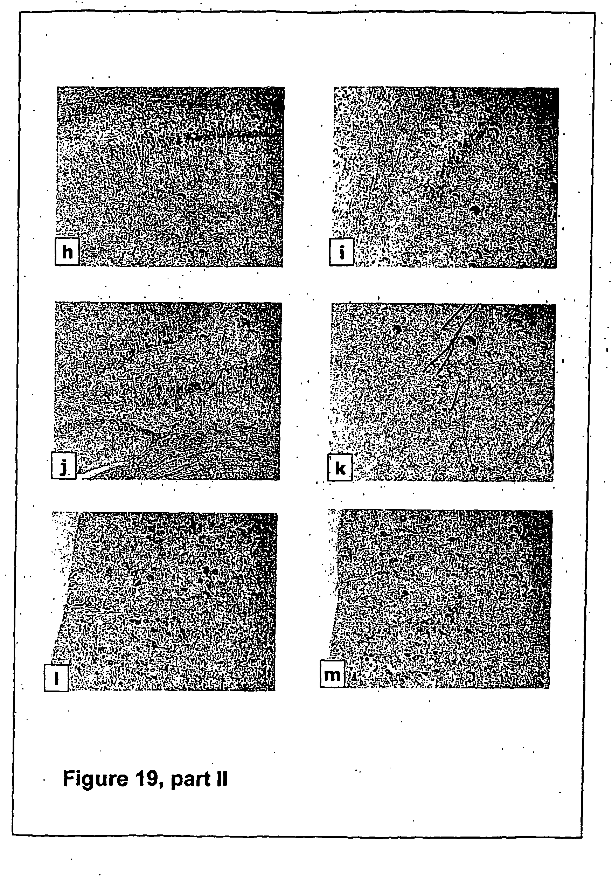

Figure 19 Immunohistochemistry showing the distribution of GMCSF-receptor alpha (a-d) and GMCSF (e-g) in different brain regions in the mouse (paraffin sections, 2 µm). a: In the cerebellum, Purkinje cells are labeled (arrow). b-d: localization of GMCSF-R alpha in the hippocampus. Note the presence of the receptor in the hilus of the dentate gyrus (b, arrow). The antibody predominantly stains neurons in the CA3 area (c) with a sharp boundary between the CA3 and CA2 region (c, arrow). GMCSF-R alpha is distributed over the soma (CA3), as well as processes of neurons (CA2). GMCSF-receptor was also detected in the entorhinal cortex (d). GMCSF shows similar distribution in comparison with GMCSF-receptor alpha. Note that the GMCSF antibody stains as well Purkinje cells (e, arrow), neurons in the CA3 area (f) with sharp boundary between CA3 and CA2 region (f, arrow), and neurons in the entorhinal cortex (g). Figures 19 h-m: Shown here is the surprising colocalization of the GMCSF receptor and its ligand in neurons. h, localization of the GMCSF receptor (antibody sc690, Santa Cruz) in neurons in hipocampal subfield CA3, arrow points to the sharp expression boundary to the adjacent CA2 region. i, the GMCSF ligand (antibody sc13101) shows the same subfield-specific expression, the arrow points to one neuron in the CA3 region. j, expression of the GMCSF receptor in the hilus and subgranular zone of the dentate gyrus. An arrow points to a neuron in the subgranular zone. k, expression of the GMCSF ligand in that region. Here, the ligand shows a slightly different expression compared to its receptor. There is clear expression in the CA3 region (arrow), but less in the dentate gyrus region. l,m: expression of the GMCSF receptor (l) and ligand (m) in the large motoneurons of the spinal cord. This surprising expression is a clear indication for the therapeutic applicability of the GMCSF system for motoneuron diseases, especially amyotrophic lateral sclerosis (ALS).

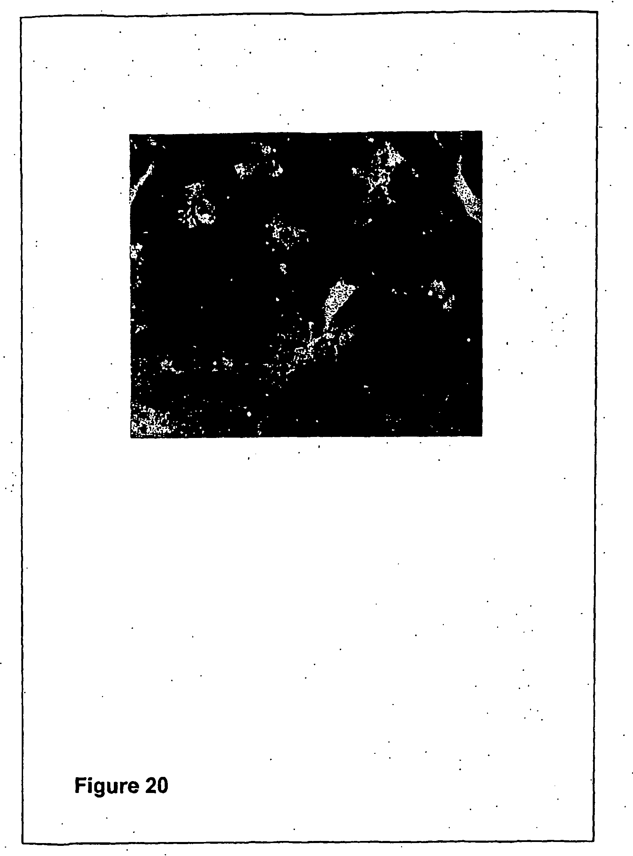

Figure 20 shows a staining for the GMCSFR alpha on cortical neuronal cultures. The receptor is both present on the somata and processes of the neurons (verified by double labeling with an antibody directed against the nuclear epitope NeuN, and an antibody for synaptophysin, which is not included in the Figure). Preincubation of the antibody with the respective peptide, or omission of the primary antibody did not result in specific staining (not shown).

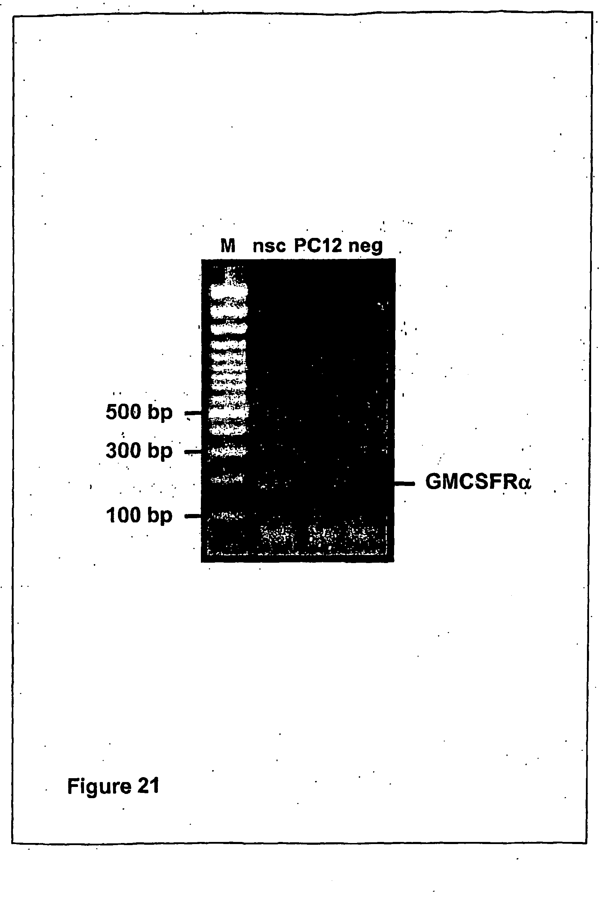

Figure 21 demonstrates the presence of the GMCSF-receptor alpha on adult neuronal stem cells (nsc) and on PC12 cells (PC12) by RT-PCR. Shown is an agarose gel. Lane 1: size marker (M); lane 2: PCR on neuronal stem cells (nsc); lane 3: PCR on PC12 cells (PC12); lane 4: negative control (neg). The rat GM-CSFR alpha-specific band (176 bp) is visible in lanes 2 and 3.

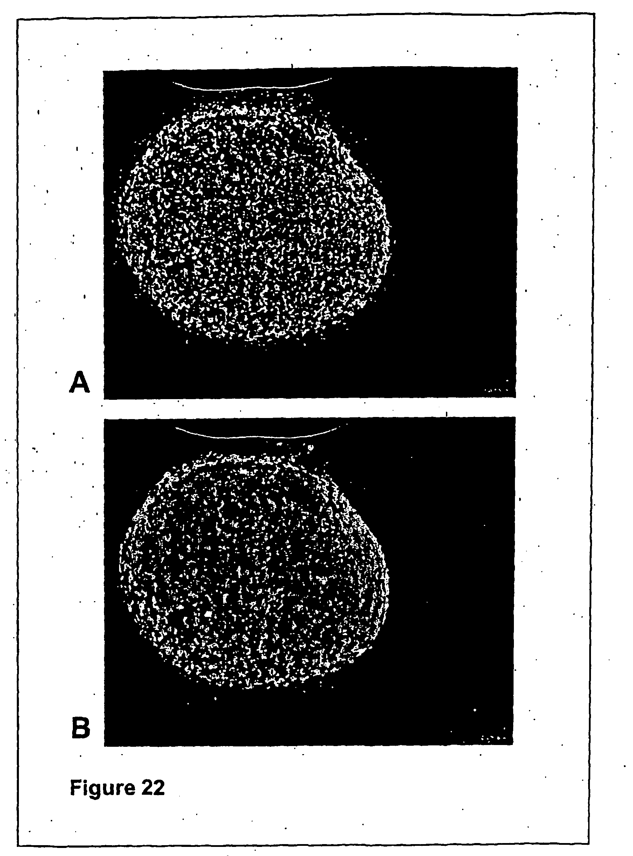

Figure 22 demonstrates the presence of the GMCSF-receptor alpha on adult neuronal stem cells (nsc) by immunocytochemistry. Shown is one neurosphere that is stained with DAPI (A, stains all cell nuclei), and an antibody specific for the GMCSF receptor (B) (magnification 10x).

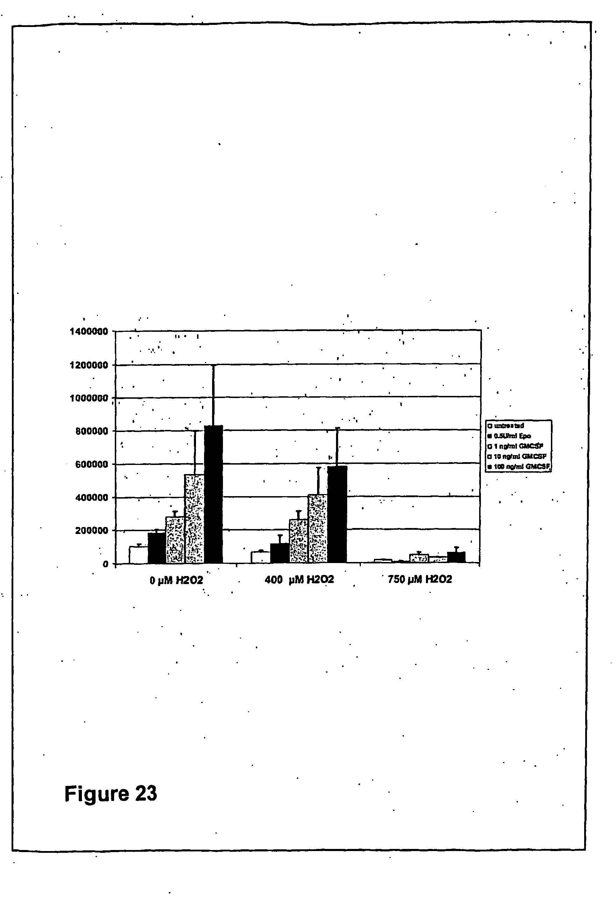

Figure 23 demonstrates effective neuroprotection by GMCSF in vitro. Cell survival assay in NGF-treated PC12 cells under increasing oxidative stress by H₂O₂ (O uM, 400 uM, 750 uM). GMCSF treatment produces dramatic increases in cell survival. In comparison, cell survival after treatment of the cells with Erythropoetin (EPO; 0.5 U/ml), a known neuroprotective substance, is given. Y-axis: Rel. cell survival (light units of luciferase activity).

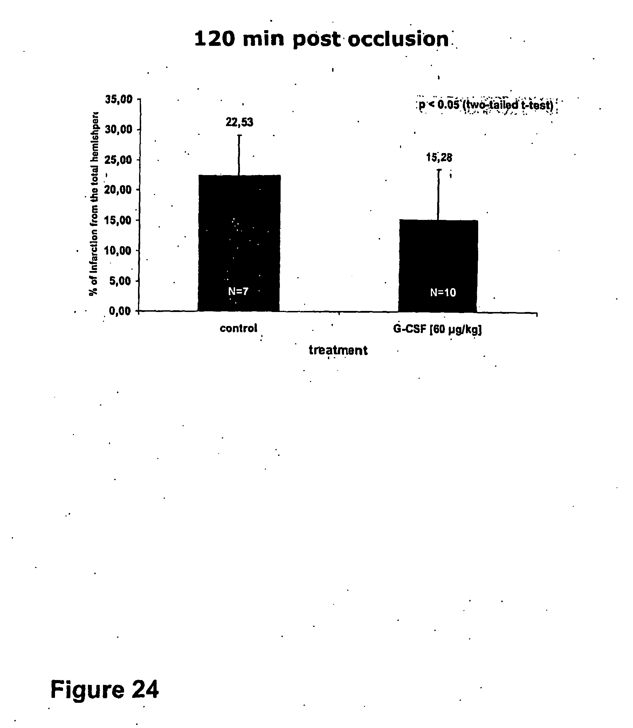

Figure 24 shows the effect of intravenously applied G-CSF on infarct volume in the rat MCAO model when applied 120 min after onset of ischemia compared to no C-CSF administration (control).

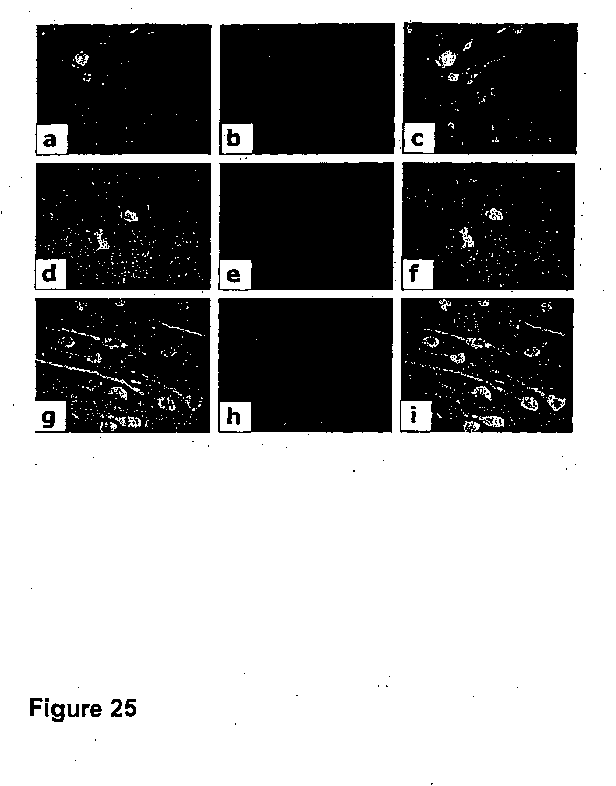

Figure 25 shows TTC-staining in the hippocampus and cortex for the G-CSG receptor and G-CSF. The data shows that the GCSF receptor ( a, d, g) shows a co-localization with its ligand (b, e, h) both in the hippocampus (a-c dentate gyrus, d-f hilus) and the cortex (g-i).

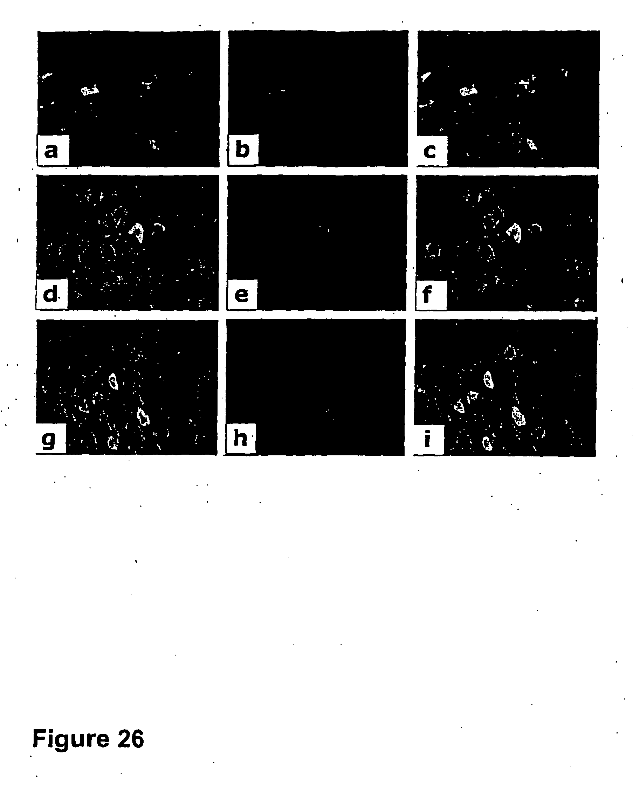

Figure 26 shows coexpression of both the G-CSF and GM-CSF receptors on neurons in the dentate gyrus (a-c) and the hilus (d-f) of the hippocampus as well as in the cortex (g-i). The GCSF receptor (a, d, g) and the GMCSF receptor (b, e, h) are expressed on the same neurons in both the hippocampus and the cortex (c, f, i).

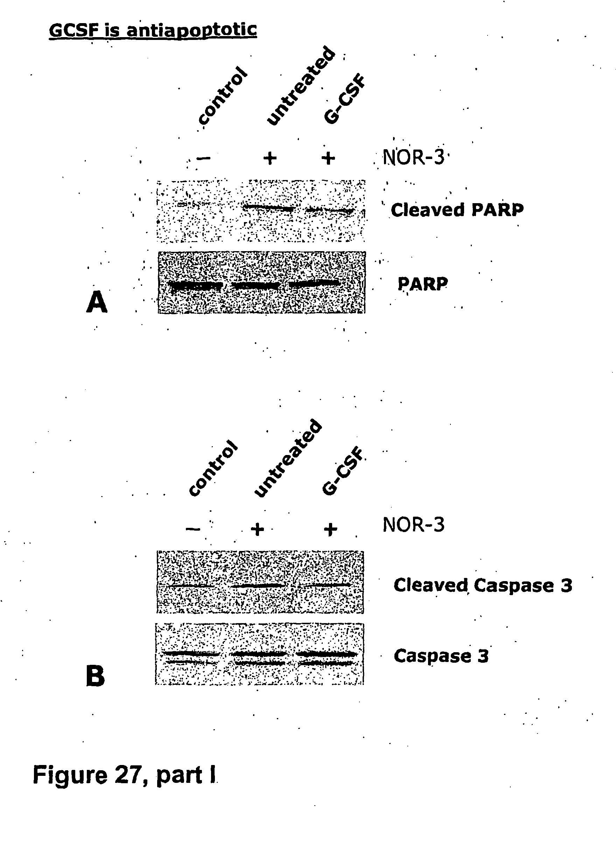

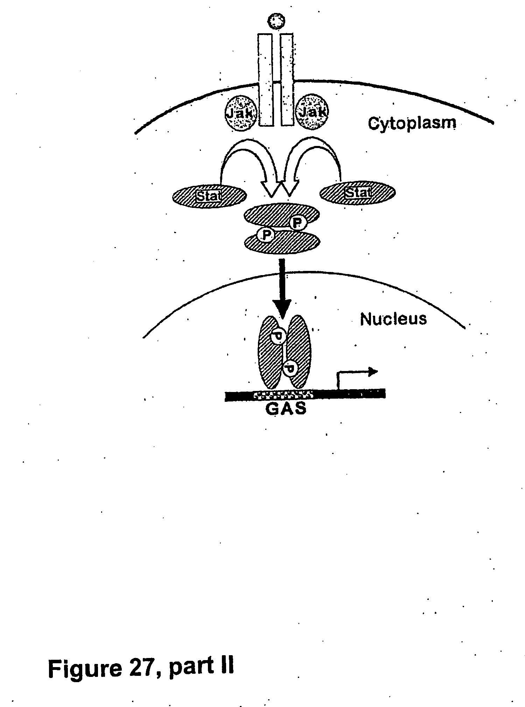

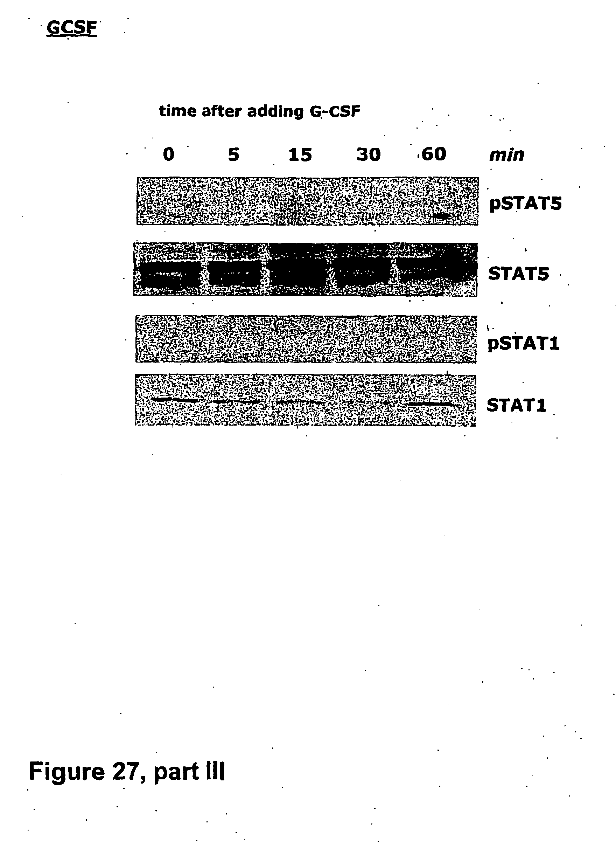

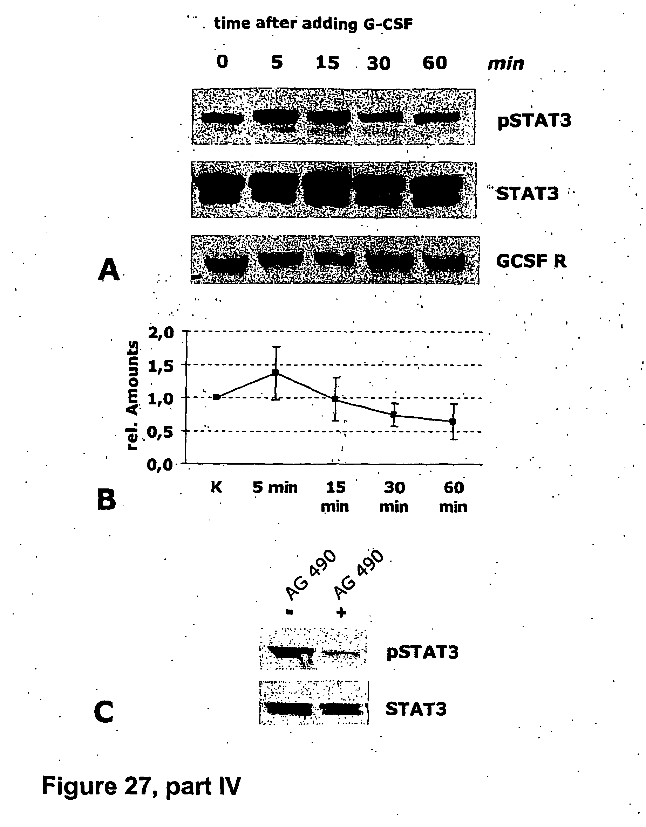

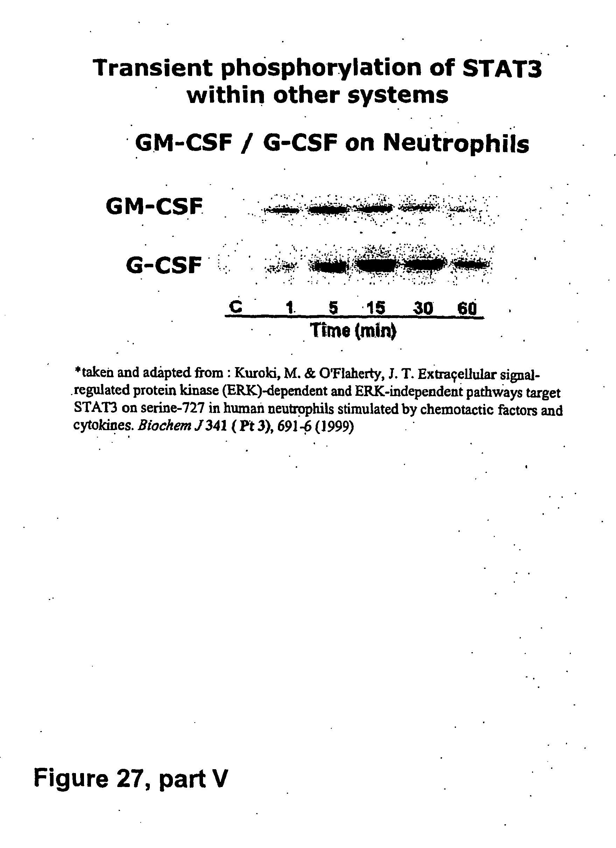

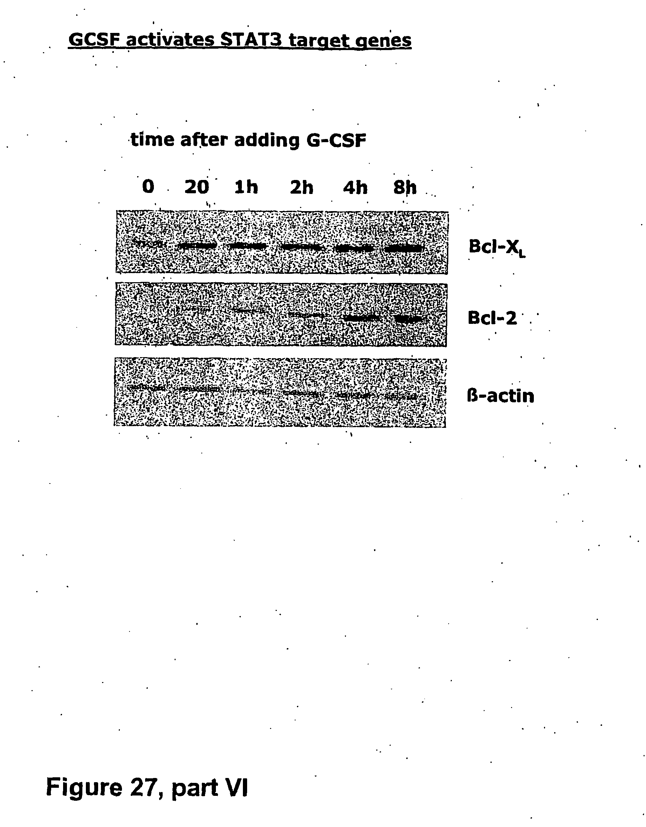

Figure 27 shows that G-CSF acts anti-apoptotically by activiating stat3 in neurons.

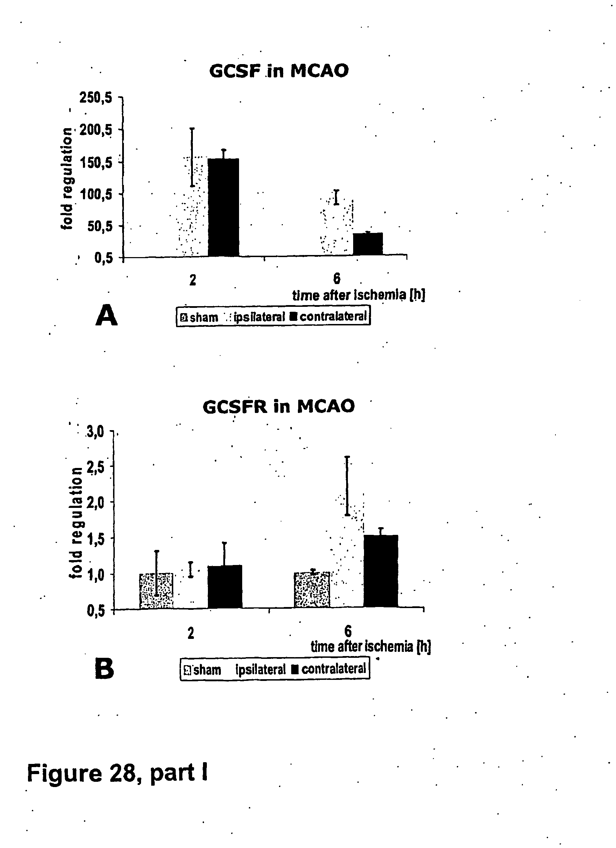

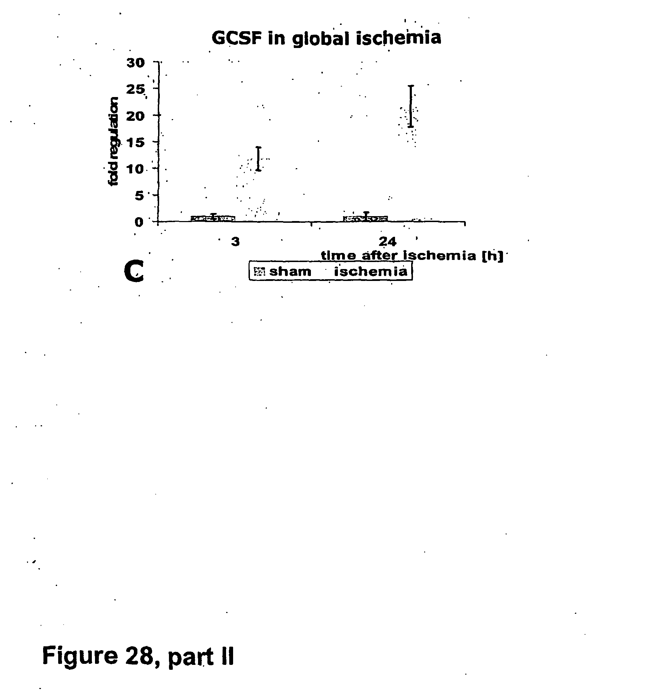

Figure 28 (parts I and II) A shows a very strong upregulation of GCSF 2h and 6h after focal ischemia on the ipsi- and contralateral side, whereas the receptor is moderately regulated (B) after 6h. An induced expression of GCSF is not specific to the MCAO model, but could also be seen albeit to a lesser degree in global ischemia (C).

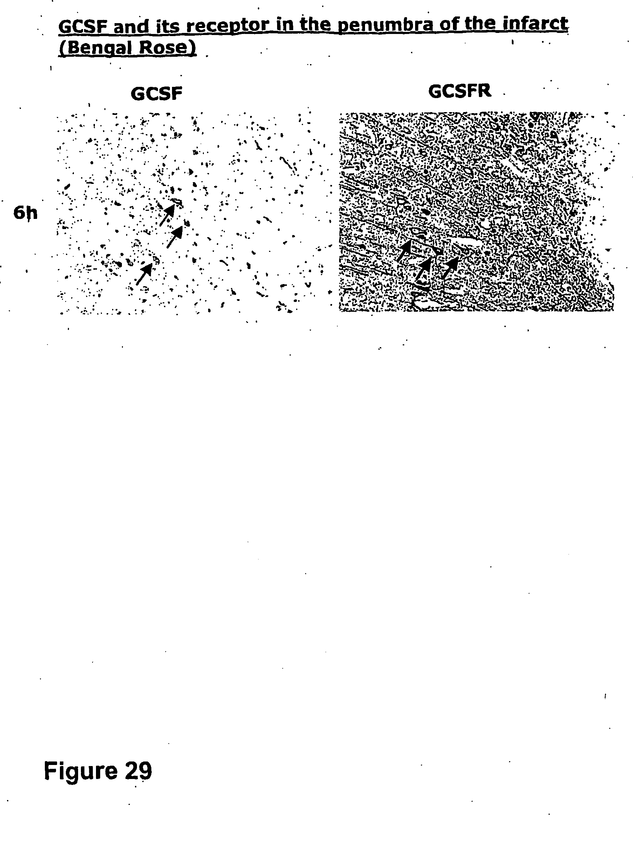

Figure 29 shows G-CSF and its receptor in the penumbra of the infarct (Benegal Rose).

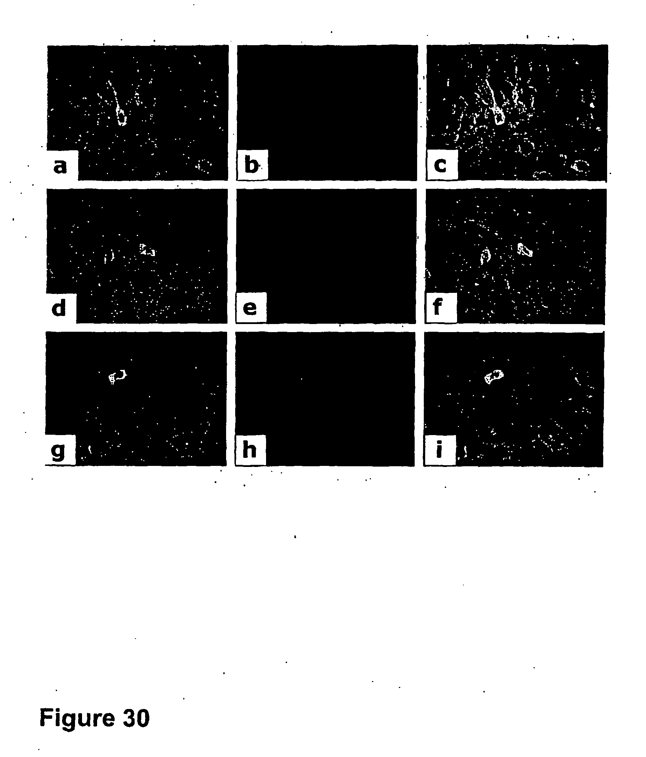

Figure 30 shows the expression of GCSF receptors (a, d, g) and doublecortin (b, e, h) on neurons in the dentate gyrus (a-f) and the hilus (g-i) of the hippocampus.

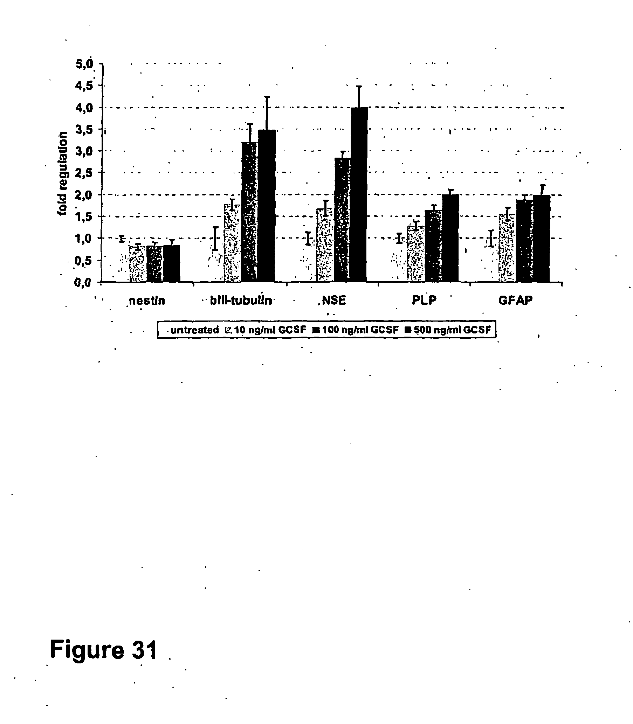

Figure 31 shows a strong and concentration dependent upregulation of the neuronal markers NSE and beta III-tubulin 4d after GCSF treatment PLP and GFAP are moderately regulated in depedency of the GCSF-concentration. Error bars indicate standard deviations, calculated from 3-fold serially diluted cDNA-samples, and reflect reliability of measurements.

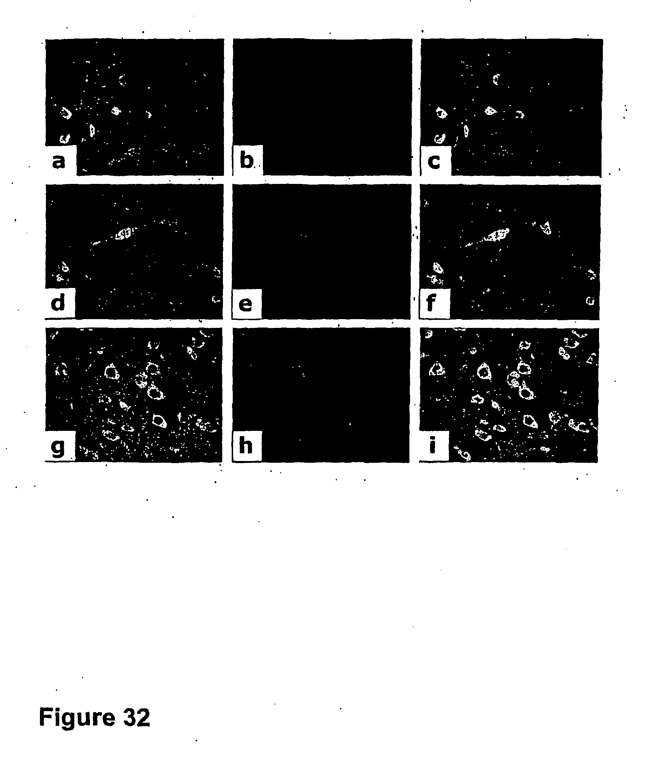

Figure 32 shows co-localisation of GMCSF and its receptor in the brain. The GMCSF receptor (a, d, g) and GMCSF (b, e, h) are colocalised on neurons in the dentate gyrus (a-c), the hilus (d-f), and the cortex (g-i).

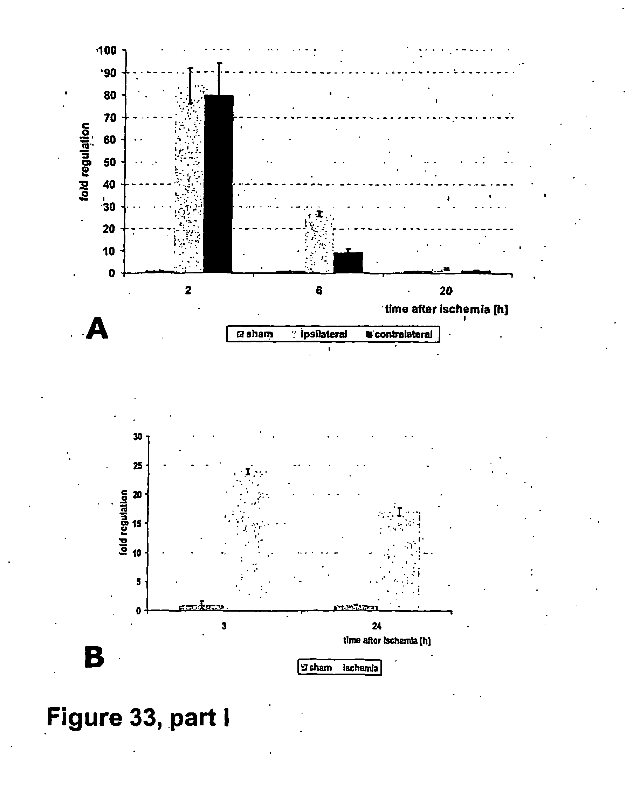

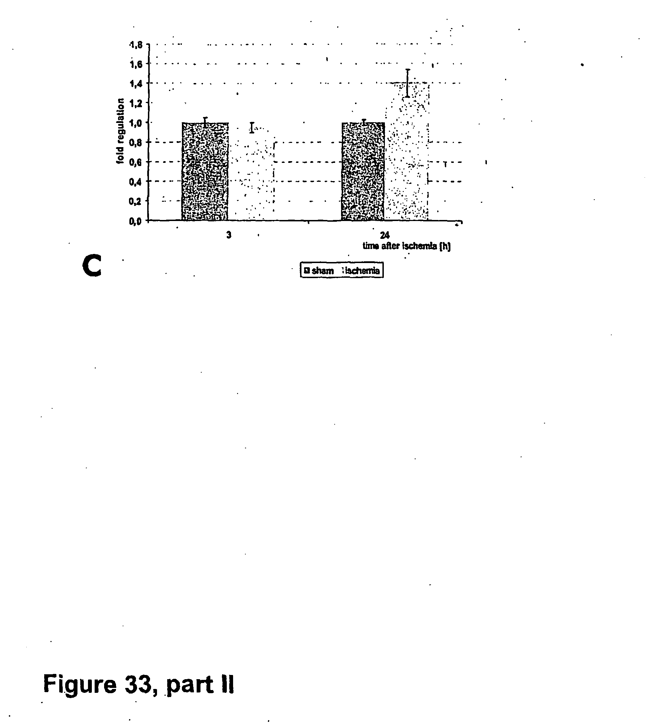

Figure 33 shows that GMCSF and its receptor are upregulated by cerebral ischemia.

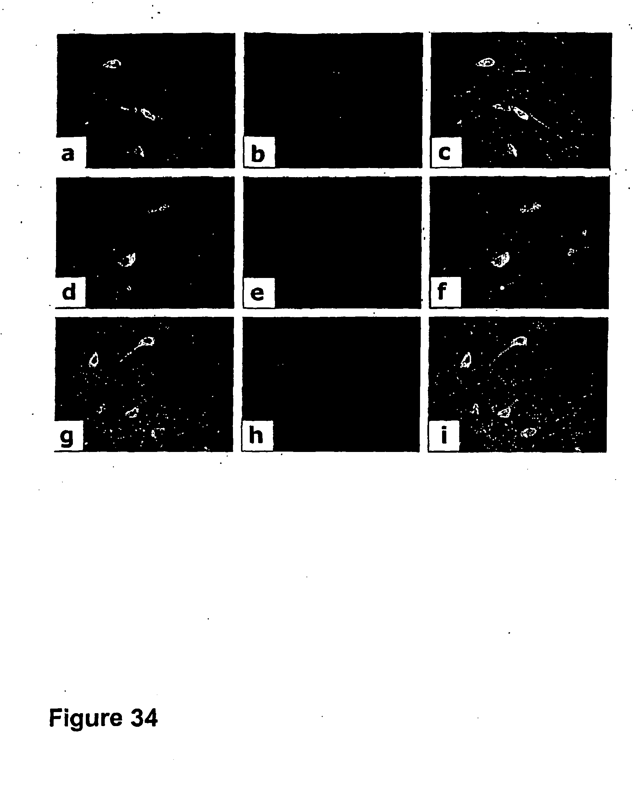

Figure 34 shows the expression of the GMCSF receptor (a, d, g) and doublecortin (b, e, h) on neurons in the dentate gyrus (a-c) and the hilus (d-i) of the hippocampus.

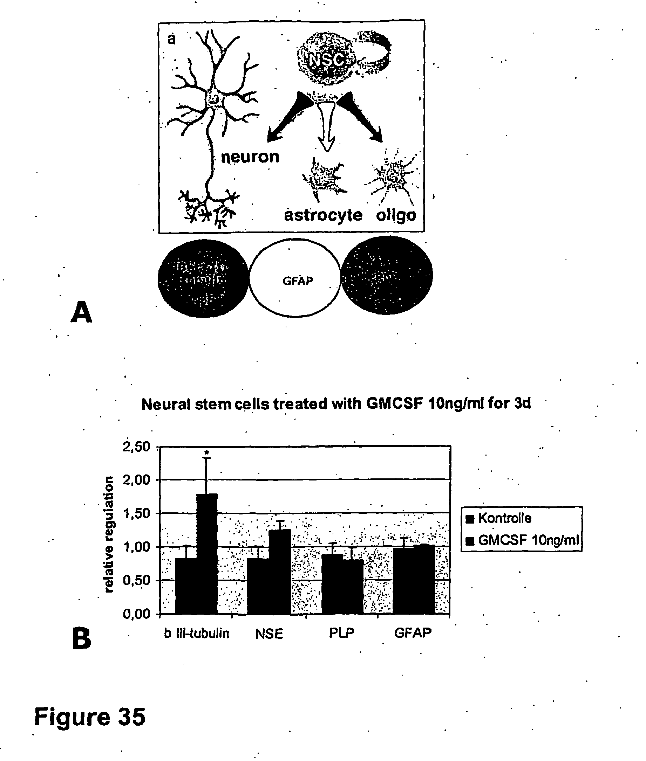

Figure 35 shows the differentiation potential of neural stem cells and the specific marker expressions of differentiated cells and that reatment of neural stem cells with 10ng/ml GMCSF results in a significantly induced expression of beta III-tubulin (n=3; p<0.05, two-tailed t-test) and to a lesser extend of NSE, a marker for mature neurons (B).

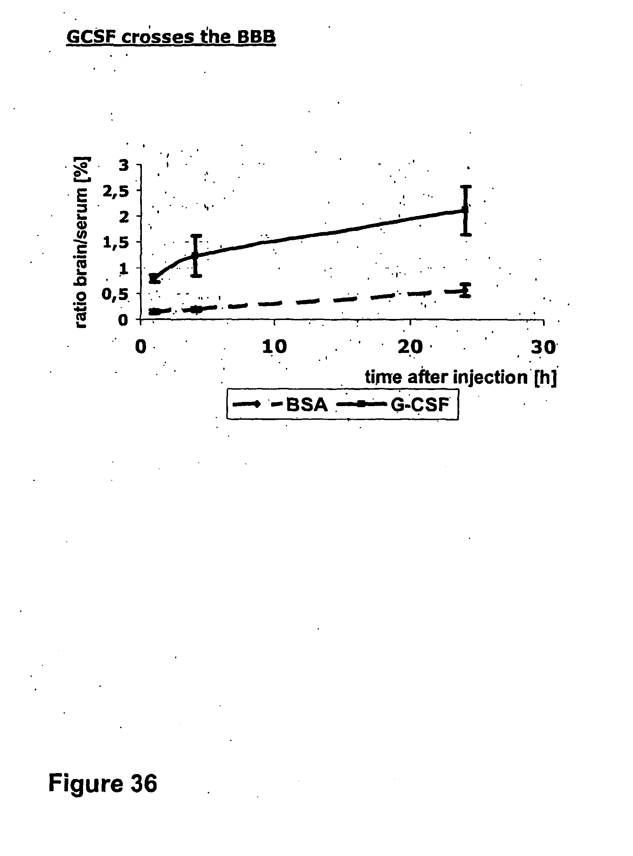

Figure 36 shows that G-CSF passes the blood-brain-barrier (BBB).

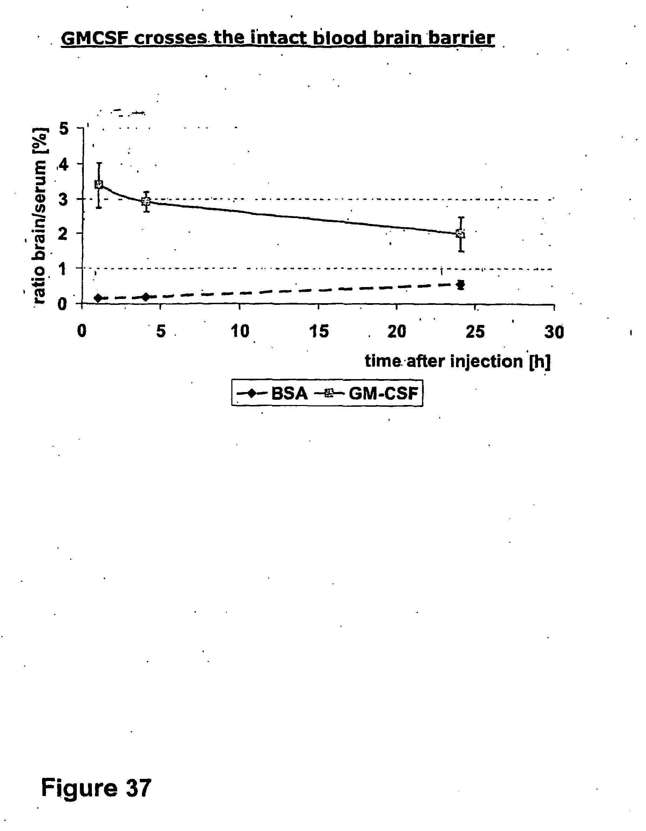

Figure 37 shows that GM-CSF passes the blood-brain-barrier (BBB).

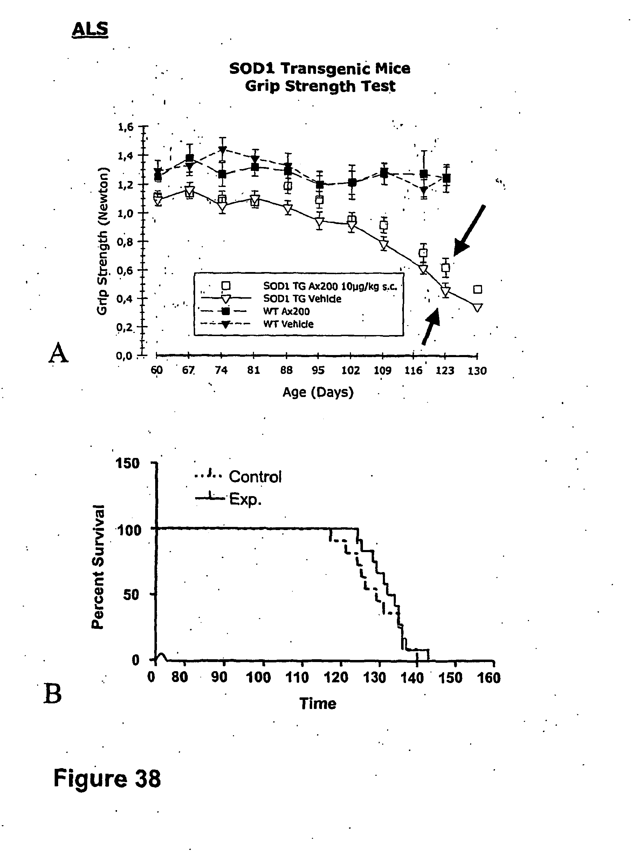

Figure 38 shows the efficacy of G-CSF for the treatment of Amyotrophic Lateral Sclerosis (ALS).

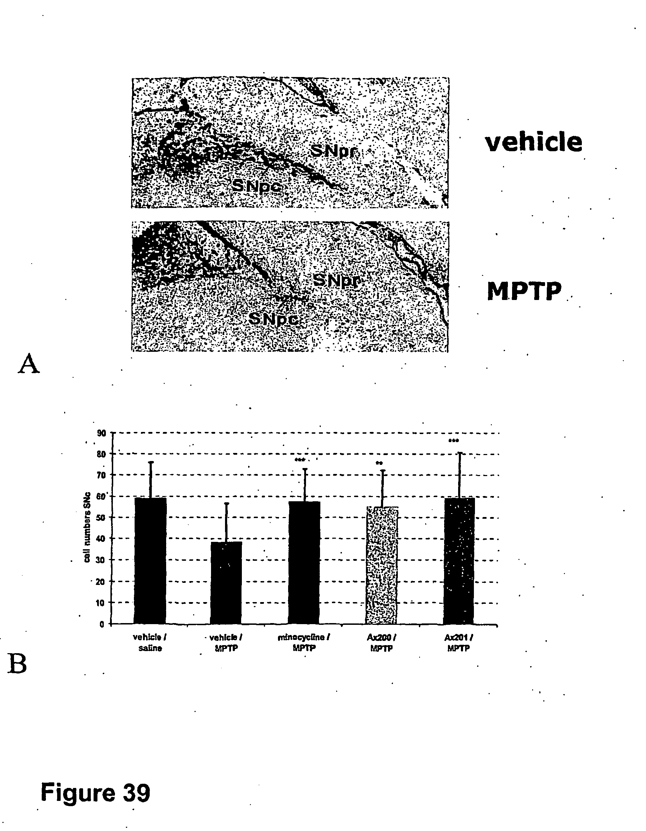

Figure 39 shows the efficacy of GCSF in rodent models of Parkinson's disease (PD).

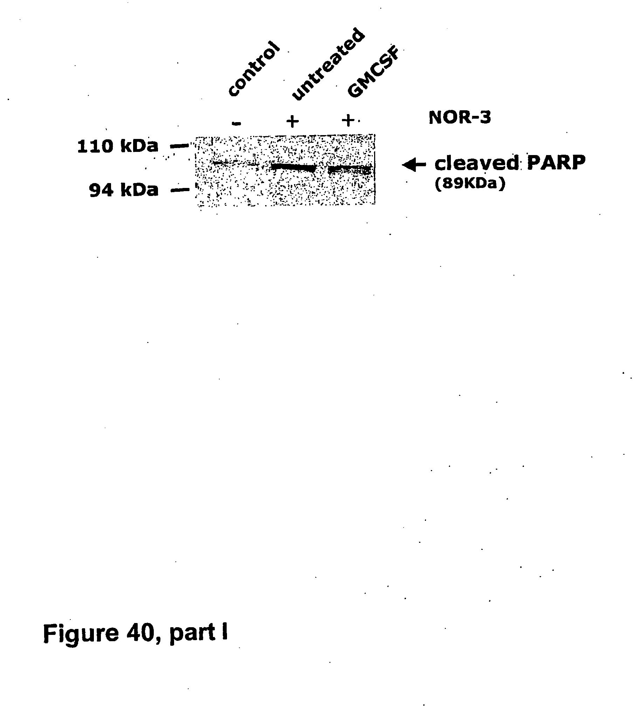

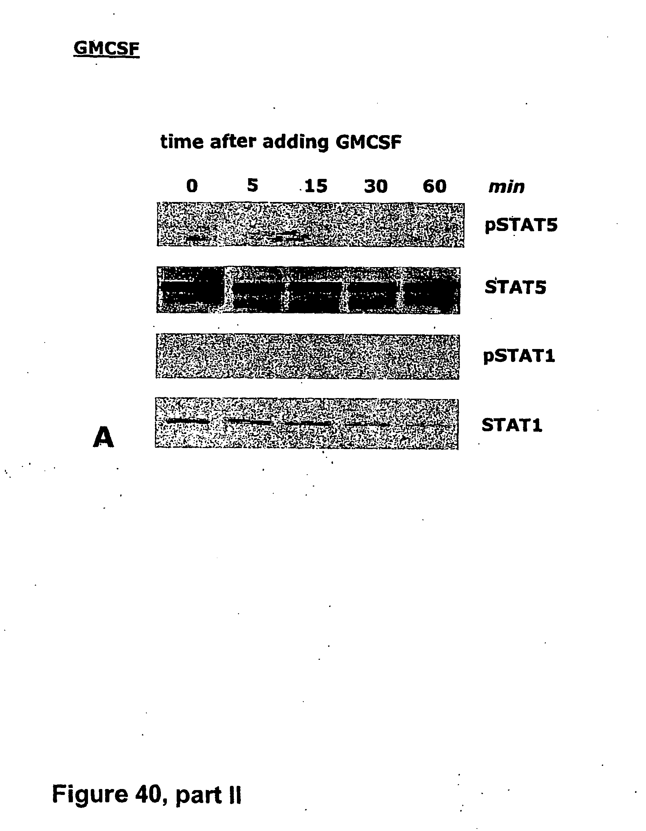

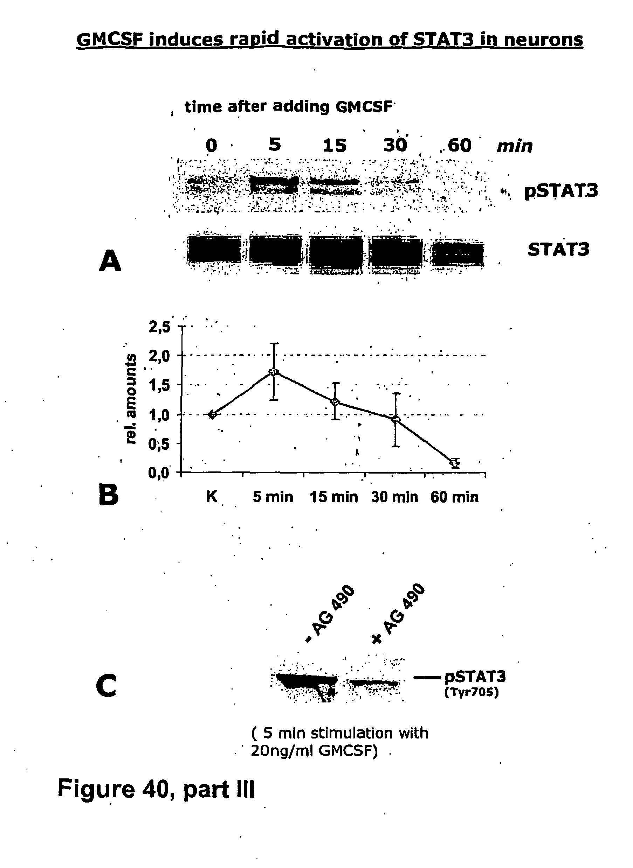

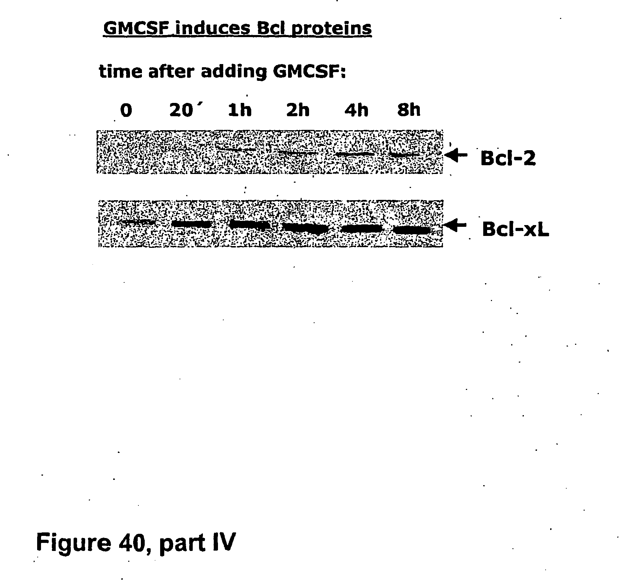

Figure 40 shows that GMCSF acts anti-apoptotically in neurons by activating stat3 pathways, part I shows that GMCSF inhibits PARP cleavage, that is specifically occurring as a sign of apoptosis. Cell death was induced in primary neurons by using the NO-donor NOR-3. Part II shows that GMCSF does not lead to increased phosphorylation of STAT1 ("pSTAT1") or STAT5 ("pSTAT5"), although the proteins themselves are expressed in neurons. part III A shows that GMCSF leads to a time-dependent activation of STAT3, that is maximal at 5 min following the GMCSF stimulus. B, quantification of three independent experiments. C. Activation of STAT3 by phosphorylation can be inhibited by the JAK2 inhibitor AG490. part IV shows that GMCSF strongly induces the expression of the stat3 target genes Bcl2, and BclXI. These genes are known as being antiapoptotic.

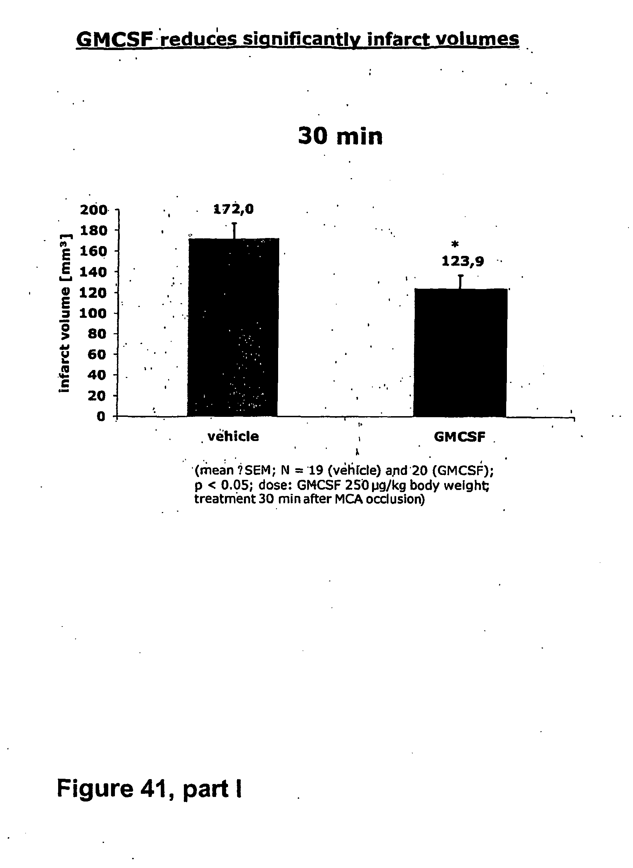

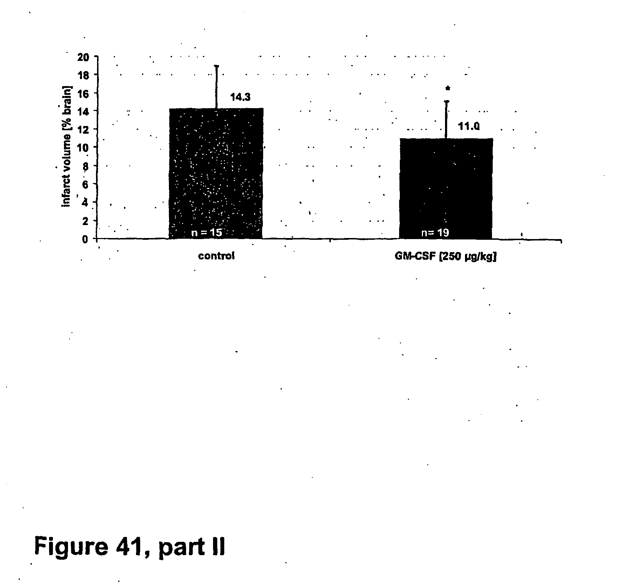

Figure 41 demonstrates that there is a significant reduction of infarct volume in the GMCSF-treated animals, both at the early ( part I), and at the late time window of 3 h (part II).

### DETAILED DESCRIPTION OF THE INVENTION

### GCSF

Granulocyte-colony stimulating factor (GCSF) is a well known growth factor. The GCSF that can be employed in the inventive methods described herein are those full length coding sequences, protein sequences, and the various functional variants, muteins, and mimetics that are known and available. In the discussion that follows these are referred to as GCSF derivatives.

The structure of both the coding DNA and protein are known as well as methods for recombinantly producing mammalian pluripotent granulocyte colony-stimulating factor (WO 87/01132; U.S. Patent 4,810,643). For example, several amino acid sequences corresponding to G-CSF is shown in Figure 10 and the Sequence Listing, i.e., SEQ ID NOS: 28, 29, 30, 31, 32, and 33.

In one embodiment, the proteins that are at least 70%, preferably at least 80%, more preferably at least 90% identical to the full-length human GCSF amino acid sequences described herein can be employed in the present invention, e.g., SEQ ID NO:28. In another embodiment, the GCSF that can be used are those that are encoded by polynucleotide sequence with at least 70%, preferably 80%, more preferably at least 90%, 95%, and 97% identity to the wildtype full-length human GCSF coding sequence, e.g., a polynucleotide encoding SEQ ID NO:28, these polynucleotides will hybridize under stringent conditions to the coding polynucleotide sequence of the wild-type full length human GCSF. The terms "stringent conditions" or "stringent hybridization conditions" includes reference to conditions under which a polynucleotide will hybridize to its target sequence, to a detectably greater degree than other sequences (e.g., at least 2-fold over background). Stringent conditions will be those in which the salt concentration is less than about 1.5 M Na ion, typically about 0.01 to 1.0 M Na ion concentration (or other salts) at pH 7.0 to 8.3 and the temperature is at least about 30°C. for short probes (e.g., 10 to 50 nucleotides) and at least about 60° C. for long probes (e.g., greater than 50 nucleotides), for example, high stringency conditions include hybridization in 50% formamide, 1 M NaCl, 1% SDS at 37°C., and a wash in 0.1X SSC at 60 to 65°C (see Tijssen, Laboratory Techniques in Biochemistry and Molecular Biology-Hybridization with Nucleic Acid Probes, Part I, Chapter 2 "Overview of principles of hybridization and the strategy of nucleic acid probe assays", Elsevier, New York (1993); and Current Protocols in Molecular Biology, Chapter 2, Ausubel, et al., Eds., Greene Publishing and Wiley-Interscience, New York (1995)). Amino acid and polynucleotide identity, homology and/or similarity can be determined using the ClustalW algorithm, MEGALIGN™, Lasergene, Wisconsin)

Examples of the various GCSF functional variants, muteins, and mimetics include functional fragments and variants (e.g., structurally and biologically similar to the wild-type protein and having at least one biologically equivalent domain), chemical derivatives of GCSF (e.g., containing additional chemical moieties, such as polyethyleneglycol and polyethyleneglycol derivatives thereof, and/or glycosylated forms such as Lenogastrim™), and peptidomimetics of GCSF (e.g., a low molecular weight compound that mimics a peptide in structure and/or function (see, e.g., Abell, Advances in Amino Acid Mimetics and Peptidomimetics, London: JAI Press (1997); Gante, Peptidmimetica - massgeschneiderte Enzyminhibitoren Angew. Chem. 106: 1780-1802 (1994); and Olson et al., J. Med. Chem. 36: 3039-3049 (1993)).

Additional examples of GCSF derivatives include a fusion protein of albumin and GCSF (Albugranin™), or other fusion modifications such as those disclosed in US Pat No. 6261250); PEG-GCSF conjugates and other PEGylated forms; those described in WO 00/44785 and Viens et al., J. of Clin. Oncology, Vl., Nr. 1, 2002: 24-36; norleucine analogues of GCSF, those described in US Pat. No. 5,599,690; GCSF mimetics, such as those described in WO 99/61445, WO 99/61446, and Tian et al., Science, Vol. 281, 1998:257-259; GCSF muteins , where single or multiple amino acids have been modified, deleted or inserted, as described in US Patent No. 5,214,132 and 5,218,092; those GCSF derivatives described in US Patent No. 6,261,550 and U.S. Pat. No. 4,810,643; and chimeric molecules, which contain the full sequence or a portion of GCSF in combination with other sequence fragments, e.g. Leridistim--see, for example, Streeter, et al. (2001) Exp. Hematol., 29, 41-50, Monahan, et al. (2001) Exp. Hematol., 29, 416-24. , Hood, et al. (2001) Biochemistry, 40, 13598-606, Farese et al. (2001) Stem Cells, 19, 514-21, Farese, et al. (2001) Stem Cells, 19, 522-33, MacVittie, et al. (2000) Blood, 95, 837-45. Additionally, the GCSF derivatives include those with the cysteines at positions 17, 36, 42, 64, and 74 (of the 174 amino acid species (SEQ ID NO:37) or of those having 175 amino acids, the additional amino acid being an N-terminal methionine(SEQ ID NO:38)) substituted with another amino acid, (such as serine) as described in United States Patent 6,004,548 , GCSF with an alanine in the first (N-terminal) position; the modification of at least one amino group in a polypeptide having GCSF activity as described in EP 0 335 423; GCSF derivatives having an amino acid substituted or deleted in the N-terminal region of the protein as described in EP 0 272 703; derivatives of naturally occurring GCSF having at least one of the biological properties of naturally occurring GCSF and a solution stability of at least 35% at 5 mg/ml in which the derivative has at least Cys¹⁷ of the native sequence replaced by a Ser¹⁷ residue and Asp²⁷ of the native sequence replaced by a Ser²⁷ residue as described in EP 0 459 630; a modified DNA sequence encoding GCSF where the N-terminus is modified for enhanced expression of protein in recombinant host cells, without changing the amino acid sequence of the protein as described in EP 0 459 630; a GCSF which is modified by inactivating at least one yeast KEX2 protease processing site for increased yield in recombinant production using yeast as described in EP 0 243 153; lysine altered proteins as described in U.S. Pat. No. 4,904,584; cysteine altered variants of proteins as described in WO/9012874 (US 5,166,322 ); the addition of amino acids to either terminus of a GCSF molecule for the purpose of aiding in the folding of the molecule after prokaryotic expression as described in AU-A-10948/92; substituting the sequence Leu-Gly-His-Ser-Leu-Gly-Ile (SEQ ID NO:11) at position 50-56 of GCSF with 174 amino acids (SEQ ID NO:37), and position 53 to 59 of the GCSF with 177 amino acids (SEQ ID NO:39), or/and at least one of the four histadine residues at positions 43, 79, 156 and 170 of the mature GCSF with 174 amino acids (SEQ ID NO:37) or at positions 46, 82, 159, or 173 of the mature GCSF with 177 amino acids (SEQ ID NO:39) as described in AU-A-76380/91; and a synthetic GCSF-encoding nucleic acid sequence incorporating restriction sites to facilitate the cassette mutagenesis of selected regions and flanking restriction sites to facilitate the incorporation of the gene into a desired expression system as described in GB 2 213 821. Further examples of G-CSF analogs include SEQ ID NOS:64 and 65, and others described in U.S. patent no. 6,632,426.

The various functional derivatives, variants, muteins and/or mimetics of GCSF preferably retain at least 20%, preferably 50%, more preferably at least 75% and/or most preferably at least 90% of the biological activity of wild-type mammalian GCSF activity-the amount of biological activity include 25%, 30%, 35%, 40%, 45%, 55%, 60%, 65%, 70%, 75%, 80%, 85%, 95%; and all values and subranges there between. Furthermore, the functional derivatives, variants, muteins and/or mimetics of GCSF can also have 100% or more of the biological activity relative to wild-type mammalian GCSF activity-the amount of biological activity including at least 105%, at least 110%, at least 125%, at least 150%, and at least 200%.

To measure the biological activity of GCSF, several known assays can be employed singularly or in combination. One example of determining GCSF function is illustrated in Example 1. Other methods for determining GCSF function are known and include a colony formation assay employing murine bone marrow cells; stimulation of proliferation of bone marrow cells induced by G-CSF; specific bioassays with cells lines that depend on G-CSF for growth or that respond to GCSF (e.g., AML-193 ; 32D ; BaF3 ; GNFS-60 ; HL-60 , M1 ; NFS-60 ; OCI/AML1a ; and WEHI-3B ). These and other assays are described in Braman et al. Am. J. Hematology 39: 194-201 (1992); Clogston CL et al Anal Biochem 202: 375-83 (1992); Hattori K et al Blood 75: 1228-33 (1990); Kuwabara T et al Journal of Pharmacobiodyn 15: 121-9 (1992); Motojima H et al Journal of Immunological Methods 118: 187-92 (1989); Sallerfors B and Olofsson European Journal of Haematology 49: 199-207 (1992); Shorter SC et al Immunology 75: 468-74 (1992); Tanaka H and Kaneko Journal of Pharmacobiodyn. 15: 359-66 (1992); Tie F et al Journal of Immunological Methods 149: 115-20 (1992); Watanabe M et al Anal. Biochem. 195: 38-44 (1991).

In one embodiment, the GCSF is modified or formulated, or is present as a GCSF mimetic that increases its ability to cross the blood-brain barrier, or shift its distribution coefficient towards brain tissue. An example of such a modification is the addition of PTD or TAT sequences (Cao et al. (2002) J. Neurosci. 22:5423-5431; Mi et al. (2000) Mol. Ther. 2:339-347; Morris et al. (2001) Nat Biotechnol 19:1173-1176; Park et al. (2002) J Gen Virol 83:1173-1181). These sequences can also be used in mutated forms, and added with additional amino acids at the amino- or carboxy-terminus of proteins. Also, adding bradykinin, or analogous substances to an intravenous application of any GCSF preparation will support its delivery to the brain, or spinal cord (Emerich et al. (2001) Clin Pharmacokinet 40:105-123; Siegal et al (2002) Clin Pharmacokinet 41:171-186).

### GM-CSF

Granulocyte-macrophage colony stimulating factor (GMCSF) is a well known growth factor (see, e.g., Figure 18, SEQ ID NOS:25, 26, and 27). The GMCSF that can be employed in the inventive methods described herein are those full length coding sequences, protein' sequences, and the various functional variants, chimeric proteins, muteins, and mimetics that are known and available, for example PEGylated forms or albumin-coupled forms. The structure of both the coding DNA and protein are known as well as methods for recombinantly producing mammalian pluripotent granulocyte macrophage colony-stimulating factor (US Patent 5,641,663). The GMCSF receptor is also known and is described, for example, in U.S. Patent 5,629,283.

In one embodiment, the proteins that are at least 70%, preferably at least 80%, more preferably at least 90% identical to the full-length human GMCSF amino acid sequences can be employed in the present invention. In another embodiment, the GMCSF that can be used are those that are encoded by polynucleotide sequence with at least 70%, preferably 80%, more preferably at least 90%, 95%, 97% and 98% identical to the wildtype full-length human GMCSF coding sequence, e.g., a polynucleotide which encodes SEQ ID NO:25, these polynucleotides will hybridize under stringent conditions to the coding polynucleotide sequence of the wild-type full length human GMCSF. The terms "stringent conditions" or "stringent hybridization conditions" includes reference to conditions under which a polynucleotide will hybridize to its target sequence, to a detectably greater degree than other sequences (e.g., at least 2-fold over background). Stringent conditions will be those in which the salt concentration is less than about 1.5 M Na ion, typically about 0.01 to 1.0 M Na ion concentration (or other salts) at pH 7.0 to 8.3 and the temperature is at least about 30°C. for short probes (e.g., 10 to 50 nucleotides) and at least about 60° C. for long probes (e.g., greater than 50 nucleotides), for example, high stringency conditions include hybridization in 50% formamide, 1 M NaCl, 1% SDS at 37°C., and a wash in 0.1X SSC at 60 to 65°C (see Tijssen, Laboratory Techniques in Biochemistry and Molecular Biology--Hybridization with Nucleic Acid Probes, Part I, Chapter 2 "Overview of principles of hybridization and the strategy of nucleic acid probe assays", Elsevier, New York (1993); and Current Protocols in Molecular Biology, Chapter 2, Ausubel, et al., Eds., Greene Publishing and Wiley-Interscience, New York (1995)). Amino acid and polynucleotide identity, homology and/or similarity can be determined using the ClustalW algorithm, MEGALIGN™, Lasergene, Wisconsin)

The various functional derivatives, variants, muteins and/or mimetics of GMCSF preferably retain at least 20%, preferably 50%, more preferably at least 75% and/or most preferably at least 90% of the biological activity of wild-type mammalian GMCSF activity-the amount of biological activity include 25%, 30%, 35%, 40%, 45%, 55%, 60%, 65%, 70%, 75%, 80%, 85%, 95%; and all values and subranges there between. Furthermore, the functional derivatives, variants, muteins and/or mimetics of GMCSF can also have 100% or more of the biological activity relative to wild-type mammalian GMCSF activity-the amount of biological activity including at least 105%, at least 110%, at least 125%, at least 150%, and at least 200%.

For practicing the present invention derivatives of GMCSF, more preferably GMCSF-mimetics, that retain their potential to protect neurons and that also have diminished action on leukocytes, thereby reducing potential adverse effects, are preferred. Derivatives of GMCSF, preferably GMCSF-mimetics, can be tested in an *in vitro* neuroprotective assay, such as described in Example 17. Substances demonstrating a positive neuroprotective effect in this assay can be further tested for their immune-modulatory activity.

To measure the biological activity of GMCSF, several known assays can be employed singularly or in combination. Those GMCSF functions include its known immunmodulatory functions and to one or more functions relating to its role in neuroprotection. Other methods for determining GMCSF function include, for example, in a colony formation assay by the development of colonies containing macrophages, neutrophils, eosinophils, and megakaryocytes; in specific Bioassays with cell lines that depend in their growth on the presence of GM-CSF or that respond to this factor (e.g., cell lines: AML-193 ; B6SUt-A ; BAC1.2F5 ; BCL1 ; Da ; FDCP1 ; GF-D8 ; GM/SO ; IC-2 ; MO7E ; NFS-60 ; PT-18 ; TALL-103 ; TF-1 ; UT-7 ). These and other assays are described in Cebon J et al Blood 72: 1340-7 (1988); Katzen NA et al European Cytokine Network 3: 365-72 (1992); Lewis CE et al Journal of Immunological Methods 127: 51-9 (1990); Mortensen BT et al Experimental Hematology 21: 1366-70 (1993); Oez S et al Experimental Hematology 18: 1108-11 (1990); Roncaroli F et al Journal of Immunological Methods158: 191-6 (1993); Sallerfors B and Olofsson European Journal of Haematology 49: 199-207 (1992); Zenke G et al Journal of Immunoassay 12: 185-206 (1991).

Also preferred are modifications or formulations of GMCSF, or mimetic substances that increase its ability to cross the blood-brain barrier, or shift its distribution coefficient towards brain tissue. An example for such a modification is the addition of Protein transduction domain (PTD) or TAT sequences (Cao G, et al (2002) J. Neurosci. 22:5423-5431; Mi Zet al (2000) Mol. Ther. 2:339-347; Morris et al (2001) Nat Biotechnol 19:1173-1176; Park et al (2002) J Gen Virol 83:1173-1181). These sequences can also be used in mutated forms, and added with additional amino acids at the amino- or carboxyterminus of proteins. Also, adding bradykinin, or analogous substances to an intravenous application of any GMCSF preparation will support its delivery to the brain, or spinal cord (Emerich et al (2001) Clin Pharmacokinet 40:105-123; Siegal et al (2002) Clin Pharmacokinet 41:171-186). Examples for different suitable forms and derivatives are sargramostim (Leukine®, Prokine ®), Leucotropin ® or molgramostim (Leucomax®).

GMCSFR mRNA has been detected in isolated microglia, astrocytes, oligodendrocytes and neurons (Sawada, et al. (1993), Neurosci Lett, 160, 131-4); Baldwin, et al. (1993), Blood, 82, 3279-82. ). It has been shown that GMCSF stimulates the proliferation of astrocytes (Guillemin, et al. (1996), Glia, 16, 71-80). GMCSF also induces the release of interleukin 6 from microglia (Suzumura, et al. (1996), Brain Res, 713, 192-8). Recently, a publication showed that interleukin 1 increased GMCSF protein production in the neuronal cell line NT2 (Dame, et al. (2002), Eur Cytokine Netw, 13, 128-33). The presence of GMCSFR in the brain was shown on material from a human fetus on a systematic search for GMCSF receptor expression in the fetus (Dame, et al. (1999), Pediatr Res, 46, 358-66). Dame et al. conclude from their findings, that GMCSF may have a role in neural development in the fetus, and a role in immunosurveillance in the adult brain. Importantly, they do not mention any possible disease relevance, especially no involvement in neuroprotection. GMCSF augments choline acetyltransferase activity in vitro (SN6.10.2.2 cell line and cultured mouse septal neurons) and in vivo increases survival of lesioned rat cholinergic septal neurons after fimbria-fornix transections (Kamegai, et al. (1990), Brain Res, 532, 323-5. ) (Konishi, et al. (1993), Brain Res, 609, 29-35). Importantly, this finding only pertains to a certain subtype of neurons, and has not been generalized by the authors to other neuronal or neural cell types. They have not derived a general neuroprotective property of GMCSF from these data, nor have they mentioned any possible therapeutic applicability. GMCSF is upregulated in astrocytes upon addition of advanced glycosylation end products (AGEs) (Li, et al. (1998), Mol Med, 4, 46-60). GMCSF has been shown to promote microglia proliferation in vitro (Lee, et al. (1994), Glia, 12, 309-18). Recently, it has been found that GMCSF level were elevated in the cerebrospinal fluid (csf) of patients with Alzheimer's disease (Tarkowski, et al. (2001), Acta Neurol Scand, 103, 166-74). GMCSF has also been studied in the CSF of stroke patients ( Tarkowski, et al. (1999), Stroke, 30, 321-7. ). In the latter study, the level of the FAS ligand, a proapoptotic protein, correlates positively with GMCSF levels in the cerebrospinal fluid of stroke patients at day 21-26 and later than 3 months. However, there is no conclusion drawn to any therapeutic usefulness of this finding in stroke patients, and no mentioning of any possible neuroprotective role of GMCSF. A release of GMCSF after stroke per se can have many reasons, and does not allow any functional prediction to be made. GMCSF crosses the blood-brain-barrier (McLay, et al. (1997), Brain, 120, 2083-91. ). This study has not been performed with the purpose of showing any therapeutic applicability of GMCSF to neurological diseases, and there is no contextual mentioning of a possible usefulness of GMCSF in neurodegenerative diseases or stroke.

In summary, the above mentioned references in the literature provide evidence for presence of the GMCSF receptor and ligand in the nervous system, but do not show any general neuroprotective property of GMCSF. These studies certainly do not imply use of GMCSF for the treatment of neurodegenerative and ischemic disorders such as stroke.

### OTHER HEMATOPOEITIC GROWTH FACTORS

In other embodiments described herein, combinations of GCSF and GMCSF preparations that support its therapeutic actions, preferably its neuroprotective action can be used. The effect exerted by these combinations can be cumulative or superadditive/synergistic. In a one embodiment, various GCSF and/or GMCSF derivatives are used in combination with each other. Likewise, GCSF can be used in combination with one or more additional hematopoietic growth factors such as Erythropoietin, and derivatives thereof, which has recently been shown to mediate strong neuroprotective properties (e.g., Brines et al (2000) Proc Natl Acad Sci USA 97:10526-10531; Cerami et al (2002) Nephrol Dial Transplant 17:8-12; Siren AL, Ehrenreich H (2001) Eur Arch Psychiatry Clin Neurosci 251:179-184.). In addition, GCSF can be used in combination with, for example, various colony stimulating factors (such as M-CSF ), SCF (stem cell factor ), SCPF (stem cell proliferation factor ), various Interleukins (IL1 , IL3, IL4 , IL5 , IL6 , IL11 , IL12 ), LIF , TGF-β, MIP-1-α, TNF-α , and also many other low molecular weight factors.

In another embodiment, GCSF can be combined with substances that have thrombolytic activities, e.g., tissue-plasminogen activator (TPA), streptokinase, urokinase, and/or Ancrod. GCSF can also be combined with either acetylsalicylic acid (Aspirin) or Heparin; with Melatonin; and/or substances that interfere with apoptotic signaling (e.g. inhibitors of caspases). Also, combinations of GCSF with Riluzole (Rilutek®), vitamins such as vitamin E, Q10, or antioxidative substances are possible. Other substances that may be combined with G-CSF include bFGF, anti-GPIIb/IIa, anti-ICAM, nitric oxide inhibitors, thrombolytic agents, rotamas inhibitors, calcineurin inhibitors, and cyclosporin. In addition, when treating neurodegenerative disorders the administration of one or more agents effecting neurotransmitter levels can also be included. Further, when treating cognitive conditions, one or more cholinergic agents, catecholamine reuptake inhibitors and the like may also be administered.

Combinations with existing antidepressants may also be used where advantageous, e.g., to treat depression in the individual. Non-limiting examples of antidepressants include SSRI's (selective serotonin reuptake inhibitors): Paxil (paroxetine); Prozac (fluoxetine); Zoloft (sertraline); Celexa (citalopram); Lexapro (escitalopram oxalate); Luvox (fluvoxamine), MOAI's (monoamine oxidase inhibitors): Nardil (phenelzine); Parnate (tranylcypromine); TCA's (tricyclic antidepressants) Adapin (doxepin); Anafranil (clomipramine); Elavil (amitriptyline); Endep (amitriptyline); Ludiomil (maprotiline); Norpramin (desipramine); Pamelor (nortryptyline); Pertofrane (desipramine); Sinequan (doxepin); Surmontil (trimipramine); Tofranil (imipramine); Vivactil (protriptyline), or other drug types: Effexor (venlafaxine); Cymbalta (duloxetine); Desyrel (trazodone); Buspar (buspirone); Edronax, Vestra (reboxetine); Remeron (mirtazapine); Serzone ( nefazodone); Wellbutrin (bupropion).

### NEUROLOGICAL CONDITION

Neurological conditions can be generally classified into three classes: those disease with ischemic or hypoxic mechanisms; neurodegenerative diseases (see Adams et al, Principles of Neurology, 1997, 6th Ed., New York, pp 1048 ff); and neurological and psychiatric diseases associated with neural cell death. Other neurological conditions also include enhancing cognitive ability and the treatment of brain tumors, such as glioblastomas, astrocytomas, meningiomas, and neurinomas.

Diseases with ischemic or hypoxic mechanisms can be further subclassified into general diseases and cerebral ischemia. Examples of such general diseases involving ischemic or hypoxic mechanisms include myocardial infarction, cardiac insufficiency, cardiac failure, congestive heart failure, myocarditis, pericarditis, perimyocarditis, coronary heart disease (stenosis of coronary arteries), angina pectoris, congenital heart disease, shock, ischemia of extremities, stenosis of renal arteries, diabetic retinopathy, thrombosis associated with malaria, artificial heart valves, anemias, hypersplenic syndrome, emphysema, lung fibrosis, and pulmonary edema. Examples of cerebral ischemia disease include stroke (as well as hemorrhagic stroke), cerebral microangiopathy (small vessel disease), intrapartal cerebral ischemia, cerebral ischemia during/after cardiac arrest or resuscitation, cerebral ischemia due to intraoperative problems, cerebral ischemia during carotid surgery, chronic cerebral ischemia due to stenosis of blood-supplying arteries to the brain, sinus thrombosis or thrombosis of cerebral veins, cerebral vessel malformations, and diabetic retinopathy.

Examples of neurodegenerative diseases include amyotrophic lateral sclerosis (ALS), Parkinson's disease, Huntington's disease, Wilson's disease, multi-system atrophy, Alzheimer's disease, Pick's disease, Lewy-body disease, Hallervorden-Spatz disease, torsion dystonia, hereditary sensorimotor neuropathies (HMSN), Gerstmann-Sträussler-Schanker disease, Creutzfeld-Jakob-disease, Machado-Joseph disease, Friedreich ataxia, Non-Friedreich ataxias, Gilles de la Tourette syndrome, familial tremors, olivopontocerebellar degenerations, paraneoplastic cerebral syndromes, hereditary spastic paraplegias, hereditary optic neuropathy (Leber), retinitis pigmentosa, Stargardt disease, and Kearns-Sayre syndrome.

Examples of neurological and psychiatric diseases associated with neural cell death include septic shock, intracerebral bleeding, subarachnoidal hemorrhage, multiinfarct dementia, inflammatory diseases (such as vasculitis, multiple sclerosis, and Guillain-Barre-syndrome), neurotrauma (such as spinal cord trauma, and brain trauma), peripheral neuropathies, polyneuropathies, epilepsies, schizophrenia, depression, metabolic encephalopathies, and infections of the central nervous system (viral, bacterial, fungal).

By "treating" is meant the slowing, interrupting, arresting or stopping of the progression of the disease or condition and does not necessarily require the complete elimination of all disease symptoms and signs. "Preventing" is intended to include the prophylaxis of the neurological disease, wherein "prophylaxis" is understood to be any degree of inhibition of the time of onset or severity of signs or symptoms of the disease or condition, including, but not limited to, the complete prevention of the disease or condition.

Since strong expression of the GCSF receptor, and the GMCSF receptor on the large motor neurons in the spinal cord was found (see Figure 4 i-l), and GCSF is effective in focal cerebral ischemia (see Figure 1), where the same basic pathogenetic mechanisms are operative like in ALS and other neurodegenerative diseases, such as glutamate involvement, oxidative stress, and programmed cell death GCSF is especially suited for long-term therapy in a chronic condition such as ALS, since it is well-tolerated in humans when given chronically (Ozer et al. (2000), J. Clin. Oncol., 18, 3558-85). Accordingly, GCSF alone, in combination with GMCSF, and/or in combination with one or more additional factors can be used to treat ALS according to the present invention.

The pathophysiology associated with Parkinson's disease, such as the involvement of oxidative stress and apoptosis also places Parkinson's disease amongst the other neurodegenerative disorders and stroke. GCSF is strongly neuroprotective in H₂O₂-invoked cell death in the cell line PC12 (Figure 1b). PC12 cells display features of dopaminergic cells, for example presence of a functional dopamine transporter (Maruyama, et al. (2001), Arch Toxicol, 75, 209-13), and are used as in vitro models for important aspects of Parkinson's disease, for example toxicity by the MPTP metabolite MPP+ (Bai, et al. (2002), Neurosci Lett, 321, 81-4). H₂O₂ is a noxious stimulus for PC12 cells that clearly models some aspects of Parkinson's disease in PC 12 cells. For example, H₂O₂ is one of the intermediates of MPTP-evoked cellular events (Fabre et al 1999 J Physiol Biochem 55(4):325-31). There is a remarkable overlap in the cascade of cellular events and signaling mechanisms involved in MPTP- and H₂O₂-mediated cell death in dopaminergic neurons (Chun, HS, et al., J Neurochem 2001 Feb;76(4):1010-21; Lai et al., Biochem Pharmacol 1993 Feb 24;45(4):927-33). H₂O₂ acts by producing reactive oxygen species (ROS) that lead to oxidative stress and apoptosis. Damage by oxygen radicals is one of the main pathophysiological events in Parkinson's disease (Bonnet and Houeto (1999), Biomed Pharmacother, 53, 117-21., Beal (2001), Nat Rev Neurosci, 2, 325-34), and antioxidant therapy is effective in patients (Beal (2002), Free Radic Res, 36, 455-60. , Shults, et al. (2002), Arch Neurol, 59, 1541-50). Therefore, efficacy of GCSF in H₂O₂ evoked cell death in PC12 cells, a model for the important pathophysiological mechanisms of oxidative stress and apoptosis predicts efficacy in Parkinson's disease (PD): Surprisingly, the expression of the GCSF receptor in the area affected by Parkinson's disease, the substantia nigra (SN), in particular the substantia nigra pars compacta (SNC) (see Figure 4 m-o) was demonstrated. Efficacy in a cellular model, *in vivo* localization of receptors, and overlap of pathophysiological mechanisms with cerebral ischemia (oxygen radicals/ apoptosis) provides compelling evidence that GCSF and/or other growth factors, for example, GMCSF, can be used to treat Parkinson's disease. Efficacy testing in rodent models can be performed as exemplified in Example 5.

The inventors have shown, by the discussion contained herein, that GCSF and GMCSF have the ability to enhance neurogenesis, and improve behavioural outcome after an ischemic lesion. Neurogenesis is one mechanism that can lead to increased plasticity of neural networks, and can replace gradual loss of neurons. Therefore, one embodiment described herein is to provide enhancement, improvement or an increase in cognitive ability to an individual suffering from, displaying, and/or believed to some level of cognitive loss by administering one or more compositions as described herein to the individual in accordance with the administration discussion herein. In an alternative embodiment, cognitive enhancement may also benefit those indidivuals even useful under non-pathological conditions, e.g,, those individuals who do not present with cognitive impairment. Determining cognitive ability and therefore enhancement is known by one of skill in the art. Where increases or enhancement of cognitive ability are measured, they are compared before administration of the compositions of the invention and after the administration (and can also be measured during the administration in some embodiments) using the same test, e.g., with same criteria, parameters, etc.

In addition, the use of compositions according to the invention in cognition enhancement is not limited to a non-pathological decline in mental capacity, but can also be applied to boosting the normal, physiological repertoire of mental capabilities, for example memory enhancement, enhancement of fine motor coordination, and/or enhancement of logical capabilities.

Depression is characterized by sadness, loss of interest in activities, and decreased energy. Other symptoms include loss of confidence and self-esteem, inappropriate guilt, thoughts of death and suicide, diminished concentration, and disturbance of sleep and appetite. A variety of somatic symptoms may also be present. Though depressive feelings are common, especially after experiencing setbacks in life, depressive disorder is diagnosed only when the symptoms reach a threshold and last at least two weeks. Depression can vary in severity from mild to very severe.

Depression can affect individuals at any stage of the life span, although the incidence is highest in the middle ages. There is, however, an increasing recognition of depression during adolescence and young adulthood (Lewinsohn, et al. (1993), J Abnorm Psychol, 102, 110-20). Depression is essentially an episodic recurring disorder, each episode lasting usually from a few months to a few years, with a normal period in between. In about 20% of cases, however, depression follows a chronic course with no remission (Thornicroft and Sartorius (1993), Psychol Med, 23, 1023-32), especially when adequate treatment is not available. The recurrence rate for those who recover from the first episode is around 35% within 2 years and about 60% at 12 years. The recurrence rate is higher in those who are more than 45 years of age. One of the particularly tragic outcomes of a depressive disorder is suicide. Around 15-20% of depressive patients end their lives by committing suicide. Suicide remains one of the common and avoidable outcomes of depression. To summarize, depression is a common mental disorder, causing a very high level of disease burden, and is expected to show a rising trend during the coming 20 years.

Depression can harm a patient constantly (unipolar) or have a bipolar character (manic or bipolar depression). The bipolar depression is marked by extreme mood swings, from "highs" of excessive energy and elation to "lows" of utter despair and lethargy. Manic depression is often treated with Lithium, which evens out the mood swings.

The depression can be a Seasonal Affective Disorder (SAD). It is a type of depression which generally coincides with the approach of winter, starting with September and lasting until Spring brings longer days and more sunshine.

The depression can be a postnatal depression that can occurs from about 2 weeks and up to 2 years after the birth.

Attempts have been made to classify the severity of a depression, eg. (Abdel-Khalek (2003), Psychol Rep, 93, 544-60) identified the following eight basic dimensions i.e., Pessimism, Weak Concentration, Sleep Problems, Anhedonia, Fatigue, Loneliness, Low Self-esteem, and Somatic Complaints to define the profile of children's and adolescents' depression.

Depression can occur as an idiopathic disease (with no somatic disease associated with it), or it can be a psychatric symptom of a somatic disorder, especially a number of neurodegenerative disorders. Depressive disorders (DDs) are frequent psychiatric comorbidities of neurological disorders like multiple sclerosis, stroke, dementia, migraine, Parkinson's disease, and epilepsy. The clinical manifestations of DDs in these neurological disorders are identical to those of idiopathic mood disorders. Neurodegenerative disorders often exhibit "classical" psychiatric symptoms as an initial presentation of the disease. The symptomatology of depression in the context of a neurodegenerative disorder may differ from depression alone. In the following some neurodegenerative disorders that have depression as a symptom are listed:
1. Multiple Sclerosis
2. Traumatic brain injury: Depression also affects patients with traumatic brain injury (TBI): Between 30% and 38% of patients with TBI can be classified as depressed (Seel and Kreuzer 03)
3. Stroke: Approximately 30% of stroke patients are clinically depressed. (Williams 86)
4. Dementia and Alzheimers disease
5. Migraine
6. Parkinsons disease: Depressive symptoms occur in approximately half of PD patients and are a significant cause of functional impairment for PD patients. There is accumulating evidence suggesting that depression in PD is secondary to the underlying neuroanatomical degeneration, rather than simply a reaction to the psychosocial stress and disability. The incidence of depression is correlated with changes in central serotonergic function and neurodegeneration of specific cortical and subcortical pathways. Understanding comorbid depression in PD may therefore add to the understanding of the neuroanatomical basis of melancholia.
7. Epilepsy: Epilepsy is a chronic condition that has complex effects on social, vocational, and psychological function. Several psychiatric disorders have been shown to have increased prevalence in persons with epilepsy compared to the general population. Depression appears to be the most common psychiatric comorbidity, but anxiety and other diagnoses have not been extensively investigated. In epilepsy, however, Depressive Disorders can frequently also present with clinical characteristics that differ from those of idiopathic depression and fail to meet the criteria included in the Diagnostic and Statistical Manual of Psychiatric Disorders-Fourth Edition. Several studies have found that depression or psychological distress may be the strongest predictors of health-related quality of life, even including seizure frequency and severity, employment, or driving status (Gilliam, et al. (2003), Epilepsy Behav, 4 Suppl 4, 26-30).Clinically depressed people with epilepsy reported higher levels of perceived severity and bother from seizures, as well as greater problems with overall seizure recovery than did nondepressed people experiencing similar types of seizures. The pervasive influence of depressive symptoms on reports of seizure activity suggests that people with epilepsy should be screened for depression. These data highlight the importance of detecting and treating depression among people with epilepsy (Gilliam, Hecimovic, and Sheline (2003), Epilepsy Behav, 4 Suppl 4, 26-30).
8. Huntington's disease: Huntington's disease (HD) is a neurodegenerative process that is manifest as deterioration in a person's motor control, cognition and emotional stability. Emotional instability is reflected through a variety of symptoms such as personality change, anxiety and irritability. Cognitive decline may precede motor symptoms in Huntington's disease (HD). Depression is common in HD and has also been linked to cognitive impairment. Depressed mood and estimated time to disease onset, calculated by using DNA mutation length, both were significant predictors of working memory performance. Findings are consistent with and contribute to existing research with individuals presymptomatic for HD, identifying a potentially remediable contribution to cognitive decline (i.e., depressed mood) (Nehl, et al. (2001), J Neuropsychiatry Clin Neurosci, 13, 342-6). Depression may also occur in relation to other somatic diseases not listed here.

Recently, new exciting progress has been made towards the pathogenesis of depression that implies that depression is linked to neurodegenerative events in brain structures, and to the possible failure of correct adult neurogenesis in hippocampal structures. Therefore, hematopoietic growth factor, e.g., G-CSF and/or GM-CSF, treatment with both its anti-apoptotic actions and pro-regenerative actions (including stimulation of endogenous adult neural stem cells) as described herein may be used to treat depression.

The following describes research related to the neurodegenerative concept of depression (Reviewed by Kempermann and Kronenberg (2003), Biol Psychiatry, 54, 499-503).

The study of morphological alterations of depressive patients has revealed structural changes in the hippocampus including grey matter changes (Sheline (2000), Biol Psychiatry, 48, 791-800). Studies of early-onset recurrent depression, late life depression associated with neurologic disorders, and bipolar illness have revealed structural brain changes within a neuroanatomical circuit. This circuit has been termed the limbic-cortical-striatal-pallidal-thalamic tract and is comprised of structures which are extensively interconnected. In three-dimensional magnetic resonance imaging studies of affective illness, many of the structures that comprise this tract have been found to have volume loss or structural abnormalities. Mechanisms proposed to explain volume loss in depression include neurotoxicity caused neuronal loss, decreased neurogenesis, and loss of plasticity. These aspects can all be treated by activities of GCSF or GMCSF. One favoured hypothesis is a disturbance in adult hippocampal neurogenesis (Kempermann and Kronenberg (2003), Biol Psychiatry, 54, 499-503, Jacobs, et al. (2000), Mol Psychiatry, 5, 262-9, Jacobs (2002), Brain Behav Immun, 16, 602-9). Depression and other mood disorders are therefore 'stem cell disorders'. Recent research brought up new aspects on how adult hippocampal neurogenesis is regulated. One of the factors that potently suppresses adult neurogenesis is stress, probably due to increased glucocorticoid release. (Jacobs, Praag and Gage (2000), Mol Psychiatry, 5, 262-9).

Adult hippocampal neurogenesis was first described in 1965 by Altman and Das, and underwent several rediscoveries. A broader interest in the phenomenon was first sparked in the early 1980s by reports on activity correlated neurogenesis in the brain of adult canaries. Marking proliferating cells in the brain with bromodeoxyuridine (BrdU) in combination with confocal laser scanning microscopy, instead of the more cumbersome use of tritiated thymidine and autoradiographic techniques, allowed a more straightforward quantitative approach to adult neurogenesis. A subset of the new cells survives, migrates into the granule cell layer and differentiates into neurons. They establish axonal projections to area CA3 along the mossy fiber tracts, as do all other granule cells in the granule cell layer, and become virtually indistinguishable from the surrounding older cells.

The finding that chronic antidepressive treatment alleviates the decrease in adult neurogenesis strengthens the hypothesis outlined above (Benninghoff, et al. (2002), J Neural Transm, 109, 947-62, Dremencov, et al. (2003), Prog Neuropsychopharmacol Biol Psychiatry, 27, 729-39, D'Sa and Duman (2002), Bipolar Disord, 4, 183-94, Duman, et al. (2001), J Pharmacol Exp Ther, 299, 401-7, Duman, et al. (1999), Biol Psychiatry, 46, 1181-91).

Therefore, G-CSF alone or in combination wire GM-CSF, in combination with additional factors as described herein, could be used to treat depression and/or provide prophylactic depression therapy, by for example, administration to a patient predisposed to depression or expected to develop depression symptoms.

Depression can be treated with formulations of GCSF alone or in combination wire GMCSF with longer plasma half-lives, such as described hereinabove, for example PEGylated forms (PEGfilgrastim), albumin-coupled forms (albugranin). Treatment will not be limited to idiopathic depression, but used also for symptomatic depressions, and depression as a co-morbidity. In another embodiment, the treatment can provide weekly subcutaneous injections and which may be combined with orally available agonists of GCSF or GMCSF receptors in the brain. Treatment doses can be as described hereinabove, and in one preferred embodiment, can be from 0.1 to 1000 µg/kg body weight GCSF or GMCSF daily. The similar neurochemical milieu around the ischemic core and the site of trauma, along with similarly altered gene transcription suggest that similar neuroprotective strategies, aimed at interference with harmful mechanisms should be effective in cerebral ischemia and traumatic brain injury. The goal of such therapy in both types of injuries is to minimize activating toxic pathways and to enhance activity of endogenous neuroprotective mechanisms as the balance between these pathways will eventually determine.the fate of the tissue at risk. Indeed, most neuroprotectants found to be effective in models of experimental stroke are also effective in models of experimental traumatic brain injury (TBI).

In light of the common pathological and protective processes active in cerebral ischemia and traumatic brain injury, as well as, a common response to neuroprotective strategies indicates that GCSF therapy will be effective in traumatic brain injury. There has been one study that examined GCSF under conditions of traumatic brain injury *(*Heard, et al. (1998), Crit. Care Med , 26, 748-54). However this study did not aim at any neuroprotective effects of GCSF (filgrastim), but merely reducing infection parameters (primary endpoints of the study: increase in absolute neutrophil count, safety of filgrastim, and frequency of nosocomial infections (pneumonia, bacteremia, and urinary tract infection)). There was no improvement of mortality in that study. In the context of clinical safety, this study demonstrated that GCSF administration is safe for TBI, confirming the safe practicability of GCSF treatment for neuroprotection according to the present invention.

Since the basic pathophysiological mechanisms operative in cerebral ischemia due to cardiac failure and resuscitation are comparable to those occurring under cerebral ischemia due to occlusion of blood vessels (see Example 1), GCSF therapy will also be effective under conditions of cardiac problems for neuroprotection. Therapy can be started as soon as emergency resuscitation is started. Alternatively, in patients belonging to known risk groups for cardiac problems (prior myocardial infarction, high blood cholesterol levels, high blood pressure, diabetes, smoking), a prophylactic continued therapy with the hematopoietic growth factors, for example, GCSF, can also be performed, e.g. using a slow release form of the factor(s).

Likewise, these above considerations apply to the large group of patients that undergo surgery with subsequent cerebral ischemia. In particular, cardiac surgery (Hogue et al. (1999), Circulation, 100, 642-7), and surgery on the large blood vessels supplying the brain (e.g. carotid endarterectomies) have a high risk of neurological complications associated with them. An objective, retrospective review of 358 carotid endarterectomies performed in the neurosurgical teaching units of the University of Toronto in the year 1982 demonstrated a perioperative stroke rate of 3.9% and a death rate of 1.5%. Most (82%) surgical neurological complications occurred after the immediate post-operative period (24 hours). This high incidence of delayed stroke suggests that most perioperative strokes are embolic rather than hemodynamic. A 5-6% combined morbidity and mortality should be expected in carotid endarterectomy (Group (1986), Stroke, 17, 848-52). These and other data demonstrate a clear need for a prophylactic neuroprotective therapy in these procedures.

It is further described that the hematopoeitic growth factors, for example, such as GCSF and GMCSF, alone, in combination with each other, and/or in combination with one or more additional factors can be used to treat multiple sclerosis (MS) and/or provide prophylactic neuroprotective therapy in multiple sclerosis patients. This method is based on the presence of the GCSF receptor on oligodendrocytes, supporting a direct efficacy of GCSF on the primary target cells of the MS. In addition, the GCSF receptor is present on nerve cells and their processes, which are compromised at later stages of the disease, and could correlate with lasting disabilities (Cid, et al. (2002), J Neurol Sci, 193, 103-9). Indeed, recently a paradigm shift in therapeutic concepts for multiple sclerosis from immunomodulation to neuroprotection has occured, as it appears that neurodegenerative mechanisms are most important for the disabling aspects of multiple sclerosis (Bo, et al. (2003), Mult Scler, 9, 323-31, Bjartmar, et al. (2003), J Neurol Sci, 206,165-71, Wujek, et al. (2002), J Neuropathol Exp Neurol, 61, 23-32, Bjartmar, et al. (2001), Neurology, 57, 1248-52, Peterson, et al. (2001), Ann Neurol, 50, 389-400, Bjartmar and Trapp (2001), Curr Opin Neurol, 14, 271-8, Bjartmar, et al. (2000), Ann Neurol, 48, 893-901, Bjartmar, et al. (1999), J Neurocytol, 28, 383-95, Trapp, et al. (1999), Curr Opin Neurol, 12, 295-302, Trapp, et al. (1998), N Engl J Med, 338, 278-85, Neuhaus, et al. (2003). Trends Pharmacol Sci, 24, 131-8, Pryce, et al. (2003), Brain, 126, 2191-202, Waubant (2003), Expert Opin Emerg Drugs, 8, 145-61, Graumann, et al. (2003), Brain Pathol, 13, 554-73, Golde, et al. (2003), Eur J Neurosci, 18, 2527-37). Even areas in the brain that appear normal with regard to white matter changes show signs of neurodegeneration. Axonal pathology and neurodegeneration therefore are important therapeutic targets in Multiple Sclerosis. GCSF and GMCSF with their anti-apoptotic activity in neurons, as shown in the present invention, and their pro-regenerative potential (by enhancing neurogenesis and plasticity) supports that the compositions described herein can be used as new therapies for treating Multiple Sclerosis.

Furthermore, pathophysiological mechanisms in multiple sclerosis overlap with important mechanisms in cerebral ischemia, e.g. the involvement of nitric oxide (Smith, et al. (2001), Ann Neurol, 49, 470-6), and involvement of glutamate excitotoxicity (Pitt, et al. (2000), Nat Med, 6, 67-70). In light of this information, the hematopoeitic growth factors such as GCSF and GMCSF are a novel treatment option for multiple sclerosis and inflammatory brain disorders.

There has been surprising evidence in the recent years of progressive grey matter loss in schizophrenics. This evidence has been primarily provided by novel magnetic resonance imaging techniques. Although neurodegenerative processes in schizophrenia are not understood at the molecular level, neuroprotective treatment in schizophrenia with GCSF and/or GMCSF is a novel approach to this disease.

To test the efficacy of hematopoietic growth factors, such as GCSF, in the protection of primary neurons neurons can be prepared as follows. 10-12 rat cortices can be prepared from embryos of the stage E18 (embryonic day 18). Tissue can be dissociated using trypsin [10mg/ml] /EDTA /DNase [5mg/ml] (Roche diagnostics, Mannheim, Germany) in HBSS (Hanks balanced salt solution, BioWithakker). The digest can be stopped using 4 parts medium (neurobasalmedium + 1ml 50x B-27 supplement (Invitrogen) + 0.5mM L-glutamine + 25µM glutamate) and can be centrifuged at room temperature for 5 min at 800g. The pellet can be dissolved in 5ml medium and cell number determined by counting (Neubauer slide). The cells can be plated at a density of 250 000 cells per well of a 24-well-plate on cover slips which can be coated with poly-L-lysine. These neurons can then be treated with combinations of a protective stimulus (GCSF) and a noxious stimulus (glutamate, 100 µM). OCSF is applied 30 min prior to treatment of cultures with glutamate. Control groups are treated with either no GCSF (just saline) or no glutamate. After 24 h, neuronal cell death can be determined using the LDH assay (Roche Diagnostics, Mannheim, Germany), following the manufacturers recommendations. Alternatively, other noxious stimuli known for inducing cell death can be used, e.g., NMDA and glycine, 3-nitropropionic acid (3-NPA), H₂O_{2,} staurosporine, hypoxia/glucose deprivation, potassium withdrawal, MPP+, Interleukin-1beta, TNFalpha, FAS ligand or others known to be harmful to cells and neurons. Different assays can also be used for assessing cell death or relative cell survival, e.g. the cell-death ELISA (Roche Diagnostics), Annexin/propidium iodide staining followed by a laser-scanning cytometry analysis (Kamentsky (2001), Methods Cell Biol, 63, 51-87 ), Compucyte, Cambridge, Massachusetts), counting of cell nuclei with apoptotic features following DAPI or HOECHST33342 staining (condensation, fragmentation), counting of cells positive for activated caspase 3 after immunostaining with a cleavage-specific antibody (e.g., Promega caspase 3 antibody), or an assay for caspase3 activity in cell lysates (e.g., ApoOne Assay, Promega; Western blots, Elisas), or any other assay suited for measuring cell survival or apoptotic features. Alternatively , other cells can be used, for example differentiated PC12 cells, HN33 cells, SHSY5 cells, primary hippocampal neurons, primary motor neurons, primary sensory ganglia cells, primary mesencephalic cultures, neuronal stem cells, differentiated ES cells, or other neuron-like cells known in the art, or cells exhibiting one or more neuronal phenotypes. Times and concentrations exemplified here can also be varied, for example, GCSF can be applied concomitantly with a stimulus, or before or after the stimulus. Varying concentrations of GCSF can also be used, e.g. 0.1 - 100 µg/ml. In principal, this assay can also be adapted for the use in brain slice cultures.To test the effectiveness of **hematopoietic growth factors, such as GCSF,** in a model of brain trauma (controlled cortical impact) the following can be performed. Experimental protocols can be approved by the local ethics committee. Twenty male Wistar rats (Charles River, Germany) weighing 280 to 320 g can be randomly assigned to the following groups: A (Control group, n=10, traumatic brain injury (TBI), treatment with 2 ml saline 0.9% for 90 min beginning 30 min TBI); B (GCSF group, n=10, ischemia for 90 min, treatment with 60 µg /kg body weight of recombinant G-CSF, Neupogen^{®}, Amgen, Europe B.V., Netherlands, soluted in 2 ml saline 0.9% for 90 min beginning 30 min TBI); C (sham-operated GCSF-treated control group, n=10, sham operation, treatment with 60 µg /kg body weight of recombinant G-CSF, Neupogen^{®}, Amgen, Europe B.V., Netherlands, soluted in 2 ml saline 0.9% for 90 min beginning 30 min after TBI).

Animals can be anesthetized with an intraperitoneal injection of 100 mg/kg body weight ketaminehydrochloride (WDT, Garbsen, Germany). Anesthesia can be maintained with 50 mg/kg body weight, if necessary. A PE-50 polyethylene tube can be inserted into the right femoral artery for continuous monitoring of mean arterial blood pressure, blood gases, hematocrit, leukocyte count, and blood glucose levels. The right femoral vein can be cannulated by a PE-50 tube for treatment infusion. During the experiment, rectal temperature can be monitored and maintained at 37 °C by a thermostatically controlled heating pad (Föhr Medical Instruments, Germany).

For TBI the skin then can be cut around the probe and the skull exposed and cleaned. TBI can be inflicted using a weight-drop device with indirect impact, modified for compatibility with microdialysis (a weight of 150 g dropped from 40 cm onto a PVC cylinder with a Teflon point of 2.0 mm diameter). Sham operated controls can be identically prepared to rats that received TBI, without the trauma.

In all animals outcome can be measured by mortality, as well as, Neurological Severity Scores (NSS), performed daily for 1 week after traumatic brain injury by an investigator blinded to the experimental groups. Neurological function can be graded on a scale of 0 to 16 (normal score, 0; maximal deficit score, 16). NSS is a composite of motor, sensory, and reflex tests and includes the beam balance test. In the severity scores of injury, 1 score point is awarded for the inability to perform the test or for the lack of a tested reflex; thus, the higher score, the more severe is the injury.

One week after TBI, the rats can be anesthetized with ketamine 150 mg/kg body weight and decapitated. The brains can be removed, and fixed with 4% paraformaldehyde in 0.1 mol/l phosphate buffer for 24 hrs. After paraffin-embedding, 1-µm-thick sections can be cut and used for H&E staining, Nissl staining and immunohistochemical analysis.

Immunohistochemical study can be performed with antisera against myeloperoxidase (DAKO, USA), and G-CSFR (Santa Cruz Biotechnology Inc., USA). Antisera can be generated in rabbits immunized with the isolated human protein (anti-myeloperoxidase) or with a synthetic peptide mapping the carboxy terminus of G-CSFR of mouse origin, respectively. For antigen retrieval, sections provided for G-CSFR immunohistochemistry can be heated for 20 min in a 10 mM citrate buffer at 99°C. Sections can be then incubated in normal swine serum (10% in phosphate-buffered saline) for 30 min and then in the primary antisera overnight at 4°C. The primary antibodies can be diluted 1:150 (myeloperoxidase) 1:400 (G-CSFR). Immunoreactivity can be visualized by the avidin biotin complex method. (Vectastain, Vector Laboratories, USA). Sections can be developed in 0.02% diaminobenzidine (DAB) with 0.02% hydrogen peroxide. The reaction product can be intensified by the addition of 0.02% cobalt chloride and nickel ammonium sulfate. Neuronal survival after TBI can be measured by quantifying neurons under the microscope (magnification x 40) in the hippocampus of G-CSF treated animals and controls. Invasion of neutrophilic granulocytes (NG) can be measured semiquantitatively on a four point scale (0=MPO negative, 1=low MPO expression, 2=moderate MPO expression, 3=strong MPO expression).

### Statistical analysis

Values are displayed as means ± SD. After acquiring all the data, the randomization code can be broken. ANOVA and subsequent post hoc Fisher protected least significant difference test can be used to determine the statistical significance of differences in continuous variables such as physiological parameters. The t-test can be used for comparison of neuronal damage and immunohistochemical data. The Mann-Whitney U test can be performed for nonparametric data such as the mortality rate and MPO immunohistochemistry. A p value <0.05 is considered statistically significant.

Based on the effect of hematopoietic factors, such as GMCSF and GCSF, and the effects of agonizing the cognate receptors on neuronal cells, another embodiment of the present invention is to treat brain tumors or other neurological cancers by antagonizing the GMCSF and/or GCSF receptors on the cancerous cells.

### NEURONAL STEM CELLS

Recently, the importance of forming new nerve cells (neurogenesis) for treating neurological disease has been recognized. Unlike many other tissues, the mature brain has limited regenerative capacity, and its unusual degree of cellular specialization restricts the extent to which residual healthy tissue can assume the function of damaged brain. However, cerebral neurons are derived from precursor cells that persist in the adult brain, so stimulation of endogenous neural precursors in the adult brain could have therapeutic potential.

Neurogenesis occurs in discrete regions of the adult brain, including the rostral subventricular zone (SVZ) of the lateral ventricles and the subgranular zone (SGZ) of the dentate gyrus (DG). Neurogenesis occurs in the adult animal especially after a particular neurological paradigm (e.g. cerebral ischemia (Jin, et al. (2001), Proc. Natl. Acad Sci. USA, 98, 4710-5, Jiang, et al. (2001), Stroke, 32, 1201-7, Kee, et al. (2001), Exp. Brain. Res., 136, 313-20, Perfilieva, et al. (2001), J. Cereb. Blood Flow Metab., 21, 211-7)). Neurogenesis has also been demonstrated in humans (Eriksson, et al. (1998), Nat Med, 4, 1313-7. ), and indeed leads to functional neurons (van Praag, et al. (2002), Nature, 415, 1030-4). In particular, the subgranular zone of the dentate gyrus, and the hilus has the potential to generate new neurons during adult life (Gage, et al. (1998), J Neurobiol, 36,249-66). It is striking that the GCSF Receptor is expressed in this area (Figure 4 a,d). Together with the surprising data demonstrating improvement of functional outcome after GCSF treatment (Figure 8), and the fact that GCSF is a stem cell factor in another system (hematopoesis), it is expected that GCSF exerts part of its actions, especially the long-term effects observed (Figure 8) via its stimulating function on adult stem cells at least in the dentate gyrus.

This is confirmed in the present application by demonstrating the presence of the GCSF receptor on adult neuronal stem cells, isolated from the hippocampal region encompassing the dentate gyrus from rat (Figures 12 and 13). The importance in neurogenesis provides another reason for the applicability and usefulness of GCSF treatment in all facets of neurodegenerative disease, and all conditions where neurons die. In contrast to acting on endogenous stem cells in the brain for the treatment of neurological conditions, GCSF can be applied to *in vitro* manipulations of stem cells, for example differentiation and proliferation. Stem cell therapy in humans is presently being explored for a number of diseases, in particular Parkinson's disease and stroke. It is desirable to differentiate stem cells in culture to particular types of neural cells, e.g., dopaminergic cells for the treatment of Parkinson's disease. Differentiated, or otherwise adapted cells to the new environment, are then administered via different routes to the organism. In Parkinson's disease, for example, stem cells have been injected directly into the brain to substitute for the loss of dopaminergic neurons in the substantia nigra ("replacement therapy")(Arenas (2002), Brain Res. Bull, 57, 795-808, Barker (2002), Mov. Disord., 17,233-41).

Therefore, it is possible to stimulate the growth and differentiation of neuronal stem cells or precondition neuronal stem cells prior to implantation into a mammal using the hematopoietic growth factors and derivatives thereof, and to utilize these neuronal stem cells as means for treating neurological disease as described herein, preferably by providing a neuroprotective effect when the neuronal stem cells are administered to the individual.

In one embodiment, the stem cells can be administered intravenously or intraarterially. It has been shown, for example, in cerebral ischemia or traumatic brain injury, that bone marrow stromal cells injected i.v. find their way to target areas in the brain (Mahmood, et al. (2001), Neurosurgery, 49, 1196-203; discussion 1203-4, Lu, et al. (2001), J Neurotrauma, 18, 813-9, Lu, et al. (2002), Cell Transplant, 11, 275-81, Li et al. (2002), Neurology, 59,514-23). Stem cells may thus be treated by GCSF or GMCSF, or other hematopoetic factors *in vitro,* and then injected via different routes to patients with any of the diseases described herein.

The stern cells that are transplanted can be genetically engineered to express factors that are secreted, and enhance the survival of neighboring cells, or lead to increase proliferation and/or differentiation of adult endogenous stem cells. For example, stem cells may be engineered to stably express GCSF, GMCSF, and/or one or more additional hematopoietic factors; and then be delivered to the central nervous system to constantly secrete GCSF or GMCSF, or other hematopoetic factors to the local environment

Stem cells can be treated with GCSF, GMCSF, and/or other hematopoetic factor receptor agonists. Stem cells that can be used include immortalized stem cells (e.g., oncogene immortalized), neurospheres, and embryonic stem cell (ES)-derived neural cells (Gottlieb (2002), Annu Rev Neurosci, 25, 381-407), but can also include cells like bone marrow stromal cells, or umbilical cord cells (Lu, et al (2002), Cell Transplant, 11, 275-81 , Li et al (2002), Neurology, 59, 514-23. ) Transplantation of stem cells of variable types is a therapeutic possibility in a variety of neurological diseases, including Parkinsons disease (Isacson (2002), Brain Res Bull, 57, 839-46) and stroke (Kondziolka, et al. (2002), J Clin Neurosci, 9, 225-30.).

The stem cells, e.g., human neuronal stem cells, can be treated with factors to condition them prior to transplantation. One example of those conditioning factors is growth factors. One example of conditioning is differentiating them in the direction of desired cells, e.g. neurons.(Svendsen, et al. (1999), Brain Pathol, 9, 499-513). The presence of the GCSF receptor on stem cells indicates the importance of agonists for this receptor for conditioning these cells. Adult neuronal stem cells can be treated with different concentrations of GCSF, or other GCSF receptor agonists, and assayed for increased differentiation potential by a quantitative PCR approach, e.g., by quantifying the ratio of neuronal markers (Map2, NSE (neuron-specific enolase), neurofilament, NeuN) compared to markers of neuronal stem cells (nestin). An increased ratio after treatment signals an increased differentiation of stem cells towards the neuronal lineage. A suited concentration and time window can be used to treat stem cells prior to transplantation for neurological disease.

It is also described that GCSF, GMCSF, derivatives thereof as well as GCSF and GMCSF receptor agonists can be used to facilitate culturing of neural cell, such as, for example, neural stem cells. In this method, the GCSF, GMCSF, derivatives thereof as well as GCSF and GMCSF receptor agonists can be added to the media and premixed before adding to the cells or can be added into the media in which the cells are being cultured. In another embodiment of this method, the neural cells are transfected with a polynucleotide which encoded GCSF, GMCSF, and derivatives thereof, which when transfected express the respective factors in the cell.

### ADMINISTRATION/FORMULATION/DOSAGE

G-CSF, GM-CSF, and the other factors, derivatives, genes, and combinations thereof, may be administered in a variety of dosage forms which include, but are not limited to, liquid solutions or suspensions, tablets, pills, powders, suppositories, polymeric microcapsules or microvesicles, liposomes, and injectable or infusible solutions. The preferred form depends upon the mode of administration and the therapeutic application.

The composition may be in the form of a liquid, slurry, or sterile solid which can be dissolved in a sterile injectable medium before use. The parenteral administration is preferably intravenously. This injection can be via a syringe or comparable means. This may contain a pharmaceutically acceptable carrier. Alternatively, the compositions, e.g. containing G-CSF, may be administered via a mucosal route, in a suitable dose, and in a liquid form. For oral administration, the compositions, e.g., containing G-CSF, can be administered in liquid, or solid form with a suitable carrier.

The mammal to be treated can be, for example, a guinea pig, dog, cat, rat, mouse, horse, cow, sheep, monkey or chimpanzee. In one embodiment, the mammal is a human. Likewise, in one embodiment the hematopoietic factors, such as GCSF and GMCSF used for therapy or prophylaxis is a human factor or derived from a human source.

A therapeutically effective amount of the hematopoeitic factors for use in the methods of treating neurological disease when the factors are used either singularly or in combination should be used in an amount that results in a neuroprotective effect. Such an amount can range from about 100 ng to about 10mg/kg body weight per factor or as a combination and can be determined based on age, race, sex, and other factors based on the individual patient. For example, an amount of GCSF for use in the present methods would be from about 5 to about 60 µg/kg and for GMCSF from about 5 to about 750 µg/kg body weight. When the factors are administered in combination, they may be premixed prior to administration, administered simultaneously, or administered singly in series.

In certain embodiments of treating neurological conditions as described herein, higher doses of G-CSF and GM-CSF can be especially useful, for example, at least 1 mg, at least 2 mg, or at least 3 mg may be used, a range from about 1 to 3 mg is preferable. These higher doses cannot be conveniently achieved for adult patients by using the currently available packaging units. Therefore, in one embodiment, the present description provides packages, especially for doses in the range of 20 to 250 µg/kg body weight, which may be administered, e.g., intravenously, or sub-cutaneously, for the purpose of neuroprotection according to the description herein. Examples of the packaged units useful for delivery of the high dosage compositions include, for example, single use vials and prefilled syringes containing 0.75 to 25 mg G-CSF and/or GM-CSF at a fill volume of 1.0 ml or 2.0 ml. In a preferred embodiment, the packaged units would also contain instructions, on a label or separate printed material, for the delivery of the composition(s) contained therein.

Preferred are also prefilled syringes for G-CSF and/or GM-CSF administration calibrated with kg/bdy weight scales that allow the exact and fast dosage for each individual patient according to the patient's body weight These packaging units are very useful under emergency settings, for example in the case of stroke, where patients have to be treated very quickly after injury. Accordingly, another embodiment of the present invention is to provide neuroprotection, for example, in a patient who has suffered a stroke or other neurotraumatic event, by administration of one or more composition as described herein a short time after the injury has occurred. In an alternative embodiment, the compositions described herein, such as the prefilled syringes, may also be administered/used anytime after the injury not specifically limited to only a short time after the injury.

As used in the context of this embodiment, "a short time after injury" means within about 10 minutes, 15 minutes, 20 minutes, 25 minutes, 30 minutes or even up to about 1 hour after the occurance of the injury or the event causing the injury. The compositions may be administered by a medical professional or in some instances a non-medical professional who has access to a packaging unit containing the appropriate dosages of factor(s). In the case of a medical professional, such as an ambulance technician, nurse, doctor, etc, the administration may be performed at the location where the patient has suffered a neurotrauma, e.g., such as stroke, in an ambulance or other vehicle while being transported to the hospital (or other medical facility), or in the hospital, such as in the emergency room. Without being limited to theory, the administration of one or more hematopoietic factors, such as G-CSG and/or GM-CSG containing compositions, within a short time after injury, a greater neuroprotective effect may be achieved.

Also described is a device especially suited for slow release and constant long-term application that may be an implanted mini-pump, preferably implanted sub-cutaneously (for example, as described in Edith Mathiowitz; (Ed.), Encyclopedia of Controlled Drug Delivery, John Wiley & Sons vol. 2, pp. 896-920, 1999). Such pumps are known to be useful in insulin therapy. Examples of such pumps include those manufactured/distributed by Animas, Dana Diabecare, Deltec Cozm, Disetronic Switzerland, Medtronic, and Nipro Amigo as well as those described, for example, in U.S. patent nos.5474552; 6558345; 6122536; 5492534; and 6551276.

A device that measures endogenous serum-levels of G-CSF or GM-CSF and upon this basis, taking into account age and body weight, calculates and injects the appropriate amount of drug (for example, as described in Renard E., Curr Opin Pharmacol. 2002 Dec;2(6):708-16 is also described). This can be done on a chronic basis, for example by using the above described mini-pump, or on an acute basis. An example of such a device is produced by the company Medtronic minimet (Medtronic MiniMed, 18000 Devonshire Street, Northridge, CA 91325-1219) for insulin therapy. A way of measuring the GCSF or GMCSF serum level may be accomplished using protein chips or arrays, for example, as described in U.S. patent nos. 6,630,358; 6,537,749; and 6,475,809.

The above embodiments of drug delivery using pumps and/or coupled with serum level detection of GCSF and/or GCCSF can be especially useful for the treatment of chronic neurodegenerative diseases, for example, Parkinson's disease, amyotrophic lateral sclerosis (ALS), dementia, and others.

The route of administration can include the typical routes including, for example, orally, subcutaneously, transdermally, intradermally, rectally, vaginally, intramuscularly, intravenously, intraarterially, by direct injection to the brain, and parenterally. In addition, in some circumstances, pulmonary administration may be useful, e.g., pulmonary sprays and other respirable forms.

In addition to pulmonary sprays, intranasal (IN) delivery (for example by nasal sprays) is a preferred application mode of delivering the compositions of the present invention for neurological / psychiatric conditions. Intranasal delivery is well suited to serve as application mode of proteins and peptides, and is very convenient to use, especially for long-term treatments. Examples for the use of intranasal delivery (nasal sprays) for applying peptides or proteins to the brain can be found in: ( Lyritis and Trovas (2002), Bone, 30, 71S-74S, Dhillo and Bloom (2001), Curr Opin Pharmacol, 1, 651-5, Thorne and Frey (2001), Clin Pharmacokinet, 40, 907-46, Tirucherai, et al. (2001), Expert Opin Biol Ther, 1, 49-66, Jin, et al. (2003), Ann Neurol, 53, 405-9, Lemere, et al. (2002), Neurobiol Aging, 23, 991-1000, Lawrence (2002), Lancet, 359, 1674, Liu, et al. (2001), Neurosci Lett, 308, 91-4). For intranasal application, GCSF / GMCSF and other compositions as described herein can be combined with solvents, detergents and substances that increase penetration of the nasal epithelium or delivery into blood vessels, such as drugs that widen nasal blood vessel, increase perfusion, etcetera.

Based on the mode of administration, and under consideration of the relevant pharmacokinetics involved, the dose may be further modified, e.g., for a direct injection into the brain the dose would be lower, and the amount would be specified in absolute doses, based on local availability of GCSF, GMCSF or their derivative (e.g., 5 µg total dose). Preferably, GCSF, GMCSF and the other hematopoietic factors and derivatives thereof are administered intravenously, subcutaneously, or by direct intracerebral injection, which may be performed with an osmotic pump.

GCSF, GMCSF and the other hematopoietic factors and derivatives thereof can be provided to the individual by administrating one or more nucleic acids that encodes these factors. The coding sequence nucleic acid is preferably administered in the form of a recombinant vector, such as a viral vector. The selection of a suitable vector and expression control sequences as well as vector construction is know. Examples, of viral vectors include an adenovirus (Acsadi et al., Hum. Gene Ther. 7(2): 129-140 (1996); Quantin et al., PNAS USA 89(7): 2581-2584 (1992); and Ragot et al., Nature 361 (6413): 647-650 (1993)), an adeno-associated viral vector (Rabinowitz et al., Curr. Opin. Biotechnol. 9(5): 470-475 (1998)), a retroviral vector (Federico, Curr. Opin. Biotechnol. 10(5): 448-453 (1999)), a *Herpes simplex* viral vector (Latchman, Gene 264(1): 1-9 (2001)), a lentiviral vector, a *Sindbis* viral vector, or a Semliki forest viral vector. Suitable vectors are also liposomes containing proteins that will attach to neural cells, e.g., virus epitopes, and contain either nucleic acid encoding GCSF or GMCSF, or protein, or oligonucleotides. An example of such a transfer system is the HVJ-liposome (Kaneda, et al. (2002), Mol Ther, 6, 219-26. Kaneda (1999), Mol Membr Biol, 16, 119-22.). Preferably, the isolated and purified nucleic acid encoding and expressing the protein or polypeptide is operably linked to a promoter that is suitable for expression in neural cells. These and other suitable vectors are reviewed in Kay et al., Nature Medicine 7: 33-40 (2001); Somia et al., Nature Reviews 1: 91-99 (2000); and van Deutekom et al., Neuromuscular Disorders 8: 135-148 (1998).

Suitable promoters for operable linkage to the isolated and purified nucleic acid are known in the art. Preferably, the isolated and purified nucleic acid encoding the polypeptide is operably linked to a promoter selected from the group consisting of the muscle creatine kinase (MCK) promoter (Jaynes et al., Mol. Cell Biol. 6: 2855-2864 (1986)), the cytomegalovirus (CMV) promoter, a tetracycline/doxycycline-regulatable promoter (Gossen et al., PNAS USA 89: 5547-5551 (1992)).

Generally, to ensure effective transfer of the vectors of the present invention, about 1 to about 5,000 copies of the vector are employed per cell to be contacted, based on an approximate number of cells to be contacted in view of the given route of administration, and it is even more preferred that about 3 to about 300 pfu enter each cell. These viral quantities can be varied according to the need and use whether *in vitro* or *in vivo*. The actual dose and schedule can also vary depending on whether the composition is administered in combination with other compositions, e.g., pharmaceutical compositions, or depending on individual differences in pharmacokinetics, drug disposition, and metabolism. Similarly, amounts can vary in *in vitro* applications depending on the particular type of cell or the means by which the vector is transferred.

The above-described proteins or derivatives thereof, substances or nucleic acids can be formulated for medical purposes according to standard procedures available in the art, e.g., a pharmaceutically acceptable carrier (or excipient) can be added. A carrier or excipient can be a solid, semi-solid or liquid material which can serve as a vehicle or medium for the active ingredient. The proper form and mode of administration can be selected depending on the particular characteristics of the product selected, the disease, or condition to be treated, the stage of the disease or condition, and other relevant circumstances (Remington's Pharmaceutical Sciences, Mack Publishing Co. (1990)). The proportion and nature of the pharmaceutically acceptable carrier or excipient are determined by the solubility and chemical properties of the substance selected the chosen route of administration, and standard pharmaceutical practice. The pharmaceutical preparation may be adapted for oral, parenteral or topical use and may be administered to the patient in the form of tablets, capsules, suppositories, solution, suspensions, or the like. The growth factors, derivatives thereof, a nucleic acid coding sequence thereof of the present invention, while effective themselves, can be formulated and administered as pharmaceutically acceptable salts, such as acid addition salts or base addition salts, for purposes of stability, convenience of crystallization, increased solubility, and the like.

For some neurological diseases, especially in ischemic diseases it is crucial for an effective therapy not to delay the onset of the therapy. In a preferred embodiment, the present invention relates to a method, wherein GCSF, GMCSF, or a derivative thereof or a substance activating STAT proteins or an agonist to the GCSF or GMCSF receptors is administered within 24, preferably within 10, most preferably within 3 to 6 hours after the occlusion of a blood vessel, or the onset of neurological symptoms, or an otherwise detected onset of an ischemic event. As GCSF and GMCSF also have beneficial effects for long-term functional improvement, treatment begins at considerable time after the ischemic insult is also possible (e.g., 1 day to 7 days after the insult). Treatment may be continued for several days and weeks after the ischemic event, as a means to improve functional recovery of the patient in analogy to the functional improvement seen in our long-term cortical infarct model (see Figure 8). Treatment may consist of several doses per day (e.g. short i.v. infusions) to reach steady-state trough levels of GCSF, GMCSF or related factors. Treatment may also include continuous slow infusion of the described substances (e.g., by a perfusor unit) to gurantee steady serum levels.

In the case of chronic neurodegenerative processes, such as Parkinson's disease, or amyotrophic lateral sclerosis, treatment will more likely consist in one daily dose of GCSF or related factors, or preferably use slow-release formulations, or more stable derivatives of GCSF or related factors.

### SCREENING FOR NEUROPROTECTIVE SUBSTANCES WHICH BIND TO A GCSF OR GMCSF RECEPTOR ON NEURAL CELLS

For practicing the present invention, derivatives of GCSF, GMCSF (e.g., GCSF-or GMCSF-mimetics), and/or other hemotopoietic factors that retain their potential to protect neurons and that also have diminished action on leukocytes, thereby reducing potential adverse effects, are preferred. Therefore, one embodiment described herein is to screen for compounds that bind to the GCSF or GM-CSF receptor on neural cells and which stimulate the neuroprotective effect observed with GM-CSF or GCSF as described in this application.

Derivatives of GCSF, e.g., GCSF-mimetics, can be tested in an *in vitro* neuroprotective assay such as exemplified in Example 10. This neuroprotective assay can be varied without changing the basic principle of the test by adapting for, for example, (i) other damaging stimuli like Interleukin-1, oxygen deprivation, A4 peptides, FAS ligand, or (ii) other cells, e.g. neuronal-like cells like SH-SY5Y cells, or PC12 cells, or (iii) other readouts for cell-viability or cell-death such as e.g. DNA-fragmentation, nuclear condensation, activity of co-transfected beta-galactosidase, detection of a fluorigenic substrate of caspases etc.. All these numerous adaptations are known and may also be used in high-throughput systems. High throughput screening technology is commonly used to define the rapid processing of cells on a large scale. Substances demonstrating a positive neuroprotective effect in this assay can be further tested for their granulopoetic activity as, for example, described in Tian et al., Science 281, 257-259. Selection of GCSF derivatives is preferably based on the comparison of these two specific effects elicited by the test substances with those effects elicited by known forms of GCSF. Likewise, derivatives of GMCSF, e.g., GMCSF-mimetics can be tested in an *in vitro* neuroprotective assay as described for GCSF.

Further neuroprotective substances can be obtained by measuring the presence of activated transcription factors such as STAT-proteins, including STAT-3 and STAT-5 in neuronal or neuron-like cells (for example, PC12, SH-SY5Y, hNT, NT2, hn33) after exposure of these cells to a test substance. Therefore, in another embodiment of the present invention, the ability of a particular substance or compound to act as an agonist to the GCSF or GMCSF receptor can be assessed by the activation of the STAT proteins, e.g., STAT3 and/or STAT5, in neuron-like cells as described herein and using conventional gene expression assays, such as quantitative PCR by LightCycler™ or Taqman™ analysis.

Activated STAT proteins are phosphorylated (pSTAT) and can be detected by a commercially available pSTAT-specific antibody (Santa Cruz Biotechnology) in Western Blot or in immunohistochemical studies, or ELISAs.

Alternatively, STAT activity can be measured using a reporter construct which includes a STAT-responsive promoter element (for example, a multimerized STAT-binding element, such as a multimerized STAT-3 or STAT-5-binding element) linked to a reporter gene, such as luciferase, SEAP (secreted alkaline phosphatase), chloramphenicol transferase (CAT), green fluorescent protein (GFP), or other common gene expression reporters. After transfecting cells with reporter construct, the cell is contacted with the test substance and the amount of the reporter expression can be identified. This method of measuring STAT activity can be adapted to a high-throughput format.

A typical reporter assay can be conducted using, for example, commercially available assay systems (Mercury Pathway Profiling System from Clontech; Path Detect-System from Stratagene). An example of a protocol that can be performed is as follows.

Cells are cultured in a multiwell plate, e.g. 20.000 cells per well in a 96 well plate. Two days after seeding the cells culture medium is exchanged to an serum-free medium (40µl per well) and cells can be transfected with a reporter plasmid, encoding the STAT-binding element and the reporter protein (STAT-3_firefly-luciferase plasmid; 50ng/well), and a transfection control plasmid (renilla-luciferase expression plasmid; 10-20ng/well) under optimized conditions referring to each cell type (for example: PC-12 cells can be transfected by lipofection using LIPOFECTAMINE2000™, Invitrogen, as recommended). 48-72h after transfection the assay can be run. Cells are stimulated with the testing substance in multiple concentrations which should cover a broad range of concentrations. Multiple assaying time points starting within 5 minutes of stimulation should be chosen. When using a Luciferase assay, the readout can be assessed in a Luminometer, plate format (Berthold, Germany), measuring stepwise the activity of renilla and firefly luciferase. The detection of the two luciferase-activities is done by the use of commercially available detection kits (Dual luciferase reporter assay kit, Promega; Luciferase reporter assay kit, Clonetech). Values of firefly luciferase are then normalized to renilla luciferase values and relative induction of reporter gene can be calculated.

In an alternative example of a screening method, the agonist activity on the GCSF or GMCSF receptor on neuronal cells can be utilized and measured. For example, the homodimerization upon ligand binding can be assayed by using fluorescence resonance transfer energy measurements (FRET, or BRET(bioluminescence resonance energy transfer) (Siegel RM et al Sci STKE (2000) 2000 (38):L1; Xu Y et al Proc Natl Acad Sci USA (1999) 96(1):151-6 ), or reporter systems for dimerization events, e.g. the double switch system (DE 10211063.8), the beta-gal reporter system (Rossi F et al Proc Natl Acad Sci USA (1997) 94 (16):8405-10), or the split-ubiquitin system (WO 954/29195, US 5,585,245, or Johnsson, N. and Varshavsky, Proc Natl Acad Sci USA (1994) 91 (22):10340-4).

In an additional alternative to screening for neuroprotective substances which bind to GCSF and GMSCF on neuronal cells, inducing.the brain-endogenous levels of GCSF and/or GMCSF is described. As described herein and demonstrated in the following examples, GCSF as well as GMCSF were surprisingly discovered as brain-endogenous ligands. These ligands were induced by several ischemic conditions of the brain. Therefore, they act as part of a brain-endogenous neuroprotective system. In the same sense as exogenously added GCSF or GMCSF that cross the blood-brain barrier are beneficial for a wide range of conditions where neuroprotection is a valid therapeutic principle, drugs that increase the brain-endogenous levels of GCSF / GMCSF can be beneficial for these conditions.

A screening system to identify such substances can be setup in the basic way of adding substances (for example from a large small molecule library) to cells in culture, for example, neuronal cells, either primary cells or neuron-like immortalized cells such as PC 12 cells, SHSY5-cells, and others, and measuring levels of GCSF and GMCSF.

Measuring the levels of GCSF / GMCSF can be performed by assaying the proteins themselves (by Western blotting, ELISA, RIA, and other techniques known to one skilled in the art), by assaying the mRNA encoding these proteins (such as quantitative PCR, Northern blotting, RNAse protection assay, RNA dot-blotting, and other techniques known to one skilled in the art), or by assaying the activity of the regulatory elements of the genes for GCSF / GMCSF. Preferably, the activity of regulatory elements can be assessed by reporter constructs consisting of DNA segments from the promoter, enhancer, and/or intronic elements coupled to cDNAs encoding reporters (such as luciferase, beta-galactosidase, green fluorescent protein, or other reporting genes that can be easily assayed). These reporter constructs can be transfected into cells, either stably, or transiently.

The cells may be kept under physiological conditions, or additionally exposed to noxious stimuli (such as hypoxia/glucose deprivation, NO donors, glutamate excess and others) to observe effects of concomitant addition of the respective substances to test.

Identified substances can be further validated by giving the substance to an animal, and measuring content of GCSF / GMCSF in the brain (e.g. by quantitative PCR).

Increasing the expression levels of the receptors for GCSF and GMCSF can be useful for increasing the responsiveness of the cells to the ligands. Therefore, the screening assays outlined above can also be adaptred to screen for increases in receptor expression.

Having generally described this invention, a further understanding can be obtained by reference to certain specific examples which are provided herein for purposes of illustration only and are not intended to be limiting unless otherwise specified.

### EXAMPLES

The experiments underlying the present invention demonstrate that GCSF is neuroprotective *in vitro,* and that GCSF displays significant infarct reducing effects after transient focal cerebral ischemia. Neurons in the periphery of the infarction but also on the contralateral side exhibited specific binding of anti GCSFR-antibody, indicative for a GCSF receptor. The presence of GCSFR on neurons is novel and was verified by Western blot, RT-PCR, and a detailed immunohistochemistry in the brain, and by PCR and immunocytochemistry in cultured primary neurons. Furthermore, STAT3 activation as judged by nuclear translocation of STAT3 was significantly increased in neurons of the penumbra of GCSF treated animals compared to controls suggesting a GCSFR / STAT mediated mechanism of action. In the in vivo ischemic experiment (middle cerebral artery occlusion, MCAO) there was no effect on cerebral blood flow as measured by laser Doppler flowmetry (Idf) when comparing both groups. There were no significant differences in physiological parameters and weight decline between both groups during the MCAO experiment. Mortality rate was significantly improved in animals treated with GCSF compared to controls in the MCAO experiment. Neutrophilic blood count (NGC) was significantly increased after 24 hours in GCSF treated animals compared to controls. Myeloperoxidase (MPO)-staining as a measure of invading neutrophilic granulocytes (NGs) into the ischemic hemisphere was not significantly different between GCSF treated animals and controls.

The dose of the i.v. delivered GCSF (60 µg/kg/body weight) used in the experiments was comparable to the doses used for other experimental conditions. It had been tested for safety in an earlier pilot project before and consequently no significant side effects were observed. This dose (60 µg/kg/body weight) is six times higher than the approved dose for the treatment of human myelodysblastic and other diseases, and has shown no appreciable side effects in the rat model.

An infarct reducing effect of 50% achieved with GCSF is comparable with that of other growth factors such as bFGF, or IGF after systemic application (Fisher M. et al., J. Cereb. Blood Flow Metab., 1995;15:953-9; Schäbitz WR, et al., Stroke 2001; 32:1226-33). It seems that glucose deprivation and excitotoxicity with subsequent Ca²⁺ overload of cells as well as apoptosis, reactive oxygen species, and decreased energy reserve in the face of increased requirements (e.g., from spreading depression) are the main causes of neuronal cell death following ischemia (Lee JM, et al., Nature 1999;399(Suppl):A7-14). As demonstrated in the examples, GCSF protects *in vitro* neuronal-like cells (NGF-differentiated PC12 cells) against H₂O₂ induced cell death. H₂O₂ elicits oxidative stress by the production of reactive oxygen species (ROS), which invokes cell death. H₂O₂-mediated cellular stress in mammalian cells is well-characterized in terms of cellular phenotype, dosage, time-course and signaling pathways involved. The wide-spread usage of H₂O₂ in a multitude of studies supports apoptotic mechanisms as effects of H₂O₂ in cells (see for example FASEB J. 2002 Jan;16(1):11.1-3; : J Cell Biochem 2001;82(3):437-44). For example, roles of the proapoptotic Kinase ASK1 and the FasLigand have been convincingly demonstrated in H₂O₂-mediated cell-death (Tobiume K, EMBO Rep 2001 Mar;2(3):222-8; Kwon, D., J Neuroimmunol. 2001 Feb 1;113(1):1-9; Facchinetti, F., J Neurosci Res. 2002 Jul 15;69(2):178-88.). Therefore, it is likely that GCSF interferes with apoptotic mechanisms invoked in cerebral ischemia. GCSF's action is probably mediated by binding to the high-affinity receptor GCSFR. Interaction with this receptor activates the Janus family kinases (JAKs) and STATs (Darnell JE Jr., Science 1997; 277:1630-5). JAK are non-receptor-type tyrosine protein kinases that become activated upon ligand-induced receptor dimerization. GCSF induced activation of JAKs phophorylate STATs on a conserved tyrosine residue, which induces STAT dimerization (Quelle FW, et al., Mol. Cell Biol. 1994; 14:4335-41). Furthermore, STATs translocate to the nucleus and subsequently regulate gene expression (Shuai K, et al., Nature 1993; 366:580-3; Shuai K., Oncogene 2000; 19:2638-44). STAT3 is the principal STAT protein activated by GCSFR (Shuai K., Oncogene 2000; 19:2638-44). STAT3 mediates antiapoptotic function by activating bcl-2 and induces proliferation and differentiation of granulocytes by upregulating the c-myc gene (Fukada T, Immunity 1996;5:449-60;, Shimozaki K, J. Biol. Chem. 1997; 272:25184-9). As shown here by using immunohistochemistry, RT-PCR, and Western-Blot GCSFR exists not only on hematopoetic cells but also on neurons, glial cells, neuronal-like cells, and neuronal stem cells. Furthermore, GCSF induced STAT3 upregulation in neurons of the penumbra may mediate anti-apoptotic effects such as bcl-2 upregulation as shown for BDNF or bFGF, (Schäbitz WR, et al., Stroke 2001; 32:1226-33) and provide trophic support of neurons to survive. Dense nuclear labeling of STAT3 in the penumbra of the infarction could reflect membrane receptor-mediated translocation of STAT3 from the cytoplasm to the nucleus which was already shown for activated microglia after cerebral ischemia (Planas AM, et al., Eur. J. Neurosci. 1996; 8:2612-8). GCSF is known to stimulate release, enhancement of effector function, and extension of lifetime by delaying apoptotic cell death of neutrophilic granulocytes (NGCs), the body's first line of defense against all kinds of infections (Hartung T., Curr Opin Hematol 1998; 5:221-5). Neutrophils could occlude microvessels, subsequent invasion of leukocytes triggers the release of proteolytic enzymes, oxygen free radicals, interleukines, and TNF-α - effects known to deteriorate infarct size and outcome after cerebral ischemia (Del Zoppo GJ, Stroke 1991; 22:1276-83; Jean WC, et al., Neurosurgery 1998; 43:1382-96; Matsuo Y, et al., Brain Res. 1994; 656:344-52). In contrast, GCSF has significant anti-inflammatory effects: GCSF reduces in models of peripheral infections TNF-a, IL-1β, IL-2, IL-6 and IL-8 and elevates IL-1β receptor-antagonists (Gorgen I, et al., J Immunol 1992; 149:918-24; Heard SO, et al., Crit. Care Med. 1999; 27:1019-21; Heard SO, et al., Crit. Care Med. 1998; 26:748-54; Hebert JC, et al., Arch. Surg. 1990; 125:1075-8; Lundblad R. et al., Crit. Care Med. 1996; 24:820-6.; Squadrito F. et al., Br J Pharmacol 1997; 120:333-9.). GCSF decreased TNF-α *release in vitro* and *in vivo* in healthy volunteers and elevated levels of antagonists for TNF, IL-6, IL-8, and IL-1β (Hartung T. Curr Opin Hematol 1998; 5:221-5; Gorgen I, et al., J. Immunol. 1992; 149:918-24; Heard SO, et al., Crit Care Med 1999; 27:1019-21). Moreover, reduced neutrophil infiltration in lung and ileum was observed in a model of splanchnic ischemia and reperfusion 15 minutes after administration of GCSF and reperfusion of the small bowel (Squadrito F, et al., Br J Pharmacol 1997; 120:333-9.). Consistent with these findings an increase of neutrophil infiltration into the ischemic hemisphere was not found although total neutrophilic granulocytes (NGCs) increased after GCSF treatment.

Another possible mechanism of action of growth factors in particular bFGF includes effects on cerebral blood flow. bFGF treatment dilates collaterals in the peri-ischemic zone even at doses not promoting systemic hypotension, thus increasing the blood flow to the penumbral regions (Tanaka R, et al., Stroke 1995;26:2154-8; discussion 2158-9). However, as shown here, GCSF treatment did not reduce systemic blood pressure or change cerebral blood flow compared with the control group as measured by LDF.

In the photothrombotic bengal rose model, postischemic intravenous GCSF treatment clearly improved sensory motor functional outcome six weeks after photothrombotic stroke, further supporting the hypothesis that growth factors induce recovery and regeneration after traumatic brain lesions *in vivo*. Two other compounds, basic fibroblast growth factor (bFGF) and osteogenic protein-1 (OP-1) were reported before to improve sensorimotor function and to induce neuronal sprouting after focal cerebral ischemia (Kawamata et al Proc Natl Acad Sci U S A 1997;94:8179-84; Kawamata et al Neuroreport 1998;9:1441-5; Ren et al Neuropharmacology 2000;39:860-5). In most of these studies growth factors were administered into the cerebral ventricles which is clinically not relevant. Only Ramirez et al. (Ramirez, et al. (1999), Neuroreport, 10, 1201-4. ) reported that intravenous administration of bFGF supports lesion-induced hippocampal sprouting, but the authors did not study functional outcome measures. The results presented here indicate that a clinically relevant dose protocol of GCSF administration induces functional recovery after cerebral ischemia. The capacity for enhancement of plasticity is clearly not limited to ischemic brain damage, but also relevant for neurodegenerative diseases such as Parkinson's disease and amyotrophic lateral sclerosis (ALS), the trinucleotide repeat diseases, cerebral ischemias due to resuscitation or intrapartal problems, probably also to dementias such as Alzheimer's disease, and to the neurodegenerative aspects of schizophrenia.

### Overview of the methods for the experiments on cerebral ischemia, not part of the invention

Ischemia was induced by using the suture occlusion model of the middle cerebral artery (90 min) in the rat. 30 min after induction of ischemia, animals (n=12 per group) received 60 µg/kg body weight of GCSF intravenously for 90 min or vehicle. Infarct volume was calculated based on TTC (2,3,5-triphenyltetrazolium chloride)-staining 24 hours after ischemia. Expression of the GCSFR was studied by immunohistochemistry, verified by RT-PCR and immunoblotting. Expression of STAT3 was studied by immunohistochemistry. Efficacy of GCSF in funtional recovery was studied in the Bengal rose photothrombotic model.

### Statistical analyses

The values presented in this study are means ± SD. After acquiring all the data, the randomization code was broken. ANOVA and subsequent post hoc Fisher protected least significant difference test or Bonferroni correction were used to determine the statistical significance of differences for in vitro data and physiological parameters, or functional outcome in test batteries. The t-test was used for comparison of postmortem infarct volumes, MPO, and STAT3 immunohistochemistry. The Chi-Square test was performed for mortality data. A p value <0.05 was considered statistically significant.

### Results

GCSF reduced infarct volume to 131,96 mm³ ± 112,7 mm³ vs 278,9 mm³ ± 91,56 mm³ (p<0.05) in the control group. Immunohistochemistry, Western blotting, and RT-PCR revealed the existence of GCSF receptors in neurons and glial cells. GCSF significantly activated STAT3 in the periphery of the infarction compared to controls (p<0.05). GCSF is effective in improving functional recovery after ischemia in the model of Bengal rose photothrombosis.

It has therefore been demonstrated that GCSF has a significant neuroprotective effect in cell culture and after focal cerebral ischemia. This effect seems to be mediated by interaction with GCSFR and activation of STAT3.

### Example 1- Focal cerebral ischemia (not part of the invention)

### Procedure for inducing focal cerebral ischemia (MCAO, middle cerebral artery occlusion)

Experimental protocols were approved by the local ethics committee. Twenty-four male Wistar rats (Charles River, Germany) weighing 280 to 320 g were randomly assigned to the following groups: A (Control group, n=12, ischemia for 90 min, treatment with 2 ml saline 0.9% for 90 min beginning 30 min after vessel occlusion); B (GCSF group, n=12, ischemia for 90 min, treatment with 60 µg /kg body weight of recombinant human GCSF, Neupogen^{®}, Amgen, Europe B.V., Netherlands, soluted in 2 ml saline 0.9% for 90 min beginning 30 min after vessel occlusion. Alternatively, any GCSF or derivative or formulation of other source (another manufacturer (e.g. Lenogastrim™ by Roche or Granocyte™ by Chugai or Albugranin™ by HGS or Neulasta™ by Roche/Amgen) can be used here.

Animals then were anesthetized with an intraperitoneal injection of 100 mg/kg body weight ketamine hydrochloride (WDT, Garbsen, Germany). Anesthesia was maintained with 50 mg/kg body weight if necessary. A PE-50 polyethylene tube was inserted into the right femoral artery for continuous monitoring of mean arterial blood pressure, blood gases, hematocrit, leukocyte count, and blood glucose levels. The right femoral vein was cannulated by a PE-50 tube for treatment infusion. During the experiment rectal temperature was monitored and maintained at 37 °C by a thermostatically controlled heating pad (Föhr Medical Intruments, Germany). Transient focal cerebral ischemia was induced by using the suture occlusion model as described in detail by Zea Longa et al. (Stroke 1989; 20:84-91). Briefly, the right common carotid artery and the right external carotid artery were exposed through a midline neck incision. A 4-0 monofilament nylon suture (Ethicon, Germany) coated with silicon (Bayer, Germany) was inserted through an arteriectomy in the common carotid artery, gently advanced into the internal carotid artery and positioned approximately 17 mm from the carotid bifurcation. Using this technique, the tip of the suture occludes unilaterally the proximal anterior cerebral artery, the origins of the MCA and the posterior communicating artery. A large infarct in the territory of the MCA is typically produced. Reperfusion was performed by withdrawal of the occluder filament 90 minutes after vessel occlusion. Sham-operated animals underwent the same experimental procedures as described above but the nylon filament was not advanced beyond the common carotid artery, so that no infarction occurred. After surgery, the catheters were removed, and the animals were allowed to recover from the anesthesia and given food and water *ad libitum*.

### Measurement of regional cerebral blood flow

Laser-Doppler flowmetry (LDF) (Periflux 4001 Master, Perimed AB, Sweden) was used to monitor cerebral blood flow (CBF) before, during and after occlusion of the MCA. After placing the animal into a stereotactic frame, the animal's skull was exposed and a hole of 1.5 mm in diameter was drilled under the microscope on the right side 4 mm lateral and 2 mm caudal to the bregma. The dura was left intact and the LDF probe (1.4 mm in diameter) was placed into the burr hole. The area selected for CBF monitoring corresponded to the ischemic penumbra of the MCA occlusion model in rats.

### Infarct volume calculation

24 hours after MCA occlusion, the rats were anesthetized with ketamine 150 mg/kg body weight and decapitated. The brains were dissected and cut into 5 coronal slices of 2 mm thickness, incubated in a 2 % solution of 2,3,5-triphenyltetrazolium chloride (TTC) at 37 °C for 30 min and fixed by immersion in a 10 % buffered formalin solution. TTC-stained sections were photographed using a charge coupled device camera (EDH-1000HR Computer Camera, Electrim Corporation, Princetown, NJ, USA). A blinded investigator measured the infarct sizes with a computerized image analyzer (Bio Scan Optimas, Edmonds, WA). To compensate for the effect of brain edema the corrected infarct volume was calculated as previously described in detail: Corrected infarct area equals left hemisphere area minus (right hemisphere area minus infarct area) (Schäbitz WR, et al., Stroke 2001; 32:1226-33). Infarct volumes were expressed as a percentage of the contralateral hemisphere.

### Ischemia experiment

GCSF achieved a potent neuroprotective effect after focal cerebral ischemia. Mean infarct volume in intraperitoneal GCSF treated animals was 131,96 mm³ ± 112,7 mm³ versus 278.9 mm³ ± 91.56 mm³ in the control group or 9.96 ± 8.31% (n=12) versus 22.7 ± 6.69 % of the total hemisphere (p<0.05;Fig. 1).

GCSF treatment significantly reduced mortality: Four animals in the control group and one in the GCSF-treated group died within the 24-hour reperfusion period (p<0.05). No statistical differences were observed between the control and GCSF-treated group for rectal temperature, pH, pCO₂, pO₂, hematocrit (hct), blood glucose, heart rate, mean arterial pressure, and body weight for all animals (Table 1). Leukocyte count in the peripheral blood was significantly increased 24 hours after ischemia in GCSF treated animals compared to controls (p<0.05, Table 1).

**Table 1:**

| **Time** | **Group** | **rectal Temp** | **pH** | **pCO2 (mm Hg)** | **pO2** | **Hct** | **Gluc** | **MABP** | **HR** | **Leukocytes** | **Body Weight** |
|---|---|---|---|---|---|---|---|---|---|---|---|
| | **(n=1)** | | | | **(mm Hg)** | **(%)** | **(mg/dL)** | **(mm Hg)** | **(Beats/ min)** | **(x 10⁹/L)** | **(g)** |
| Pre-ischemia | Control | 37 ± 02 | 7.38 ± 0.03 | 39 ± 7 | 89 ± 7 | 47.4 ± 3.6 | 263 ± 25 | 98 ± 12 | 358 ± 13 | 1.9 ± 0.3 | 314 ± 25 |
| | rGCSF | 37 ± 0.3 | 7.35 ± 0.02 | 38 ± 5 | 91 ± 7 | 46 ± 0.9 | 251 ± 31 | 102 ± 15 | 350 ± 24 | 1.8 ± 0.4 | 318 ± 29 |
| 1 h | Control | 37 ± 0.1 | 7.38 ± 0.02 | 41 ± 6 | 88 ± 5 | 45.3 ± 0.8 | 160 ± 13 | 112 ± 21 | 384 ± 16 | 6.5 ± 0.6 | |
| | rGCSF | 37 ± 0.2 | 7.37 ± 0.03 | 39 ± 4 | 89 ± 8 | 44.3 ± 0.7 | 172 ± 17 | 109 ± 19 | 371 ± 27 | 6.8 ± 0,3 | |
| 2 h | Control | 37 ± 0.3 | 7.39 ± 0.03 | 37 ± 3 | 87 ± 8 | 44.6 ± 0.8 | 149 ± 12 | 101 ± 14 | 368 ± 13 | 8.2 ± 0.4 | |
| | rGCSF | 37 ± 0.2 | 7.4 ± 0.04 | 39 ± 6 | 89 ± 5 | 44.2 ± 0.4 | 152 ± 14 | 99 ± 8 | 372 ± 9 | 8.5 ± 0.3 | |
| 3 h | Control | 37 ± 0.2 | 7.38 ± 0.02 | 38 ± 5 | 91 ± 5 | 43.3 ± 0.9 | 133 ± 7 | 102 ± 16 | 366 ± 17 | 9.7± 0.8 | |
| | rGCSF | 37 ± 0.1 | 7.37 ± 0.03 | 37 ± 4 | 94 ± 10 | 43.1 ± 1.2 | 141 ± 10 | 99 ± 12 | 384 ±13 | 10.6 ± 0.5 | |
| 4 h | Control | 37 ± 0.2 | 7.36 ± 0.02 | 37 ± 6 | 87± 5 | 42.1 ± 0.9 | 168 ± 13 | 113 ± 24 | 373 ± 21 | 13.2 ± 0.6 | |
| | rGCSF | 37 ± 0.3 | 7.38 ± 0.03 | 39 ± 7 | 89 ± 9 | 42.8 ± 1.1 | 174 ± 16 | 120 ± 17 | 361 ± 12 | 13.7 ± 0.6 | |
| 24 h | Control | 37 ± 0.2 | 7.37 ± 0.03 | 38 ± 4 | 86 ± 6 | 46.7 ± 1.5 | 198 ± 13 | 115 ± 17 | 365 ± 10 | *3.8 ± 0,8 | 285± 24 |
| | rGCSF | 37 ± 0.2 | 7.37 ± 0.04 | 41 ± 5 | 87 ± 8 | 47 ± 0.5 | 204 ± 16 | 117 ±21 | 359 ± 15 | 9.7 ± 0.4 | 293 ± 16 |

| | | | | | | | | | | | |
|---|---|---|---|---|---|---|---|---|---|---|---|
| Table 1 lists physiological parameters of GCSF treated animals and controls. Values are given as mean ± SD (p<0.05; ANOVA;F-test). | | | | | | | | | | | |

LDF-monitoring revealed no statistical differences between the two treatment groups (data not shown).

### Example 2 - Immunohistochemistry in the context of focal cerebral ischemia (not part of the invention)

### Immunohistochemical methods used

For morphological analysis of STAT3 activation (Fig. 6) and GCSFR distribution in infarcted brains, and counts of neutrophilic granulocytes, a 2-mm-thick brain slice of GCSF-treated animals and controls was immersion fixed in 4% paraformaldehyde in 0.1 mol/l phosphate buffer for 24 hrs (n=5 per group). After paraffin-embedding, 1-µm-thick sections were cut and used for H&E staining, Nissl staining and immunohistochemical analysis.

Immunohistochemical studies were performed with antisera against myeloperoxidase (DAKO, Carpinteria, CA, USA), glial fibrillary acidic protein (GFAP) (DAKO, Carpinteria, CA, USA), GCSFR (Santa Cruz Biotechnology Inc., Santa Cruz, CA, USA) and STAT3 (Santa Cruz Biotechnology Inc., Santa Cruz, CA, USA). For antigen retrieval, sections provided for GCSFR and STAT3 immunohistochemistry were heated for 20 min in a 10 mM citrate buffer at 99°C. Sections were then incubated in normal swine serum (10% in phosphate-buffered saline) for 30 min and then in the primary antisera overnight at 4°C. The primary antibodies were diluted 1:150 (myeloperoxidase), 1:400 (GFAP), 1:400 (GCSFR) and 1:100 (STAT3), respectively. Immunoreactivity was visualized by the avidin biotin complex method (Vectastain, Vector Laboratories, USA). Sections were developed in 0.02% diaminobenzidine (DAB) with 0.02% hydrogen peroxide. The reaction product was intensified by the addition of 0.02% cobalt chloride and nickel ammonium sulfate. In a subset of control slides preabsorption of the GCSFR antiserum with the respective peptide did not produce immunostaining (not shown). When omitting the primary antisera, no immunostaining was produced either (not shown).

Invasion of neutrophilic granulocytes (NG) was quantitatively measured by counting NGs per infarcted hemisphere. STAT3 protein expression was quantified in 2 overlapping fields rostro-caudal in the vincinity of the infarction of the parietal cortex and the corresponding contralateral side (magnification x 400). To this end, neurons with nuclear translocation were counted, given as percent of STAT3 positive neurons from all neurons.

### Results of the immunohistochemical experiments in focal cerebral ischemia

Myeloperoxidase (MPO) staining detected no neutrophilic granulocytes (NGs) in the non-ischemic hemispheres of both groups. MPO staining was not significantly different between GCSF treated animals and controls based on quantified MPO positive cells in the ischemic hemisphere (14±17.6 versus 14.3±12.5, n.s.).

GFAP immunoreactivity (IR) was present in scattered astrocytic processes throughout the cortex, striatum and white matter of the non-infarcted hemisphere. No difference in the pattern and intensity of GFAP staining was detectable in the cortical peri-infarct zone both in untreated and GCSF treated rats. In particular, GFAP IR was not increased in the cortical penumbra, neither in the placebo group, nor in the GCSF group (not shown). Within the infarct core, scattered GFAP immunoreactive astrocytes were detectable (not shown).

Immunohistochemically, staining for GCSFR was detectable in scattered cortical neurons and neurites (not shown) both in untreated and GCSF treated animals. Glial cells were also stained with the GCSF-R antibody (not shown). In the infarct core, no GCSF-R immunreactive cells were seen. No difference in the pattern and intensity of GCSF-R IR was evident between the two experimental groups.

STAT3 IR was seen in scattered nuclei of neurons and glial cells within the uninfarcted hemisphere of both placebo and GCSF treated rats. Some cytoplasmic staining was also present in a few scattered neurons. STAT3 protein expression was significantly increased after GCSF treatment in the penumbra of the infarction compared to untreated controls (34.4±7.05 versus 13.7± 4.4; p<0.0003)(Fig. 6, 7). No difference occurred on the contralateral side (16.2±6.9 versus 13.3±6.9; n.s.).

### Example 3 - Western Blots and PCR in the context of focal cerebral ischemia (not part of the invention)

### Western Blots (Figure 3)

For immunoblotting, brain tissue (transient ischemia of 2 hours) was lysed in 20 volumes (w/v) of homogenization buffer (320 mM sucrose, 0.1 mM phenylmethylsulfonyl fluoride, and 2 µg/ml Pepstatin) at 4°C. Homogenates were centrifuged at 9,200 G for 15 min at 4°C. After resuspending pellets in 1/10 of the homogenization volume, aliquots for protein determination (Bio-Rad protein-assay, Munich, Germany) were separated and samples were rapidly frozen in nitric oxide and stored at -70°C. Per lane 15 µg protein were loaded on a 8 % SDS polyacrylamide gel containing 4 M urea and electrophoresed under standard conditions. Proteins were electrophoretically transferred to Immobilon-P™ membranes (Millipore Corp., Eschborn, Germany) by semi-dry blotting. After blocking in 3% nonfat dry milk in TBST (20 mM Tris base, pH 7.6,137 mM NaCl and 0.05% Tween-20) for 1 hour at room temperature (RT), membranes were incubated with the primary GCSFR antibody (1: 500) overnight at 4°C. After washing in TBST the membranes were incubated for 1 hour at RT with 1:2,000 dilutions of the appropriate horseradish peroxidase-conjugated secondary antibody. Immunoreactive bands were visualized in the linear range with enhanced chemoluminescence (Amersham Intl., Braunschweig, Germany).

In immunoblotting experiments with cortical extracts (Figure 3), the GCSF-R antiserum detected a protein band of approximately 130 kD, consistent with the deduced molecular weight (Fukunaga R, et al., J Biol Chem 1990; 265:14008-15). In addition, a few bands of lower molecular weight were seen, probably reflecting breakdown products. After preabsorption of the GCSFR antiserum with the respective peptide the bands disappeared (Fig. 3).

### PCR for the GCSF receptor in brain (Figure 2A)

After rats were deeply anesthetized and perfused transcardially, brains were rapidly dissected. RNA was extracted from brains by the RNA-clean kit (AGS, Heidelberg, Germany), according to the manufacturer's instructions. A total of 10 µg RNA was reverse transcribed with MMLV reverse transcriptase and random hexamers. For PCR, the following primers from exons 5 and 7 of the murine GCSFR were used: sense, 5'-CCC CTC AAA CCT ATC CTG CCT C-3' (SEQ ID NO:5); and antisense, 5'-TCC AGG CAG AGA TCA GCG AAT G-3' (SEQ ID NO:6). (Ashihara E, et al., J Cell Physiol 1997; 171:343-56). PCR was performed according to the following protocol: 3 min 94 °C, 1 min 94 °C, 1 min 58 °C, and 1 min 72 °C (40 cycles). The product was analyzed on a 2 % agarose gel.

Using a RT-PCR specific for the mouse GCSFR, GCSF-R mRNA was detected in the brain tissue (Figure 2A). The PCR product had the expected size of 567 bp. The identity was verified by sequencing the PCR product (Fig. 2).

### Example 4 - GCSF efficacy in ALS models

### Survival test in ALS mouse models

Previous experiments have demonstrated that SOD1 mouse models of ALS are predictive of the success of therapy in humans (Cleveland and Rothstein (2001), Nat Rev Neurosci, 2, 806-19). Primary endpoints in such analyses are both onset of motor signs, and mortality. For example, the onset of motor signs can be defined as the first day that a mouse can not remain on the rotarod for 7 min at a speed of 20 rpm (Li, et al (2000), Science, 288, 335-9). Mortality is scored as the day of death, or the day where deficits are so severe that the mouse has to be sacrificed (e.g. apathy and unability to right itself). Additional parameters are determined by the measurement of motor strength by grip strength tests, counts of motor neurons in the spinal cord, nerve thickness (e.g. sciatic nerve, phrenic nerve), and the presence of apoptotic stainings in spinal cord motor neurons. GCSF can be infused via an osmotic pump into the cerebral ventricles at a pre-determined dose, e.g. at 60 ug / kg body weight/ day. Alternatively, GCSF is given via i.v. or i.p. injection at a dose of 60 ug/kg body weight per day, or higher doses. Alternatively, a slow-release form of GCSF is administered, such as a PEG formulation (see above), or an albumin formulation (see above), or other slow-release formulations. Alternatively, any GCSF or derivative or formulation of other source (another manufacturer (e.g. LENOGASTRIM™ from Roche, GRANOCYTE™ from Chugai Pharma, Co. Ltd., ALBUGRANIN™ from Human Genome Sciences, or NEULASTA™ from Roche/Amgen) is used. Treatment is started at day 60 in the late presymptomatic stage of the SOD1 G93A mutant. In nontreated familial ALS mice, motor impairments appear at 12-14 weeks of age, whereas paralysis is not observed before 20 weeks of age. Life expectancy is 140-170 days. Effective treatment should prolong life as compared to the control group by more than 15% *(*Cleveland and Rothstein (2001), Nat. Rev. Neurosci., 2, 806-19). As a control group for treatment, both vehicle and zVADfmk (a potent caspase inhibitor that has shown efficacy in this model) treated animals will be used. Each group comprises 10 animals each.

### Example 5 - GCSF efficacy in Parkinson models (not part of the invention)

There are various rodent models of Parkinson's disease available, that are suitable for efficacy studies of GCSF (Grunblatt, et al. (2000), J Neurol, 247 Suppl 2, II95-102. ). One well-characterized model is the use of 1-methyl-4-phenyl-1,2,3,6-tetrahydropyridine (MPTP). 4 doses of MPTP-HCl (15 mg/kg per dose) can be given to eight-week-old male mice (n=20) via i.p. injection at 2 hr intervals. Sham-treated animals receive saline. 20 animals receive both the MPTP treatment, and a daily dose of GCSF (i.v., 60 ug/kg bodyweight) seven days after the last MPTP treatment mice are sacrificed. Until that time, mice are analyzed both for motor parameters (rotarod performance, free locomotion). The brains of 10 mice each are processed for immunohistochemistry to assay the total number of neurons in the substantia nigra that are positive for tyrosine hydroxylase or the dopamine transporter (using commercially available antibodies), the number of apoptosis-positive neurons (TUNEL staining, caspase-3 staining). Remaining dopaminergic neurons after MPTP treatment are compared to those receiving MPTP plus GCSF, and to the sham group.

The remaining 10 animals in each group will be perfused transcardially with saline, sacrificed, and the striatum dissected out. The striatum will be homogenized on ice, and the dopamine content measured by HPLC with electrochemical detection. Comparison of the three groups will provide a good measure of dopamine depletion due to loss of cells in the substantia nigra.

This experiment can also be performed using different dosing schemes for MPTP (i.e. 40 mg/kg body weight once; 30 mg/kg body weight twice, etc.).

### Example 6 - GCSF improves sensory-motor function after photothrombotic cerebral ischemia (Figure 8) (not part of the invention)

### Experimental groups

Experimental protocols were approved by the local ethics committee. Male Wistar rats (Charles River, Germany) weighing 280 to 320 g were randomly assigned to the following groups: A (Control group, n=6), ischemia, treatment with 0.5 ml saline 0.9% as i.v. bolus infusion beginning 1 h after ischemia); B (GCSF group, n=6), ischemia, treatment with 5 µg GCSF (Amgen) soluted in 0.5 ml saline 0.9% as i.v. bolus beginning 1 h after ischemia. Alternatively, any GCSF or derivative or formulation of other source (another manufacturer (e.g. Lenogastrim™ by Roche or Granocyte™ by Chugai or Albugranin™ by HGS or Neulasta™ by Roche/Amgen) can be used here. Repetitive i.v. bolus infusions via the tail vene followed at day I to 5. C (sham operated group, n=6), sham operation, no ischemia, treatment with 0.5 ml saline 0.9% as i.v. bolus infusion beginning 1 h after ischemia.

### Focal cerebral ischemia by photothrombosis

Animals were anesthetized with an intramuscular injection of 100 mg/kg body weight ketaminehydrochloride (WDT, Garbsen, Germany). Anesthesia was maintained with 50 mg/kg body weight if necessary. A PE-50 polyethylene tube was inserted into the right femoral artery for continuous monitoring of mean arterial blood pressure, and blood gases. The right femoral vein was cannulated by a PE-50 tube for treatment infusion. During the experiment rectal temperature was monitored and maintained at 37 °C by a thermostatically controlled heating pad (Föhr Medical Intruments, Germany).

Photothrombotic ischemia was induced in the right rat parietal cortex according to the method of Watson et al.(Watson BD, Dietrich WD, Busto R, Wachtel MS, Ginsberg MD. Induction of reproducible brain infarction by photochemically initiated thrombosis. Ann Neurol. 1985;17:497-504.). Animals were anesthetized withketaminehydrochloride and placed in a stereotaxic frame, and the scalp was incised for exposure of the skull surface. For illumination, a fiber-optic bundle with a 1.5-mm aperture was placed stereotaxically onto the skull 4 mm posterior to the bregma and 4 mm lateral from the midline. The skull was illuminated with a cold, white light beam (150 W) for 20 minutes. During the first 2 minutes of illumination, the dye rose bengal (0.133 mL/kg body wt, 10 mg/mL saline) was injected intravenously. Sham-operated animals underwent the same experimental procedures as described above without infusion of rose bengal and illumination. After surgery, the catheters were removed, and the animals were allowed to recover from the anesthesia and given food and water *ad libitum*.

### Behavioural Testing

In all animals a battery of behavioral tests was performed before ischemia and at baseline, 2, 3,4,5, and 6 weeks after ischemia by an investigator who was blinded to the experimental groups. For the rotarod test, rats were placed on an accelerating rotarod cylinder, and the time the animals remained on the rotarod was measured (Hamm et al, J Neurotrauma 1994 Apr; 11(2):187-96, Chen J, Li Y, Wang L, Zhang Z, Lu D, Lu M, Chopp M. Therapeutic Benefit of Intravenous Administration of Bone Marrow Stromal Cells After Cerebral Ischemia in Rats. Stroke. 2001;32:1005.). The speed was slowly increased from 4 to 40 rpm within 5 minutes. The trial ended if the animal fell off the rungs or gripped the device and spun around for 2 consecutive revolutions without attempting to walk on the rungs. An arbitrary limit of time was set for the rats at 500 seconds on the rotarod cylinder in training as well as in testing procedures. The animals were trained 3 days before ischemia. The mean duration (in seconds) on the device was recorded with 3 rotarod measurements 1 day before surgery. Motor test data are presented as percentage of mean duration (3 trials) on the rotarod compared with the internal baseline control (before surgery).

For the adhesive-removal test, somatosensory deficit was measured both before and after ischemia (Schallert T, Kozlowski DA, Humm JL, Cocke RR. Use-dependent structural events in recovery of function. Adv Neurol. 1997;73:229-23 8.; Chen J, Li Y, Wang L, Zhang Z, Lu D, Lu M, Chopp M. Therapeutic Benefit of Intravenous Administration of Bone Marrow Stromal Cells After Cerebral Ischemia in Rats. Stroke. 2001;32:1005.). All rats were familiarized with the testing environment. In the initial test, 2 small pieces of adhesive-backed paper dots (of equal size, 113.1 mm²) were used as bilateral tactile stimuli occupying the distal-radial region on the wrist of each forelimb. The rat was then returned to its cage. The time to remove each stimulus from forelimbs was recorded on 5 trials per day for each forepaw. Individual trials were separated by at least 5 minutes. Before surgery, the animals were trained for 3 days. Once the rats were able to remove the dots within 10 seconds, they were subjected to ischemia.

Neurological Severity Scores (NSS) were modified according to Chen J, Li Y, Wang L, Zhang Z, Lu D, Lu M, Chopp M. Therapeutic Benefit of Intravenous Administration of Bone Marrow Stromal Cells After Cerebral Ischemia in Rats. Stroke. 2001;32:1005., and Schallert T, Kozlowski DA, Humm JL, Cocke RR. Use-dependent structural events in recovery of function. Adv Neurol. 1997;73:229-238. Neurological function was graded on a scale of 0 to 16 (normal score, 0; maximal deficit score, 16). NSS is a composite of motor, sensory, reflex, and balance tests (Chen J, Li Y, Wang L, Zhang Z, Lu D, Lu M, Chopp M. Therapeutic Benefit of Intravenous Administration of Bone Marrow Stromal Cells After Cerebral Ischemia in Rats. Stroke. 2001;32:1005., Germano AF, Dixon CE, d'Avella D, Hayes RL, Tomasello F. Behavioral deficits following experimental subarachnoid hemorrhage in the rat. J Neurotrauma. 1994;11:345-353.). In the severity scores of injury, 1 score point is awarded for the inability to perform the test or for the lack of a tested reflex; thus, the higher score, the more severe is the injury.

### Results

No statistical differences were observed between the groups for rectal temperature, pH, pCO₂, pO₂, hematocrit (hct), blood glucose, heart rate, mean arterial pressure, body weight, and mortality for all animals (data not shown).

Functional recovery in GCSF treated animals was remarkably better over time compared with all other groups. GCSF treated animals had significantly lower NSS scores including Beam-Balance during the experiment compared to the control group (p<0.001, Fig.8 b), and resulted in significantly better rotarod performance compared to controls (*P*<0.05, Fig.8 a). Sensorimotor function as measured by adhesive tape removal was also better in GCSF treated animals on the contralateral forepaw over time compared to controls as well as on the ipsilateral forepaw at week 6 (see Fig.8 c,d).

### Example 7 - GCSF receptor is upregulated in the photothrombotic model of cerebral ischemia (not part of there invention)

RNA was isolated according to standard protocols (Chomczynski and Sacchi (1987), Anal. Biochem., 162, 156-159 ), followed by Qiagen RNeasy™ mini kit purification from rat cortical penumbral samples, ipsi- and contralateral to the lesion side (see Fig. 9a for localization of the tissue samples; here: 3 vs. 4). cDNA was synthesized from 1 µg total RNA using oligodT primers, superscript II reverse transcriptase (Gibco) using standard conditions. Quantitative PCR was performed using the Lightcycler® system (Roche Diagnostics, Mannheim, Germany) with SYBR-green staining of DNA doublestrands. Cycling conditions were as follows: 5 min 95°C, 5 sec 95°C, 7 sec 66°C, 30 sec 72°C; 9 sec 84°C for 55 cycles. Melting curves were done with the following parameters: 95°C cooling to 50°C; ramping to 99°C at 0.2°C/ sec. The following primer pairs were used: "rat GCSFR-frag-32s" CCATTGTCCATCTTGGGGATC (SEQ ID NO:7), and "rat GCSFR-frag-265as" CCTGGAAGCTGTTGTTCCATG (SEQ ID NO:8). The Lightcycler® PCR was performed using the SYBR green master mix, following the manufacturer's recommendations (Roche Diagnostics). Specificity of product was ensured by melting point analysis and agarose gel electrophoresis. cDNA content of samples was normalized to the expression level of Cyclophilin (primers: "cyc5" ACCCCACCGTGTTCTTCGAC (SEQ ID NO:9); "acyc300" CATTTGCCATGGACAAGATG (SEQ ID NO:10)). Relative regulation levels were derived after normalization to cyclophilin, and comparison to the sham-operated animals. Fig 9 b shows upregulation of the GCSFR after 48h on the contralateral side, error bars indicate standard deviations, these are calculated from 3-fold serially diluted cDNA-samples, and reflect reliability of measurements.

### Example 8 - GCSF receptor is present on neuronal cells in primary cultures: Immunocytochemistry

### Preparation of neurons

10-12 Cortices were prepared from embryos of the stage E18 (embryonal day 18). Tissue was dissociated using trypsin [10mg/ml] /EDTA /DNase [5mg/ml] (Roche Diagnostics) in HBSS (Hanks balanced salt solution, BioWithakker). The digest was stopped using 4 parts medium (neurobasalmedium + 1ml 50x B-27 supplement (Invitrogen) + 0,5mM L-glutamin + 25µM glutamate) and centrifuged at room temperature for 5 min at 800g. The pellet was dissolved in 5ml medium and cell number determined by counting (Neubauer slide). The cells were plated at a density of 250 000 cells per well of a 24-well-plate on cover slips which were coated with poly-L-lysine.

### Immunocytochemistry

14 days after preparation neurons were washed with PBS (Gibco) (37°C) and fixed with 2% paraformaldehyde for 10min on ice. Then, cells were washed with PBS (4°C) and stored at 4°C. Cells were incubated for 10min in 50mM glycine in PBS, and then washed with PBS. Cells were permeabilised on ice using 0,2% TritonX-100 (Sigma) in PBS, and incubated with blocking solution (0,2%Triton-X100, 4% normal goat serum (NGS) (Jackson Immunoresearch Laboratories) in PBS) at room temperature. The primary antibody (rabbit-anti-GCSF-Rezeptor-antibody directed against the C-terminus of mouse GCSFR, M-20; sc-694; SantaCruz Biotechnology, Inc.) was used in a dilution of 1:800 (in 0,1% Triton-X100/2% NGS), and incubated overnight at 4°C. Cells were then washed with 1%NGS/PBS, and incubated for 30min with the secondary antibody (anti-rabbit-FITC, 1:400; dianova) at room temperature. Cells were then washed briefly in 1%NGS/PBS, and stained with Hoechst 33342 (Molecular Probes) (1:10000 in PBS) for counterstaining the nuclei. Finally, cover slips were washed briefly twice in 1%NGS/PBS, twice in PBS, and once for 10 min in 10mM Tris/HCl, pH 7,6. The cover slips were embedded using Aquamount (Polyscience).

Pictures were taken digitally with an Olympus IX81 microscope, and the "Analysis" software package (Soft Imaging Systems, Stuttgart, Germany).

### Example 9 - GCSF receptor is present on neuronal stem cells: PCR and immunocytochemistry (Figure 12,13); GCSF receptor is present on PC12 cells: PCR (Figure 2B)

### Generation of Neural stem cells

Neural stem cells were isolated from the brain areas with known spontaneous neurogenesis, i.e., hippocampus, olfactory bulb, and subventricular zone, of 4-6 week old male Wistar rats as described . (Ray J et al (1993), Proc Natl Acad Sci U S A 90: 3602-6.). Protocols are concordant with the policy on the use of animals, as endorsed by the National Research Council of the U.S.A., and fulfill the requirements of German law. Briefly, animals were anesthesized with 1 % (v/v) isoflurane, 70 % N20, 29 % oxygen and sacrificed by decapitation. The brains were removed and washed in 50 mL ice-cold Dulbecco's Phosphate Buffered Saline (DPBS) supplemented with 4.5 g/L glucose (DPBS/Glc). Hippocampus, olfactory bulb, and subventricular zone from 6 animals were dissected, washed in 10 mL DPBS/Glc and centrifuged for 5 min at 1600 x g at 4 °C. After removal of the supernatant, the tissue was homogenized with scissors and scalpels. The tissue pieces were washed with DPBS/Glc medium for 5 min at 800 g, and the three pellets were resuspended in 0.01 % (w/v) papain, 0.1 % (w/v) dispase II (neutral protease), 0.01 % (w/v) DNase I, and 12.4 mM manganese sulfate in Hank's Balanced Salt Solution (HBSS). The tissue was triturated with plastic pipet tips and incubated for 40 min at room temperature, but every 10 min the solution was mixed well. The suspension was centrifuged at 800 x g for 5 min at 4 °C and pellets were washed three times in 10 mL DMEM-Ham's F-12 medium supplemented with 2 mM L-glutamine, 100 units/mL penicillin and 100 units/mL strepotomycin. Then, the cell pellets were resuspended in 1 mL Neurobasal medium supplemented with B27 (Invitrogen, Carlsbad, CA, USA), 2 mM L-glutamine, 100 units/mL penicillin and 100 units/mL strepotomycin, 20 ng/mL EGF, 20 ng/mL FGF-2, and 2 µg/mL heparin. Cells were plated under sterile conditions in 6-well dishes in a concentration of 25,000-100,000 cells/mL. The dishes were incubated at 37 °C in 5 % CO2. Cell culture media were changed once a week, where about two thirds of the media were replaced. (Ray J et al (1993), Proc Natl Acad Sci U S A 90: 3602-6.)

### RT-PCR protocol:

RNA was isolated according to standard protocols from hippocampal stem cells (Figure 12) that were propagated 3 weeks in culture after thawing them from frozen stocks, following the manufacturer's recommendations (RNeasy kit, Qiagen). cDNA was synthesized using oligodT primers, Superscript II reverse transcriptase (Gibco) using standard conditions. Polymerase chain reaction (PCR) was performed with the following cycling conditions: 3 min 94°, 30 sec 94°, 30 sec 60°, 1 min 72°; for 32 cycles, using the primer pairs "rat GCSFR-frag-8s" GCGGGCAAATCAGGATCTCAC (SEQ ID NO:2), and "rat GCSFR-frag-287as" CGAAGCTCAGCTTGATCCAGG (SEQ ID NO:3). The primers were derived from a fragment of rat GCSFR identified from rat genomic databases by TBLASTX searches (see Fig. 11). Reaction conditions: 2 mM MgCl2, 200 uM dNTP, 200 nM each primer, 1 unit Taq Polymerase (Invitrogen)/25 µl. The PCR product was resolved on a 1.5% agarose gel. The specific PCR product length was 279 bp, with the following sequence (SEQ ID NO:4) (primer sequences are underlined):

For PCR on PC12 cells (Figure 2B), the above protocol was followed.

### Immunocytochemistry of neurospheres:

Neurospheres consisting of the neural stem cells were pipetted onto the glass slide, coverslip was put onto the cells, and the slide was put at -80°C for at least 30 min. The coverslip was removed, and put into 4% PFA (paraformaldehyde) in 0.1 M Phosphatpuffer, pH 7.4 immediately. Cells were fixed for 20 min. Cells were washed 3 x 5min in PBS (pH 7.4) with 1% FCS and 0.02% NaN₃. Cells were permeabilised for 10 min in PBS containing 0.5% TX-100. Antigens were blocked for 60 min in PBS (pH 7.4) containing 1% FCS and 0.02% NaN₃. Cells were stained for 12 min with the nuclear dye DAPI at a concentration of 0.001 mg/ml in PBS. Cells were then washed 2 x 5 min with PBS (pH 7.4) containing 1% FCS and 0.02% NaN₃. The first antibody was applied for 2 h diluted in PBS (pH 7.4) containing 1% FCS and 0.02% NaN₃, at concentrations of: anti-G-GSF 1:1000, anti-G-CSF-R 1:1000, anti-GM-CSF-R 1:1000 (Santa Cruz), respectively. Cells were washed for 3 x 5 min with PBS (pH 7.4) containing 1% FCS and 0.02% NaN₃. The second antibody (goat anti-rabbit IgG-FITC, (DAKO)) was then applied for 60 min in PBS (pH 7.4) containing 1% FCS and 0.02% NaN₃, at a concentration of 1:30. Cells were washed for 3 x 5 min in PBS (pH 7.4) containing 1% FCS and 0.02% NaN₃. Cells were finally mounted with Aquamount and coverslips.

### Example 10- GMCSFR alpha is upregulated in the phototrombotic model of cerebral ischemia (discovery by RMDD) (not part of the invention)

Experimental protocols were approved by the local ethics committee. Male Wistar rats (Charles River, Germany) weighing 280 to 320 g were assigned to the following treatments: a) ischemia for various timepoints (6h, 48h, and 21d); b) sham operation, no ischemia, for accordant timepoints (6h, 48h, and 21d), with n=2 for each timepoint and treatment.

### Focal cerebral ischemia by photothrombosis

Animals were anesthetized with an intramuscular injection of 100 mg/kg body weight ketaminehydrochloride (WDT, Garbsen, Germany). Anesthesia was maintained with 50 mg/kg body weight if necessary. A PE-50 polyethylene tube was inserted into the right femoral artery for continuous monitoring of mean arterial blood pressure, and blood gases. The right femoral vein was cannulated by a PE-50 tube for treatment infusion. During the experiment rectal temperature was monitored and maintained at 37 °C by a thermostatically controlled heating pad (Föhr Medical Intruments, Germany).

Photothrombotic ischemia was induced in the right rat parietal cortex according to the method of Watson BD, Dietrich WD, Busto R, Wachtel MS, Ginsberg MD. Induction of reproducible brain infarction by photochemically initiated thrombosis. Ann Neurol. 1985;17:497-504. Animals were anesthetized with ketaminehydrochloride and placed in a stereotaxic frame, and the scalp was incised for exposure of the skull surface. For illumination, a fiber-optic bundle with a 1.5-mm aperture was placed stereotaxically onto the skull 4 mm posterior to the bregma and 4 mm lateral from the midline. The skull was illuminated with a cold, white light beam (150 W) for 20 minutes. During the first 2 minutes of illumination, the dye rose bengal (0.133 mL/kg body wt, 10 mg/mL saline) was injected intravenously. Sham-operated animals underwent the same experimental procedures as described above without infusion of rose bengal and illumination. After surgery, the catheters were removed, and the animals were allowed to recover from the anesthesia and given food and water *ad libitum.* Animals were killed according to the various timepoints (6h, 48h, and 21d after ischemia and sham operation, respectively) and the preparation of the penumbral cortex both ipsi- and contralateral is known to those skilled in the art.

### RNA isolation and RMDD

RNA was isolated according to standard protocols (Chomczynski and Sacchi (Anal Biochem (1987), 162, 156-9), followed by Qiagen RNeasy mini kit purification) from rat cortical penumbral samples, ipsi- and contralateral to the lesion side (see Fig. 13a for localization of the tissue samples; here: 3 vs. 4). cDNA synthesis was performed from 1 µg total RNA according to the RMDD (restriction mediated differential display)-protocol as described in EP 0 743 367 A2 and US 5,876,932. Following first strand and second strand synthesis, and MboI digestion an adaptor ligation was done. Two PCR reactions with subsets of primer combinations were performed. Subsequently the PCR reactions were loaded on a denaturing gel and blotted on a nylon membrane (GATC Biotech AG, Konstanz, Germany). Biotin-labeled bands were visualised with a common streptavidin-peroxidase reaction. PCR samples from the cortical penumbra were loaded on the gel in the following order: ipsilateral: naive (untreated), sham 6h, sham 48h, sham 21d, and 6h, 48h and 21d photothrombosis; contralateral: sham 6h, sham 48h, sham 21 d, and 6h, 48h and 21d photothrombosis. Bands having different intensity in the ipsi- and contralateral region were cut out of the nylon membrane and reamplification of the according PCR product was performed. Amplified products were cloned in the pCR-BluntII-TOPO vector (Invitrogen GmbH, Karlsruhe, Germany) and sequenced with T7 and M13rev primers (ABI 3700). Obtained sequences were compared with the EMBL-database. A sequence upregulated after 48h both in ipsi- and contralateral cortical penumbra was identified (Fig 14). The identified EST-sequence was extended with BLASTN-searches in EST-databases and a mouse homologous sequence coding for the mouse GM-CSFR alpha was identified in EST- and genomic databases (ensembl; www.ensembl.org) by using screening programs (BLAST, TBLASTN ( Altschul, et al. (1997), Nucleic Acids Res, 25,3389-402.)).

Screens were performed in rat cDNA libraries with the PCR cloning method of Shepard ((1997) Nucleic Acids Res 25:3183-3185) to confirm the obtained rat sequence. This method is based on hybridization of cDNA molecules derived from a plasmid library to a biotin-coupled oligonucleotide sequence. Following plasmid extraction with streptavidin-coupled magnetic beads the result was ensured by diagnostic PCR and two fold replication of the steps following retransformation of the obtained plasmids until recovering the single clone. The following primer combinations were used:
5'block-2.clb4-4-4: CGGGATCCGGGACCGCGTATCTGATGACGAGCGTGTCAA (SEQ ID NO:12)
25bio-2.clb4-4-4: CTCGGAGACGCTGAGGAAGGACCTG (SEQ ID NO:13)
3'block-2.clb4-4-4: CTGCGGCCCTAGACCACGCCCACCGCTCCCCGTGACGTCG (SEQ ID NO:14)
(The ORF was determined for the single clone and the sequence is shown as SEQ ID NO:40, the corresponding amino acid sequence is shown as SEQ ID NO:41).

### Example 11- GMCSF receptor alpha is upregulated in the photothrombotic model of cerebral ischemia (verification by quantitative PCR) (not part of the invention)

RNA was isolated according to standard protocols (Chomczynski and Sacchi (Anal Biochem (1987), 162, 156-9), followed by Qiagen RNeasy mini kit purification), from rat cortical penumbral samples, ipsi- and contralateral to the lesion side (see Fig. 13a for localization of the tissue samples; here: 3 vs. 4). cDNA was synthesized from 1 µg total RNA using oligodT primers, Superscript II reverse transcriptase (Gibco) using standard conditions. Quantitative PCR was performed using the Lightcycler system (Roche Diagnostics, Mannheim, Germany) with SYBR-green staining of DNA doublestrands. Cycling conditions were as follows: 5 min 95°C, 5 sec 95°C, 7 sec 62°C, 30 sec 72°C; 9 sec 80°C for 50 cycles. Melting curves were done with the following parameters: 95°C cooling to 50°C; ramping to 99°C at 0.2°C/ sec. The following primer pairs were used: "rat BR4-4s96" ACGTCGTTGGCTCAGTTATGTC (SEQ ID NO:15), and "rat BR4-4as272" ATTTATGTCAGAGATGGAGGATGG (SEQ ID NO:16). The Lightcycler™ PCR was performed using the SYBR green master mix, following the manufacturer's recommendations (Roche diagnostics). Specificity of product was ensured by melting point analysis and agarose gel electrophoresis. cDNA content of samples was normalized to the expression level of Cyclophilin (primers: "cyc5" ACCCCACCGTGTTCTTCGAC (SEQ ID NO:17); "acyc300" CATTTGCCATGGACAAGATG (SEQ ID NO:18)). Relative regulation levels were derived after normalization to cyclophilin, and comparison to the sham-operated animals. Fig 14 a shows upregulation of the GMCSFR alpha after 48h on the ipsi- and contralateral side. There is no significant regulation detectable 21d after induction of photothrombosis (Fig 14 b). Error bars indicate standard deviations, these are calculated from 3-fold serially diluted cDNA-samples, and reflect reliability of measurements.

### Example 12 - GMCSF-receptor alpha is present on neuronal cells in primary cortical cultures: Preparation of neurons

10-12 Cortices were prepared from embryos of the stage E18 (embryonal day 18). Tissue was dissociated using trypsin [10 mg/ml] /EDTA /DNase [5 mg/ml] (Roche diagnostics) in HBSS (Hanks balanced salt solution, BioWithakker). The digest was stopped using 4 parts medium (neurobasalmedium + 1 ml 50x B-27 supplement (Invitrogen) + 0.5 mM L-glutamin + 25 µM glutamate) and centrifuged at room temperature for 5 min at 800 x g. The pellet was dissolved in 5 ml medium and cell number determined by counting (Neubauer slide). The cells were plated at a density of 250 000 cells per well of a 24-well-plate on cover slips which were coated with poly-L-lysine.

### Immunocytochemistry

1 week after preparation neurons were washed with PBS (Gibco) (37°C) and fixed with 2% paraformaldehyde for 10min on ice. Then, cells were washed with PBS (4°C) and then incubated for 10min in 50mM glycine in PBS, then washed with PBS. Cells were permeabilized on ice using 0,2% TritonX-100 (Sigma) in PBS, and incubated with blocking solution (0,2%Triton-X100, 4% normal goat serum (NGS) (Jackson Immunoresearch Laboratories) in PBS) at room temperature. The primary antibody (rabbit-anti-GM-CSF-Receptor-antibody directed against the C-terminus of mouse GMCSFR, M-20; sc-691; SantaCruz) was used in a dilution of 1:300 (in 0,1% Triton-X100/2% NGS), and incubated overnight at 4°C. Cells were then washed with 1%NGS/PBS, and incubated for 30min with the secondary antibody (anti-rabbit-FITC, 1:400; dianova) at room temperature. Cells were then washed briefly in 1%NGS/PBS, and stained with Hoechst 33342 (Molecular Probes) (1:10.000 in PBS) for counterstaining the nuclei. Finally, cover slips were washed briefly twice in 1%NGS/PBS, twice in PBS, and once for 10 min in 10mM Tris/HCl, pH 7.6. The cover slips were embedded using Aquamount (Polyscience).

Pictures were taken digitally with an Olympus IX81 microscope, and the "Analysis" software package (Soft Imaging Systems, Stuttgart, Germany).

### Example 13 - GMCSF receptor is present on neural stem cells: Generation of Neural stem cells (Figure 13)

Neural stem cells were isolated from the brain areas with known spontaneous neurogenesis, i. e. hippocampus, olfactory bulb, and subventricular zone, of 4-6 week old male Wistar rats as described (Ray J et al (1993) Proc Natl Acad Sci U S A 90: 3602-6.). Protocols are concordant with the policy on the use of animals, as endorsed by the National Research Council of the U.S.A., and fulfill the requirements of German law. Briefly, animals were anesthesized with 1 % (v/v) isoflurane, 70 % N20, 29 % oxygen and sacrificed by decapitation. The brains were removed and washed in 50 mL ice-cold Dulbecco's Phosphate Buffered Saline (DPBS) supplemented with 4.5 g/L glucose (DPBS/Glc). Hippocampus, olfactory bulb, and subventricular zone from 6 animals were dissected, washed in 10 mL DPBS/Glc and centrifuged for 5 min at 1600 x g at 4 °C. After removal of the supernatant, the tissue was homogenized with scissors and scalpels. The tissue pieces were washed with DPBS/Glc medium for 5 min at 800 g, and the three pellets were resuspended in 0.01 % (w/v) papain, 0.1 % (w/v) dispase II (neutral protease), 0.01 % (w/v) DNase I, and 12.4 mM manganese sulfate in Hank's Balanced Salt Solution (HBSS). The tissue was triturated with plastic pipet tips and incubated for 40 min at room temperature, but every 10 min the solution was mixed well. The suspension was centrifuged at 800 x g for 5 min at 4 °C and pellets were washed three times in 10 mL DMEM-Ham's F-12 medium supplemented with 2 mM L-glutamine, 100 units/mL penicillin and 100 units/mL strepotomycin. Then, the cell pellets were resuspended in 1 mL Neurobasal medium supplemented with B27 (Invitrogen, Carlsbad, CA, USA), 2 mM L-glutamine, 100 units/mL penicillin and 100 units/mL strepotomycin, 20 ng/mL EGF, 20 ng/mL FGF-2, and 2 µg/mL heparin. Cells were plated under sterile conditions in 6-well dishes in a concentration of 25,000-100,000 cells/mL. The dishes were incubated at 37 °C in 5 % CO2. Cell culture media were changed once a week, where about two thirds of the media were replaced. (Ray J et al (1993) Proc Natl Acad Sci U S A 90: 3602-6.)

### RT-PCR protocol

RNA was isolated according to standard protocols from hippocampal stern cells that were propagated 3 weeks in culture after thawing them from frozen stocks, following the manufacturers recommendations (RNeasy kit, Qiagen). cDNA was synthesized using oligodT primers, superscript II reverse transcriptase (Gibco) using standard conditions. Polymerase chain reaction (PCR) was performed with the following cycling conditions: 3 min 94°, 30 sec 94°, 30 sec 60°, 1 min 72°; for 32 cycles, using the primer pairs "rat BR4-4s96" ACGTCGTTGGCTCAGTTATGTC (SEQ ID NO:19), and "rat BR4-4as272" ATTTATGTCAGAGATGGAGGATGG (SEQ ID NO:20). Reaction conditions: 2 mM MgCl2, 200 uM dNTP, 200 nM each primer, 1 unit Taq Polymerase (Invitrogen)/25 µl. PCR was resolved on a 1.5% agarose gel. The specific PCR product length was 176 bp with the following sequence (primer sequences are underlined):

### Example 14: Assay for determining the serum half-life and passage of GCSF / GM-CSF through the blood brain barrier

It is desirable to know whether GCSF and GMCSF pass the blood-brain barrier. GCSF / GM-CSF are biotinylated to make use of the highly sensitive avidin-biotin-interaction for detection of the chemokines in brain tissue. G-CSF (Neupogen, Amgen) was biotinylated using Biotin-XX-SE (Molecular Probes B 1606). G-CSF was diluted into 20 mM sodium carbonate buffer pH 8 with 250 mM sorbitol and 0.004 % Tween-80 and Biotin-XX-SE added. After 1 h at room temperature, Tris-buffer pH 8 was added to 50 mM concentration to quench unreacted labeling reagent. The sample was spun 30 min at 45000 rpm in a TLA 110 rotor (Beckman Instruments) to remove aggregates.

7.5 µg biotinylated G-CSF was injected into mice intraperitoneally at time zero (in 200 µl 20 mM sodium carbonate buffer pH 8 with 250 mM sorbitol and 0.004 % Tween-80). Mice were anesthesized with chloralhydrate at times indicated and blood samples ( approx 200 µl) were taken from the right heart chamber. EDTA was added to 5 mM and the sample centrifuged for 10 min at 1000 g to obtain serum. 4 x sample buffer was added to serum, proteins denatured by heating to 95°C for 5 min and 20 µl applied to a minigel. Proteins were transfered to nitrocellulose, blocked and incubated with Streptavidin-HRP (Amersham) in TBST. After washing, signals were detected using Pierce Supersignal chemiluminiscence reagent.

For Elisa analysis serum samples were diluted 1:20 in assay buffer and the assay performed according to the manufacturer's instructions (IBL, Hamburg, Germany).

This assay can be adapted accordingly to cerebrospinal fluid (csf) or brain homogenate to determine the transition of GCSF across the blood-brain-barrier.

### Example 15: Assay for neuroprotective action of GCSF, GMCSF (Figure 1b)

The neuroprotective action of GCSF / GMCSF was determined *in vitro* on NGF-treated PC12 cells. PC12 cells were seeded into 96 well plates coated with poly-1-lysine (0.01% final concentration) at a density of 40.000 cells/well. Cells were kept in DMEM medium containing 1000 mg glucose/l and 10% HS (horse serum) 5% FCS (fetal calf serum), 1% Penicillin/Streptomycin. Cells were then transfected with pSV40-RL (encoding the renilla luciferase gene) using the Lipofectamine2000® transfection agent (Gibco BRL) (0.2 ug DNA / well), following the manufacturers recommendations. Immediately after transfection, NGF (nerve growth factor) was added at a concentration of 40 ng/ml to induce differentiation of PC12 cells. At 24 h after treatment, PC12 cells develop a neuron-like morphology with extended processes. Cells were then treated with H₂O₂ at varying concentrations (Figure 1b), and GCSF at varying concentrations (1-100 ng/ml). EPO was added as a positive control for a substance with known neuroprotective potency in vitro Cerami, et al. (2002), Nephrol Dial Transplant, 17, 8-12. , Kawakami, et al. (2001), J Biol Chem, 276, 39469-75. , Sinor and Greenberg (2000), Neurosci Lett, 290, 213-5. , Chong, et al. (2002), J Cereb Blood Flow Metab, 22, 503-14. ) at concentrations of 0.01 U/ml to 1 U /ml. After 24 h, medium supernatant was discarded, and cells were lysed using the passive lysis buffer (Promega). Renilla luciferase activity was then recorded in a luminometer (Mithras, Berthold), and readings expressed as relative light units. This assay measures cell survival as the amount luciferase detectable. Therefore, the higher the relative light units, the more cells have survived. In this assay, GCSF showed a dose-dependent neuroprotection of PC12 cells, that was more potent than Erythropoetin.

### Example 16 - GCSF receptor is expressed in various brain regions important for neurological diseases (Figure 4); GMCSF is expressed in various important brain regions (Figure 19)

To systematically assess the distribution of the GCSF receptor in the normal mouse brain, C57/b16 mice (2-3 months old) were anesthesized using an i.p. injection of Rompun® and Ketanest®. Mice were then transcardially perfused with 20 ml hanks balanced salt solution (HBSS), followed by 20 ml of 4% paraformaldehyde (PFA) in PBS (pH 7.4). The brain was dissected out, and stored overnight in 2% PFA solution. Paraffin-embedded tissues were sectioned (2 µm), mounted on pre-treated slides (DAKO, Glostrup, Denmark), air-dried overnight and subsequently deparaffinized. After microwave treatment (citrate buffer, 500W, 10 min), the anti-GCSFR antibody (1:400) was applicated and tissues were incubated for 1 h at room temperature in a moist chamber. Antibody labeling was visualized using the routine ABC technique and DAB as a chromogen following manufacturers recommendations (DAKO, Glostrup, Denmark). Negative controls included similarly processed sections in which the primary antibody had been totally omitted as well as sections where the appropriate normal serum was used (Dianova, Hamburg, Germany). Localization of GCSF-R was seen in the hippocampus (Figure 4a-d) with predominant staining of neurons in the CA3 area (Figure 4 a,b), with a sharp boundary between the CA3 and CA2 region (Figure 4 c, arrow). GCSF-R is distributed over the soma, as well as processes of neurons (Figure 4b, arrow). The receptor is present in the hilus and the basal cell layers of the dentate gyrus (Figure 4d, arrow). GCSF-Receptor was also detected in cortical areas: piriform cortex (Figure 4 e), and perirhinal cortex (f) as examples. In the cerebellum, Purkinje cells were labeled (Figure 4g, arrow). Also, some of the large mitral cells in the olfactory bulb are GCSF-R positive (Figure 4h, arrow). Strong staining is exhibited by the anterior columns in the spinal cord (Figure 4 i, j), and higher magnification identifies the large motoneurons as GCSF-R positive ( Figure 4 k,l). Note that the neuronal processes are strongly labeled. In the midbrain, neurons in the substantia nigra show GCSF-R positivity (Figure 4 m,n,o). Apart from neurons, oligodendrocytes in white matter tracts are stained, for example, in the anterior commissure (Figure 4, p, arrow).

The same example applies for the localization of the GMCSFR (Figure 19). Here, staining was seen in the hippocampus, in the cortex, in the cerebellum, and in the choroid plexus. Midbrain and spinal cord were not examined so far.

### Example 17: Assay for neuroprotective action of GCSF, GMCSF (Figure 1b, Figure 23)

The neuroprotective action of GCSF / GMCSF was determined *in vitro* on NGF-treated PC12 cells. PC12 cells were seeded into 96 well plates coated with poly-1-lysine (0.01% final concentration) at a density of 40,000 cells/well. Cells were kept in DMEM medium containing 1000 mg glucose/l and 10% HS (horse serum) 5% FCS (fetal calf serum), 1% Penicillin/Streptomycin. Cells were then transfected with pSV40-RL (encoding the renilla luciferase gene) using the Lipofectamine2000® transfection agent (Gibco BRL) (0.2 ug DNA / well), following the manufacturers recommendations. Immediately after transfection, NGF (nerve growth factor) was added at a concentration of 40 ng/ml to induce differentiation of PC 12 cells. At 24 h after treatment, PC12 cells develop a neuron-like morphology with extended processes. Cells were then treated with H₂O₂ at varying concentrations (Figure 1b, Figure 23), and GCSF and GMCSF at varying concentrations (1-100 ng/ml). EPO was added as a positive control for a substance with known neuroprotective potency in vitro (Cerami, et al. (2002), Nephrol Dial Transplant, 17, 8-12., Kawakami, et al. (2001), J Biol Chem, 276, 39469-75. , Sinor and Greenberg (2000), Neurosci Lett, 290,213-5. , Chong, et al. (2002), J Cereb Blood Flow Metab, 22, 503-14. ) at concentrations of 0.01 U/ml to 1 U /ml (Figure 1b), or 0.5 U/ml (Figure 23). After 24 h, medium supernatant was discarded, and cells were lysed using the passive lysis buffer (Promega). Renilla luciferase activity was then recorded in a luminometer (Mithras, Berthold), and readings expressed as relative light units. This assay measures cell survival as the amount luciferase detectable. Therefore, the higher the relative light units, the more cells have survived. In this assay, GCSF as well as GMCSF showed a dose-dependent neuroprotection of PC 12 cells that was more potent than Erythropoetin.

### Example 18: Thrombembolic cerebral ischemia (not part of the invention)

Experimental protocols will be approved by the local ethics committee. Forty male Wistar rats (Charles River, Germany) weighing 280 to 320 g will be randomly assigned to the following groups: A) early thrombolysis with 10 mg rt-PA/kg body weight for 1 hour, 1 hour after thrombembolic vessel occlusion; B late thrombolysis with 10 mg rt-PA/kg body weight for 1 hour, 3 hour after thrombembolic vessel occlusion; C) no thrombolysis, but treatment with 60 µg /kg body weight of recombinant G-CSF (Neupogen^{®},Amgen, Europe B.V., Netherlands) in 2 ml saline 0.9% for 90 min beginning 30 min after thrombembolic ischemia; D) treatment with 60 µg /kg body weight of recombinant G-CSF (Neupogen^{®}, Amgen, Europe B.V., Netherlands) in 2 ml saline 0.9% for 90 min beginning 30 min after thrombembolic ischemia combined with late thrombolysis with 10 mg rt-PA/kg body weight for 1 hour, 3 hour after thrombembolic vessel occlusion.

Animals will be anesthetized with an intraperitoneal injection of 100 mg/kg body weight ketaminehydrochloride (WDT, Garbsen, Germany). Anesthesia will be maintained with 50 mg/kg body weight, if necessary. A PE-50 polyethylene tube will be inserted into the right femoral artery for continuous monitoring of mean arterial blood pressure, blood gases, hematocrit, leukocyte count and blood glucose levels. The right femoral vein will be cannulated by a PE-50 tube for treatment infusion. During the experiment rectal temperature will be monitored and maintained at 37 °C by a thermostatically controlled heating pad (Föhr Medical Intruments, Germany).

Thromboembolic stroke will be induced according to the modified method described by Busch et al (Brain Res 1997 Dec 5;778(1):16-24). Briefly, the right common carotid (CCA), internal carotid (ICA) and external carotid arteries (ECA) will be exposed through a midline incision of the neck. Further dissection identified the origin of the pterygopalatine artery (PPA). The ECA and the PPA will be permanently ligated by a 6-0 silk suture. The CCA will be only temporarily ligated for the time of embolization. A catheter will be inserted into the ECA proximal to its ligation and 12 red blood clots (each 0.35 mm in diameter and 3 mm in length) were injected, resulting in embolization of the right middle cerebral artery (MCA).

Infarct evolution will be monitored by MR-imaging at 1, 2, 4, and 24 hours by using diffusion-, perfusion-, and T2- weighted imaging. In all animals, outcome will be measured by mortality as well as neurological outcome based on a five point scale 24 hours after ischemia. 24 hours after ischemia, the rats will be anesthetized with ketamine 150 mg/kg body weight and decapitated. The brains will be removed, and fixed with 4% paraformaldehyde in 0.1 mol/l phosphate buffer for 24 hrs. After paraffin-embedding, 1-µm-thick sections will becut and used for TTC, H&E, and Nissl staining and immunohistochemical analysis.

### Statistical analysis

The values will be means ± SD. After acquiring all the data, the randomization code will be broken. ANOVA and subsequent post hoc Fisher protected least significant difference test will be used to determine the statistical significance of differences in continuous variables such as physiological parameters, and infarct volume. The Mann-Whitney U test will be performed for nonparametric data such as the mortality rate. A p value <0.05 will be considered statistically significant.

### Example 19: G-CSF is effective when given at an extended time window in middle cerebral artery occlusion (MCAO), a rodent stroke model (not part of the invention)

Figure 24 shows the effect of intravenously applied G-CSF on infarct volume in the rat MCAO model when applied 120 min after onset of ischemia. The experiment was conducted as described in Example 1, with the exception that anesthesia was induced by inhalation anesthesia (1% halothane, 30 % O₂, and 70% N₂O). Also here, a dose of 60 µg G-CSF (Neupogen®, AMGEN) /kg bodyweight of the rat was used. A significant protection was seen when comparing infarct volumes by TTC-staining (see Figure 25). The operations were performed by another set of investigators as those in example 1, demonstrating efficacy in another laboratory setup. This example further demonstrates the usefulness of G-CSF as a treatment for stroke and other ischemic disorders of the nervous system.

### Example 20: Colocalisation of GCSF and its receptor in the brain

### Immunohistochemical methods used

Sections of paraffin-embedded tissues (2 µm) were deparaffinated by treating them 2 x 5 min with Xylol, 2 x 2 min 100% ethanol, and then with descending concentrations of ethanol from 96% up to 70%. Finally the sections are washed with distilled water and microwaved (citrate buffer at 600 W for 15 min). Afterwards, sections were washed with distilled water and antigens were blocked 3 x 5min in 1 x TBS (pH 7.4) containing 0.2% BSA. The GCSF-receptor antiserum (SC694; Santa Cruz Biotechnology, Santa Cruz, CA, USA; 1:100) diluted in 1 x TBS (pH 7.4) containing 0.2% BSA was incubated at room temperature for 1h in a humid chamber. Sections were then washed 3 x 2min with 1 x TBS (pH 7.4) containing 0.2% BSA. The second antibody (goat anti-rabbit Fab-FITC, dianova, 1:50) was then applied over night at 4°C in 1 x TBS (pH 7.4) containing 0.2% BSA. After washing the sections 3 x 2min with 0.2% BSA in 1 x TBS (pH 7.4), the GCSF antiserum (SC13102; Santa Cruz Biotechnology, Santa Cruz, CA, USA) diluted 1:100 in 0.2% BSA in 1 x TBS (pH 7.4) was applied for 1h at room temperature. The sections were washed 3 x as described before and incubated for 30 min with biotinylated goat anti-rabbit IgG (Vector Laboratories, USA) diluted 1:200 in 1 x TBS (pH 7.4) containing 0.2% BSA. Again after washing the sections, TRITC-conjugated streptavidin (dianova; 1:200) was applied for 1.5 h at room temperature. Then, the sections were washed 3 x 2min with 1 x TBS and stained for 10 min with the nuclear dye DAPI diluted 1:10 000 in 1 x TBS. Finally the sections were washed 3 x 2min with 1 x TBS and mounted with mounting medium for fluorescence (Vectashield, Vector Laboratories, USA). Pictures were taken digitally with an Olympus IX81 microscope, and the "Analysis" software package (Soft Imaging Systems, Stuttgart, Germany).

All double-fluorescence experiments were controlled by parallel single-staining, which were checked for absence of any fluorescence carry-over in the second channel. As a second control, all double-fluorescence staining were done with switched chromophores for the secondary antibody.

### Results

The GCSF receptor (Fig 25 a, d, g) shows a co-localization with its ligand (Fig 25 b, e, h) both in the hippocampus (Fig 25 a-c dentate gyrus, d-f hilus) and the cortex (Fig 25 g-i). The surprising finding that the same neurons express both the receptor and the ligand, suggest an autocrine signaling mechanism of GCSF, which supports a novel endogenous neuroprotective system of the nervous system.

### Example 21: Colocalisation of the GCSF receptor and the GMCSF receptor in the brain

### Immunohistochemical methods

Immunohistochemistry was performed as described in example 20. After incubating the sections with the GCSFR antiserum and goat anti-rabbit Fab-FITC, the sections were washed 3 x 2min with 0.2% BSA in 1 x TBS (pH 7.4). Afterwards, the GMCSFR antiserum (SC690; Santa Cruz Biotechnology, Santa Cruz, CA, USA; 1:100) was applied for 1h at room temperature. The following procedure including incubation with biotinylated goat anti-rabbit IgG (Vector Laboratories, USA) and TRITC-conjugated streptavidin (dianova; 1:200) was done as described for GCSF detection in example 2.

All double-fluorescence experiments were controlled by parallel single-stainings, which were checked for absence of any fluorescence carry-over in the second channel. As a second control, all double-fluorescence stainings were done with switched chromophores for the secondary antibody.

### Results

The GCSF receptor (Fig 26 a, d, g) and the GMCSF receptor (Fig 26 b, e, h) are expressed on the same neurons in both the hippocampus and the cortex (Fig 26 c, f, i). Fig 26 shows coexpression of both receptors on neurons in the dentate gyrus (a-c) and the hilus (d-f) of the hippocampus as well as in the cortex (g-i). This finding further illustrates the claim that GCSF and GMCSF could be used in conjunction to treat neurological conditions, and that the neuroprotective properties of hematopoietic factors could be enhanced by combined application.

### Example 22: GCSF acts anti-apoptotically by activating stat3 in neurons

### Results

Primary cultured neurons are a tool to dissect mechanisms of action. We therefore checked whether the G-CSF receptor would be expressed on hippocampal or cortical neurons after 21 days in culture. Indeed, we confirmed expression by PCR (previous examples) and immuno-cytochemistry, both on cortical as well as most hippocampal neurons. One of the most important mechanisms in stroke pathophysiology is delayed neuronal cell death (Choi (1996), Curr Opin Neurobiol, 6, 667-72, Schneider, et al. (1999), Nat Med, 5, 554-9, Mattson (2000), Nat Rev Mol Cell Biol, 1, 120-9). We therefore hypothesized that G-CSF might be interfering with apoptotic cascades in neurons. In primary neuronal cultures that were found to express the G-CSF receptor, exposure to nitric oxide, a relevant event during cerebral ischemia, leads to dose-dependent increase in programmed cell death as evidenced by PARP-cleavage (not shown). Treatment with 50 ng/ml G-CSF drastically reduced apoptotic cell death after NOR3 treatment, as exemplified in Figure 27. In cells of the hematopoietic lineage, the GM-CSF receptor transmits its anti-apoptotic signal via the Janus kinase 2 (JAK2) and signal transducer and transactivator (stat) proteins (Jak-stat pathway) (e.g. ( Epling-Burnette, et al. (2001), J Immunol, 166, 7486-95, Sakamoto, et al. (2003), Int J Hematol, 77, 60-70)). While we could not detect activation by phosphorylation of stat1 or stat5 (Figure 27, part III), stat3 was strongly phosphorylated by the addition of G-CSF in a time-dependent manner typical (Figure 27, part IV) of the JAK-stat kinetics in other cell types (Figure 27, part V; taken and adapted from : Kuroki, M. & O'Flaherty, J. T. Extracellular signal-regulated protein kinase (ERK)-dependent and ERK-independent pathways target STAT3 on serine-727 in human neutrophils stimulated by chemotactic factors and cytokines. Biochem J 341 ( Pt 3), 691-6 (1999).). The stat3 tyr705 phosphorylation evoked by the addition of G-CSF to the culture medium was specifically mediated via the G-CSF receptor / JAK2 pathway, as blocking of the JAK2/ stat3 connection by the inhibitor AG490 led to drastically reduced signals in the presence of G-CSF (Figure X). Stat3 activation leads to induction of anti-apoptotic proteins of the bcl family ( Yoshida, et al. (2002), J Exp Med, 196, 641-53, Sakai and Kraft (1997), J Biol Chem, 272, 12350-8, Nielsen, et al. (1999), Leukemia,13, 735-8). Addition of G-CSF to neuronal cultures led to a time-dependent increase of both Bcl-X1 and Bcl-2, potent anti-apoptotic proteins in neurons, and during cerebral ischemia (Figure 27, part VI). Interestingly, a recent report suggests stat3 as an essential survival protein for motoneurons after nerve injury ( Schweizer, et al. (2002), J Cell Biol, 156, 287-97). G-CSF therefore seems to act differently from the proposed anti-apoptotic mechanism of EPO involving stat5 and NF-kB activation ( Digicaylioglu and Lipton (2001), Nature, 412, 641-7).

### Methods

Primary cortical neurons from rats were generated as follows: Ten to 12 cortices were dissected from rat embryos E18. The tissue was dissociated using 10 mg/ml trypsin, 5 mg/ml EDTA /DNase (Roche diagnostics, Mannheim, Germany) in HBSS (BioWhitakker, Taufkirchen, Germany). The digestion was stopped using four parts neurobasal medium containing 1X B-27 supplement (Invitrogen, Karlsruhe, Germany). After centrifugation, the pellet was dissolved in 5 ml medium and cells were plated at a density of 250,000 cells per well of a 24-well-plate on glass cover slips coated with poly-L-lysine. The medium typically contains: 50 ml neurobasal medium (life technologies, Karlsruhe, Germany), 1 ml supplement B 27 (Life technologies), 5 µl bFGF (Sigma,19 µg was dissolved in 100 µl 10 mM Tris/HCl pH 7), and 50 µl Penicillin / Streptomycin (Life technologies).

Detection of STAT1, pSTAT1, STAT5, pSTAT5, GCSFR, PARP, cleaved PARP, caspase3, cleaved caspase3, Bcl2, and Bcl xl was done by Western blotting. In brief, neurons were treated for 24 h with 150 mM NOR-3 (Sigma-Aldrich, Seelze, Germany), and 50 ng/ml G-CSF (Neupogen, Amgen, Thousand Oaks, CA, USA) as indicated. The cells were scraped off the plate, spun down at 800 rpm for 5 min, and washed in ice-cold PBS containing 2.5 mg/ml pepstatin (Sigma-Aldrich, Seelze, Germany) and aprotinin (1:1000, Sigma-Aldrich). Pellets were resuspended in 100 µl benzonase solution (containing 10 µl 10x PBS, 79,5 µl H2O, 10 µl 10% SDS, 0.5 µl 100 mM MgCl₂, 0.1 µl Aprotinin, 0.1 µl Leupeptin, 0.1 µl Pepstatin, 0.1 µl PMSF, 0.1 µl benzonase (Roche Diagnostics, Mannheim, Germany)) was added. After solubilization, 1 volume PBS was added and the protein concentration determined (BCA-Test, Pierce, Rockford, IL, USA). After denaturing at 95°C for 5 min, 100 µg was run on 8% - 12% SDS-polyacrylamide gels. Proteins were transferred to nitrocellulose membranes (Protan BA79, Schleicher & Schuell, Dassel, Germany) using a semi-dry-blotting chamber (Whatman Biometra, Göttingen, Germany). Blots were blocked with 5% milk powder in PBS/0.02% Tween 20, washed three times with PBS/0.02% Tween 20, and incubated overnight at 4°C with the respective primary antibody (anti-cleaved-PARP-antibody, Cell Signaling, 1:1000; anti PARP, Cell Signaling, 1:1000; anti cleaved caspase 3, Cell Signaling, 1:200; anti caspase3, Cell Signaling, 1:200; anti Bcl2, BD Transduction Laboratories, 1:500; anti Bcl Xl, BD Transduction Laboratories, 1:500; anti pSTAT3tyr, Cell Signaling, 1:500; anti STAT3, Cell Signaling, 1:500; anti GCSFR, Santa Cruz, 1:200; anti pSTAT5, Cell signaling, 1:500; anti stat5, Cell signaling, 1:200; anti pSTAT1, Cell signaling, 1:500). After washing, the blots were incubated with the respective secondary antibody (anti-rabbit, or anti-mouse-antiserum HRP-coupled, Dianova, Hamburg, Germany 1:4000) for 1 h at room temperature. Signals were detected using the supersignal chemiluminescence system (Pierce, Rockford, USA) and exposed to Hyperfilm-ECL (Amersham Pharmacia Biotech, Piscataway, NJ, USA). PARP cleavage was quantified on scanned autoradiographs using Windows ImageJ v1.29 (http://rsb.info.nih.gov/ij/index.html).

### Example 23: GCSF and its receptor are induced by cerebral ischemia - not part of the invention

### MCAO model of cerebral ischemia

Procedure for inducing focal cerebral ischemia (MCAO, middle cerebral artery occlusion) was performed as described in example 1.

### Global ischemia model

The experimental procedures on animals randomized for ischemia/reperfusion were performed as previously described in details (Brambrink, et al. (2000), J Cereb Blood Flow Metab, 20,1425-36). In brief, the rats were anesthetized (chloral hydrate), orally intubated and mechanically ventilated (Pa_{CO2} at 35-40 mmHg, Pa_{CO2} at 95-110 mmHg throughout the experiment). Both common carotid arteries (CCA) were exposed and the left side was catheterized with polyethylene tubing (PE-50) for blood sampling and continuous monitoring of MABP (Sirecust 310, Siemens, Danvers, MA, USA). On the right side a nylon thread was looped loosely around the CCA to be used later to induce transient cerebral ischemia. The head of the animal was fixed in a stereotaxic frame for EEG and CBF measurements (for details see (Brambrink, Schneider, Noga, Astheimer, Gotz, Komer, Heimann, Welschof and Kempski (2000), J Cereb Blood Flow Metab, 20, 1425-36)). The calvarium was exposed by a median incision and two depressions designed to hold chlorinated silver pin EEG electrodes were made (high speed dental drill, positioned over the left somatosensory cortex and frontal sinus according to the Praxinos and Watson (1986) brain atlas). Over the right hemisphere about 24 mm² of the skull bone was removed leaving a thin bone lamina (1.3 - 5.3 mm lateralto the right and 1.5 - 7.5 mm occipital from the bregma). A laser Doppler-flow probe (wavelength: 780 µm, *BPM 403A*, TSI Inc., St. Paul, MN, USA) controlled with a computer driven micromanipulator was employed to determine regional cerebral blood flow (rCBF) at 30 locations, using the "scanning technique" (Soehle, et al. (2000), Acta Neurochir Suppl, 76, 181-4)). On the contralateral side (3.3 - 5.3 mm lateral to the left and 5.0 - 7.0 mm occipital of the bregma) a smaller (4 mm²) window was established to measure local cerebral blood flow (ICBF), using a stationary laser Doppler-flow probe (wavelength: 780 µm, *BPM2*, Vasamedices Inc., St. Paul, MN, USA). Temperature probes were placed as described previously ((Brambrink, et al. (1999), J Neurosci Methods, 92, 111-22)) in the right temporal muscle, in the left auricular tube, and at 6 cm depth in the rectum. Core temperature (thermostatically controlled heating blanket) and temporal muscle temperature (near infarcted heat radiator) were controlled to 37.5 ± 0.1°C throughout the experimental procedures. The lower body section of the animal was placed in an air-tight chamber. This allows to establish hypobaric pressures by an electronically regulated vacuum pump, thereby reducing arterial blood pressure of the animal (venous pooling) in a controlled fashion ("hypobaric hypotension"). After a 30-minutes post surgical stabilization period, transient global cerebral ischemia was initiated by pulling the nylon thread attached to a defined weight around the right carotid artery to occlude the vessel (arterial pressure was measured by a catheter placed in the left carotid artery); MABP was simultaneously reduced to 35 mm Hg, using the hypobaric hypotension technique. Brain ischemia was confirmed by continuous ICBF, rCBF, and EEG measurements. After 15 minutes of global cerebral ischemia, the thread was cut and vacuum was terminated to allow reperfusion. After 90 minutes of recovery the CCA catheter was removed, incisions were closed, and the animals were weaned from the ventilator. On extubation (around 110 minutes of reperfusion) and in the presence of stabile vital signs, the rats were returned to their cage and a heating blanket was used to prevent heat loss.

### Quantitative PCR

RNA was isolated according to standard protocols ((Chomczynski and Sacchi (1987), Anal Biochem, 162, 156-9), followed by Qiagen RNeasy™ mini kit purification. Tissue samples were taken from total rat brain after global cerebral ischemia, and ipsi- and contralateral to the lesion side at various timepoints after focal cerebral ischemia, respectively. cDNA was synthesized from 10 µg total RNA using oligodT primers, superscript II reverse transcriptase (Gibco) using standard conditions. Quantitative PCR was performed using the Lightcycler® system (Roche Diagnostics, Mannheim, Germany) with SYBR-green staining of DNA doublestrands. Cycling conditions were as follows: GCSFR: 5 min 95°C, 5 sec 95°C, 10 sec 66°C, 30 sec 72°C; 10 sec 84°C for 50 cycles; GCSF: 5 min 95°C, 5 sec 95°C, 10 sec 64°C, 30 sec 72°C; 10 sec 88°C for 50 cycles. Melting curves were done with the following parameters: 95°C cooling to 50°C; ramping to 99°C at 0.2°C/ sec. The following primer pairs were used: "rat GCSFR-frag-32s" CCA TTG TCC ATC TTG GGG ATC (SEQ ID NO:42), "rat GCSFR-frag-265as" CCT GGA AGC TGT TGT TCC ATG (SEQ ID NO:43), "rat GCSF-345s" CAC AGC GGG CTC TTC CTC TAC CAA (SEQ ID NO:44), and "rat GCSF-862as" AGC AGC GGC AGG AAT CAA TAC TCG (SEQ ID NO:45). The Lightcycler® PCR was performed using the SYBR green master mix, following the manufacturer's recommendations (Roche Diagnostics). Specificity of products was ensured by melting point analysis and agarose gel electrophoresis. cDNA content of samples was normalized to the expression level of Cyclophilin (primers: "cyc5" ACC CCA CCG TGT TCT TCG AC (SEQ ID NO:46); "acyc300" CATTTGCCATGGACAAGATG (SEQ ID NO:47)). Relative regulation levels derived from normalization to cyclophilin, and comparison to the sham-operated animals. Error bars indicate standard deviations, these are calculated from 3-fold serially diluted cDNA-samples, and reflect reliability of measurements.

Fig 28 (part I and II) A shows a very strong upregulation of GCSF 2h and 6h after focal ischemia on the ipsi- and contralateral side, whereas the receptor is moderately regulated (Fig. 28 B) after 6h.

An induced expression of GCSF is not specific to the MCAO model, but could also be seen albeit to a lesser degree in global ischemia (Figure 28 C). This finding supports the hypothesis that G-CSF indeed is a brain-endogenous neuroprotective ligand, and that treatment with GCSF or GMCSF utilizes an endogenous principle of neuroprotection.

### Example 24: GCSF and its receptor are upregulated in the penumbral zone of a cortical infarct - not part of the invention

### Methods

The ischemic model of photothrombotic cortical ischemia was conducted as described in above examples.

Immunohistochemistry: Sections of paraffin-embedded tissues (2 µm) were deparaffinated and microwaved (citrate buffer at 500 W for 10 min). Afterwards, sections were incubated at room temperature with GCSF or GCSF-receptor antiserum (SC13102 or SC694; Santa Cruz Biotechnology, Santa Cruz, CA, USA; 1:500) for 1 h in a humid chamber. Staining was visualized using the ABC technique with DAB as chromogen (DAKO, Glostrup, Denmark). Negative controls were done by omitting the primary antiserum.

### Results

6 h following induction of photothrombotic ischemia, there was clear evidence for enhanced staining of neurons in the surrounding area of the primary ischemic lesion ("penumbra"), as shown by arrows in Figure 29, both for the receptor and the lignad. This finding underlines the principle that we have uncovered a novel endogenous neuroprotective system of the nervous system. It also underlines that the primary mechanism of action of GCSF in the penumbral zone is indeed most likely antiapoptotic.

### Example 25: Colocalisation of the GCSFR and doublecortin in the hippocampus

### Immunohistochemical methods used

The experiment was performed as described in example 21. Instead of the GCSF antiserum, a guinea-pig anti-doublecortin (DCX) polyclonal antibody (Chemicon International, Germany) diluted 1:200 in 0.2% BSA in 1 x TBS (pH 7.4) was applied. After washing the sections 3 x 2min with 0.2% BSA in 1 x TBS (pH 7.4), they were incubated for 30 min with biotinylated goat anti-guinea-pig IgG (Vector Laboratories, USA) diluted 1:200 in 1 x TBS (pH 7.4) containing 0.2% BSA. Again after washing the sections, TRITC-conjugated streptavidin (dianova; 1:200) was applied for 1.5 h at room temperature following the protocol described in example 21.

### Results

Fig 30 shows the expression of GCSF receptors (a, d, g) and doublecorrtin (b, e, h) on neurons in the dentate gyrus (a-f) and the hilus (g-i) of the hippocampus. The overlap of the expression of both the G-CSF receptor and the early neuronal marker doublecortin ( Jin, et al. (2001), Proc Natl Acad Sci U S A, 98, 4710-5) (Fig 30 c, f, i) implicate a functional role of G-CSF at an early stage of neuronal differentiation.

### Example 26: GCSF induces neuronal differentiation of adult neural stem cells

### Generation of Neural stem cells (NSCs)

Generation of adult neural stem cells from rat was performed as described in example 9.

39 DIV cultured neurospheres derived from the subventricular zone (SVZ) were stimulated once with the following GCSF-concentrations: 10ng/ml, 100ng/ml and 500ng/ml, respectively. 4 days after addition of recombinant human GCSF (Neupogen^{®}, Amgen, Europe B.V., Netherlands) cells were harvested and RNA was isolated. Untreated cells served as control.

### Quantitative PCR

RNA of GCSF-treated and untreated neurospheres of the SVZ was isolated using the Qiagen RNeasy mini kit following the manufacturers protocol. cDNA was synthesized from 2 µg total RNA using oligodT primers, superscript II reverse transcriptase (Gibco) using standard conditions. Quantitative PCR was performed using the Lightcycler system (Roche Diagnostics, Mannheim, Germany) with SYBR-green staining of DNA doublestrands. Cycling conditions were as follows:

Nestin and NSE (neuron specific enolase): 3 min 95°C, 5 sec 95°C, 10 sec 58°C, 30 sec 72°C; 10 sec 81°C for 50 cycles; beta III-tubulin: 3 min 95°C, 5 sec 95°C,10 sec 65°C, 30 sec 72°C; 10 sec 87°C for 50 cycles; PLP: 3 min 95°C, 5 sec 95°C, 10 sec 62°C, 30 sec 72°C; 10 sec 84°C for 50 cycles; GFAP: 3 min 95°C, 5 sec 95°C, 10 sec 60°C, 30 sec 72°C; 10 sec 81°C for 50 cycles. Melting curves were determined using the following parameters: 95°C cooling to 50°C; ramping to 99°C at 0.2°C/ sec. The following primer pairs were used: "rat nestin-plus" AGG AAG AAG CTG CAG CAG AG (SEQ ID NO:48), "rat nestin - minus" TTC ACC TGC TTG GGC TCT AT (SEQ ID NO:49), "rat NSE-plus" GGC AAG GAT GCC ACT AAT GT (SEQ ID NO:50), "rat NSE -minus" AGG GTC AGC AGG AGAC TTG A (SEQ ID NO:51), "rat beta III-tub-716s" CCA CCT ACG GGG ACC TCA ACC AC (SEQ ID NO:52), "rat beta III-tub-1022as" GAC ATG CGC CCA CGG AAG ACG (SEQ ID NO:53), "rat PLP-518s" TCA TTC TTT GGA GCG GGT GTG (SEQ ID NO:54), "rat PLP-927as" TAA GGA CGG CAA ACT TGT AAG TGG (SEQ ID NO:55), "rat GFAP3'-1123s" CCT TTC TTA TGC ATG TAC GGA G (SEQ ID NO:56), "rat GFAP3'-1245as" GTA CAC TAA TAC GAA GGC ACT C (SEQ ID NO:57). The Lightcycler PCR was performed using the SYBR green master mix, following the manufacturer's recommendations (Roche diagnostics). Specificity of product was ensured by melting point analysis and agarose gel electrophoresis. cDNA content of samples was normalized to the expression level of Cyclophilin (primers: "cyc5" ACC CCA CCG TGT TCT TCG AC (SEQ ID NO:58), "acyc300" CAT TTG CCA TGG ACA AGA TG (SEQ ID NO:59)). Relative regulation levels derived from normalization to cyclophilin, and comparison to the untreated cells. Fig 31 shows a strong and concentration dependent upregulation of the neuronal markers NSE and beta III-tubulin 4d after GCSF treatment. PLP and GFAP are moderately regulated in depedency of the GCSF-concentration. Error bars indicate standard deviations, calculated from 3-fold serially diluted cDNA-samples, and reflect reliability of measurements.

### Example 27: Colocalisation of GMCSF and its receptor in the brain

### Immunohistochemical methods used

Sections of paraffin-embedded tissues (2 µm) were deparaffinated by treating them 2 x 5 min with Xylol, 2 x 2 min 100% ethanol, and then with descending concentrations of ethanol from 96% up to 70%. Finally the sections are washed with destilled water and microwaved (citrate buffer at 600 W for 15 min). Afterwards, sections were washed with destilled water and antigens were blocked 3 x 5min in 1 x TBS (pH 7.4) containing 0.2% BSA. The GMCSF receptor antiserum (SC690; Santa Cruz Biotechnology, Santa Cruz, CA, USA; 1:100) diluted in 1 x TBS (pH 7.4) containing 0.2% BSA was incubated at roomtemperatur for 1h in a humid chamber. Sections were then washed 3 x 2min with 1 x TBS (pH 7.4) containing 0.2% BSA. The second antibody (goat anti-rabbit Fab-FITC, dianova, 1:50) was then applied over night at 4°C in 1 x TBS (pH 7.4) containing 0.2% BSA. After washing the sections 3 x 2min with 0.2% BSA in 1 x TBS (pH 7.4), the GMCSF antiserum (SC13101; Santa Cruz Biotechnology, Santa Cruz, CA, USA) diluted 1:100 in 0.2% BSA in 1 x TBS (pH 7.4) was applied for 1h at room temperature. The sections were washed 3 x as described before and incubated for 30 min with biotinylated goat anti-rabbit IgG (Vector Laboratories, USA) diluted 1:200 in 1 x TBS (pH 7.4) containing 0.2% BSA. Again after washing the sections, TRITC-conjugated streptavidin (dianova; 1:200) was applied for 1.5 h at room temperature. Then, the sections were washed 3 x 2min with 1 x TBS and stained for 10 min with the nuclear dye DAPI diluted 1:10 000 in 1 x TBS. Finally the sections were washed 3 x 2min with 1 x TBS and mounted with mounting medium for fluorescence (Vectashield, Vector Laboratories, USA). Pictures were taken digitally with an Olympus IX81 microscope, and the "Analysis" software package (Soft Imaging Systems, Stuttgart, Germany).

All double-fluorescence experiments were controlled by parallel single-stainings, which were checked for absence of any fluorescence carry-over in the second channel. As a second control, all double-fluorescence stainings were done with switched chromophores for the secondary antibody.

### Results

The GMCSF receptor (Fig 32 a, d, g) and GMCSF (Fig 32 b, e, h) are colocalised on neurons in the dentate gyrus (Fig 32 a-c), the hilus (Fig 32 d-f), and the cortex (Fig 32 g-i). These data support the notion that GMCSF is an autocrine ligand.

### Example 28: GMCSF and its receptor are upregulated by cerebral ischemia - (not part of the invention)

### MCAO model of cerebral ischemia

Procedure for inducing focal cerebral ischemia (MCAO, middle cerebral artery occlusion) was performed as described in example 1.

### Global ischemia model

Surgical preparations and transient global cerebral ischemia were performed as described before.

### Quantitative PCR

RNA isolation and cDNA synthesis were done following the protocol described before. Cycling conditions were as follows: GMCSFR: 5 min 95°C, 5 sec 95°C,10 sec 62°C, 30 sec 72°C; 10 sec 80°C for 50 cycles; GMCSF: 5 min 95°C, 5 sec 95°C,10 sec 60°C, 30 sec 72°C; 10 sec 81°C for 50 cycles. Melting curves were done with the following parameters: 95°C cooling to 50°C; ramping to 99°C at 0.2°C/ sec. The following primer pairs were used: rat GMCSFR "BR4-4s96" ACG TCG TTG GCT CAG TTA TGT C (SEQ ID NO:60), "BR4-4as272" ATT TAT GTC AGA GAT GGA GGA TGG (SEQ ID NO:61), "rat GMCSF-723s" GGA GCT CTA AGC TTC TAG ATC (SEQ ID NO:62), and "rat GMCSF-908as" GGC TCA ATG TGA TTT CTT GGG (SEQ ID NO:63). The Lightcycler® PCR was performed using the SYBR green master mix, following the manufacturer's recommendations (Roche Diagnostics). By melting point analysis and agarose gel electrophoresis the specificity of products was ensured. cDNA content of samples was normalized to the expression level of Cyclophilin (primers: "cyc5" ACC CCA CCG TGT TCT TCG AC (SEQ ID NO:58); "acyc300" CATTTGCCATGGACAAGATG (SEQ ID NO:59)). Relative regulation levels derived from normalization to cyclophilin, and comparison to the sham-operated animals. Error bars indicate standard deviations, these are calculated from 3-fold serially diluted cDNA-samples, and reflect reliability of measurements.

A very strong upregulation of GMCSF 2h and 6h after focal ischemia on the ipsi- and contralateral side is shown in Fig 33A. An induction of GMCSF can be shown in global ischemia as well albeit to a lesser extend (Fig 33 B). Even the GMCSF receptor is slightly upregulated 6h after global ischemia (Fig 33 C). These data support the hypothesis that GMCSF is part of an endogenous neuroprotective mechanism.

### Example 29: Colocalised expression of the GCSFR and doublecortin in the hippocampus

### Immunohistochemical methods used

The experiment was performed as described before. Instead of the GMCSF antiserum, a guinea-pig anti-doublecortin (DCX) polyclonal antibody (Chemicon International, Germany) diluted 1:200 in 0.2% BSA in 1 x TBS (pH 7.4) was applied. After washing the sections 3 x 2min with 0.2% BSA in 1 x TBS (pH 7.4), they were incubated for 30 min with biotinylated goat anti-guinea-pig IgG (Vector Laboratories, USA) diluted 1:200 in 1 x TBS (pH 7.4) containing 0.2% BSA. After again washing the sections, TRITC-conjugated streptavidin (dianova; 1:200) was applied for 1.5 h at room temperature following the protocol described before.

### Results

Fig 34 shows the expression of the GMCSF receptor (a, d, g) and doublecortin (b, e, h) on neurons in the dentate gyrus (a-c) and the hilus (d-i) of the hippocampus. The overlap of the expression of both the G-CSF receptor and the early neuronal marker doublecortin (Jin, Minami, Lan, Mao, Batteur, Simon and Greenberg (2001), Proc Natl Acad Sci U S A, 98, 4710-5) (Fig 34 c, f, i) implicate a functional role of GMCSF at an early stage of neuronal differentiation, and underline the applicability of GMCSF to all neurodegenerative diseases, where influencing neurogenesis may be a therapeutic target, such as stroke, Parkinson's disease, ALS, and many others.

### Example 30: GMCSF induces neuronal differentiation of adult neural stem cells

### Generation of Neural stem cells

Generation of adult neural stem cells from rat was performed as described in example 9. Cells were passaged every two to three weeks by centrifugating the cells for 5 min at 800 x g, removing the supernatant, and washing them once with 1 x PBS. After resuspension in 1 ml Accutase (Sigma) and incubation for 15 min at 37°C the cells were counted and plated at a density of 1.5-2.0 x 10⁵ cells per well in a 6 well plate. Neural stem cells derived from the hippocampus were stimulated after the 4^{th} passage with 10ng/ml GMCSF (Leukine®, Berlex, Schering AG Germany), and after 3 days they were harvested for RNA isolation. Untreated cells served as a control.

### Quantitative PCR

RNA of the GMCSF-treated and untreated neurospheres of the SVZ was isolated using the Qiagen RNeasy mini kit following the manufacturers recommendations. cDNA was synthesized from 5 µg total RNA using oligodT primers, superscript II reverse transcriptase (Gibco) using standard conditions. Quantitative PCR was performed using the Lightcycler system (Roche Diagnostics, Mannheim, Germany) with SYBR-green staining of DNA doublestrands. The performance of the Lightcycler PCR, the cycling conditions and primer pairs used for beta III-tubulin, NSE, PLP, and GFAP are described before. Relative regulation levels derived from normalization to cyclophilin, and comparison to the untreated cells. In Fig 35A the differentiation potential of neural stem cells and the specific marker expressions of differentiated cells are illustrated schematically. Treatment of neural stem cells with 10ng/ml GMCSF results in a significantly induced expression of beta III-tubulin (n=3; p<0.05, two-tailed t-test) and to a lesser extend of NSE, a marker for mature neurons (Fig 35B). There was no change observed in the expression level of PLP and GFAP. This example underlines the applicability of GMCSF to modulation of neurogenesis. This is useful for both in vitro generation of differentiating neurons, as well as influencing endogenous stem cells, and applicable to a wide range of human diseases, particularly neurodegenerative diseases.

### Example 31: GCSF passes the Blood-Brain-Barrier (BBB)

The most data available in the literature contain information about serum levels of G-CSF and related cytokines. One important parameter for the effective use of G-CSF in the therapy of neurological disorders however, is the passage of the protein through the blood-brain-barrier (BBB). This question is of special interest because it is known that many proteinacious drugs cannot cross this natural border between blood and neurons. On the other hand, it was shown for several proteins, including hematopetic factors like Erythropoeitin and also granulocyte-macrophage colony stimulating factor (GM-CSF) that they can pass the BBB and get effective on neurons in the CNS ( McLay, et al. (1997), Brain, 120,2083-91., Brines, et al. (2000), Proc Natl Acad Sci U S A, 97, 10526-31).

To prove that G-CSF can pass the blood-brain barrier, we tested the appearance of injected G-CSF in rat brain. Therefore we applied an extremely sensitive iodinination assay. G-CSF and BSA as a control were radiolabelled with ¹³¹I (Amersham Biosciences, Freiburg Germany) by the Iodogen (Sigma, Taufkirchen, Germany) method and purified on Sephadex G-25 (Amersham Biosciences, Freiburg Germany) columns. The purified proteins coeluted with the unlabeled standard compounds as a single peak of correct molecular weight when analyzed by size exclusion chromatography. The radiolabeled proteins were injected via the tail vein of female Sprague-Dawley rats (250-300 g). For competitive uptake studies the cold proteins were mixed with the labelled compound and coinjected. Shortly before dissection rats were anaesthetized with Rompun/Ketanest and perfused with 100 ml of saline via the inferior abdominal aorta (at 1, 4 and 24 hours after injection) to remove the blood. The whole brain and blood were dissected, blotted dry and weighed. Blood was centrifuged to obtain the serum. The radioactivity was measured with a γ-counter (LB 951G, Berthold, Germany) along with a sample of the injectate to calculate %ID/g of the tissues. The results showed that G-CSF was present in the brain and compared to albumin the passage of the blood brain barrier was increased by a factor of 4-5 over the time. Samples, that were taken 24 h after injection of radiolabeld G-CSF show an increased level compared to 1 and 4 h (Fig. 36). These observations shows that G-CSF injected intravenously in the blood crosses the BBB and can principally activate specific receptors on CNS-neurons.

The amount of radiolabeled G-CSF in serum and brain was measured and the ratio of brain/serum was plotted against the time. As a control bovine serum albumin was used. To avoid blood contamination of brain tissue the rats were perfused with 100 ml of saline prior to dissection.

### Example 32: GM-CSF passes the blood-brain-barrier (BBB)

To show that GM-CSF can cross the blood-brain-barrier we injected rats with iodinated GM-CSF as described in Example 31 for G-CSF. The experiment gave similar results, a comparison of GM-CSF and Albumin presence in rat brain after intravenous administration show that GM-CSF levels (ratio brain vs. serum) are 3-4 fold higher than albumin. The data demonstrate that GM-CSF can cross the blood-brain-barrier (Figure 37).

Figure 37 The amount of radiolabeled GM-CSF in serum and brain was measured and the ratio of brain/serum was plotted against the time. As a control bovine serum albumin was used. To avoid blood contamination of brain tissue the rats were perfused with 100 ml of saline prior to dissection.

### Example 33: G-CSF for the treatment of Amyotrophic Lateral Sclerosis (ALS)

The idea that G-CSF and GM-CSF is beneficial for the treatment of ALS originated from two directions: First, both receptor and ligand for both GCSF and GMCSF are expressed in the large motoneurons in the anterior horn of the spinal cord. Second, the prove that G-CSF partly acts by counteracting apoptotic cascades is very attractive in light of the evidence for apoptotic mechanisms operative in ALS (Li, et al. (2000), Science, 288, 335-9). The most commonly used mouse model for ALS is transgenic mice carrying mutations in the SOD1 gene that have been shown to be responsible for familial human ALS. The most frequently used of those is the SOD1 (G93A) transgenic line. Typically, these mice have a reduced life span, and show progressing signs of motor weakness, that can be easily accessed by behavioural tests (e.g. grip strength test, rotarod tests). A number of tests for therapeutic efficacy have been conducted in these mice, and shown life-prolonging activity for substances such as Riluzole, Minocycline, Carnitine, and others. These mice are thought to have clinically predictive relevance, as Riluzole, the only approved drug in human, has protective effects in these mice, whereas BDNF, which failed in a human trial, has not. Therefore we have tested if G-CSF prolongs life expectancy, or functional outcome in the SOD1-transgenic mouse model of ALS. SOD1 and wildtype mice were injected subcutaniously with 10µg/KG BW G-CSF or vehicle starting from postnatal day 60 and monitored over the time. The results indicated a clear trend for longer life expectancy in the G-CSF treated group (Figure 38 A), as well as improvement of motor function (grip strength test (Figure 38 B)), that reached significance at several measurement points over time. Moreover, there was a trend for increased weight in the treated group.

Figure 38 A: SOD1-tg mice were injected with 10 µg/KG BW starting from postnatal day 60. There is a clear trend for prolonged life expectancy in the G-CSF treated SOD1-tg mice (closed line vs. dashed line). At several points this difference reached significance.

Figure 38 B: SOD1-tg mice were injected with 10 µg/KG BW starting from postnatal day 60. The grip strength assay shows a improvement for motor strength in the G-CSF treated SOD1-tg mice (open squares vs. open triangles). At several points this difference reached significance.Example 34: Efficacy of GCSF in rodent models of Parkinson's disease (PD)

### MPTP model:

The best-characterized model of Parkinson's Disease (PD) has been developed by using the neurotoxin 1-methyl-4phenyl-1,2,3,6-tetrahydropyridine (MPTP). To study the efficacy of GCSF in the Parkinson model, we administrated MPTP in eight-week-old male mice. Each group of mice (n=15) was given a repeated i.p. injection ofMPTP-HCl or saline (once daily for 5 consecutice days at a concentration of 30mg/kg, 5ml/kg) and a repeated s.c. (once daily, for 22 consecutive days) administration of buffer, GCSF(0.03mg/kg/; 5ml/kg) or minocycline (45mg/kg;5ml/kg). While the first application of GCSF was performed immediately after MPTP (or saline for group 0), minocycline was administrated 30 minutes thereafter, because of possible interactions of both compounds. All animals of each group were sacrified at day 22. Until that time, mice were analyzed both for locomotor activity (accelerating RotaRod) and body weight was determined daily. Futhermore each brain is subjected to a HPLC analysis with electrochemical detection for measuring the concentration of dopamine, 3,4-Dihydroxyphenylacetic acid (DOPAC) and the Homovanilic acid (HVA) in the striatum and nucleus accumbens.

However, the most important and direct parameter for the action of a neuroprotective drug in that model is the number of surviving neurons after that toxin. Therefore, tyrosine hydroxylase (TH) immunhistochemistry and stereological quantitation of TH-positive neurons were performed with midbrain sections of each animal (Triarhou, et al. (1988), J Neurocytol, 17, 221-32).

Results indicated a trend for a gain of body weight for the GCSF treated group (data not shown). Moreover an improvement of locomotor activity is observed after the testing in accelerating Rotarod (data not shown). A recent study with MPTP induced Parkinson in mice shown that minocycline prevents nigrostriatal dopaminergic neurodegeneration in these animals (Du, et al. (2001), Proc Natl Acad Sci U S A, 98, 14669-74). In our study we selected minocycline as neuroprotective compound reference for validating our data. Subacute treatment with MPTP over 5 consecutive days significantly decreased tyrosine hydrolase (TH)-positive neurons within the substantia nigra pars compacta (Fig 39 A). A treatment with GCSF and minocycline shown nearly the same efficient counteracting this reduction of nigral level of TH-positive neurons (Figure 39 B). Furthermore GCSF and minocycline shown nearly the same therapeutic efficacy when striatal levels of dopamine (Figure 39 B), and its metabolites were taken in consideration. Example 35: GMCSF efficacy in a model for Parkinson's disease

### MPTP Mode

As described for GCSF the efficacy of GMCSF in the Parkinson model is ascertained in the following study (see previous example).

Each group of mice (n=15) was given a repeated i.p. injection ofMPTP-HCl or saline (once daily for 5 consecutice days at a concentration of 30mg/kg, 5ml/kg) and a repeated s.c. (once daily, for 22 consecutive days) administration of buffer, GMCSF(0.03mg/kg/; 5ml/kg) or minocycline (45mg/kg;5ml/kg). While the first application of GCSF was performed immediately after MPTP (or saline for group 0), minocycline was administrated 30 minutes thereafter, because of possible interaction of both compounds. All animals of each group are sacrified at day 22.

An improvement of the gain of body weight and an amelioration of the locomotor activity with the accelerating rotarod have been observed after application of GMCSF, similar to the results scored by GCSF (data not shown). The therapeutic efficacy of GMCSF is therefore better compared to GCSF when the nigral levels of TH-positive neurons is taking in consideration (Figure 39 B). The reduction of striatal dopamine levels and its metabolites can be counteract after administration of GMCSF comparable to GCSF (data not shown).

### Example 36: Efficacy of GCSF and GMCSF in the 6-Hydroxydopamine (6OHDA) model for Parkinson's disease in rats (not part of the invention)

As exemplified above, GCSF as well as GMCSF had strong neuroprotective properties in the MPTP model in mice. This is a very strong proof for the applicability to human Parkinson's disease, as MPTP is a toxin that was discovered due to its ability to cause PD in humans.

One additional model for studying efficacy of the hematopoeitic factors for Parkinson's disease is the 6-OHDA model. This model is based upon the injection of 6OHDA directly into the substantia nigra or into the striatum. The drug selectively accumulates in dopaminergic neurons and leads to the apoptosis of these cells. In rats, 6OHDA is an effective neurotoxin that has been predominantly used to produce unilateral lesions. The extent of dopamine depletion can be assessed by examining rotational behaviour in response to amphetamine or apomorphine (Ungerstedt 1971). The easily and good quantifiable motor deficit constitutes a major advantage of this model. Additionally to the behaviour parameter the striatal level of tyrosine hydroxylase (TH) positive neurons after immunhistochemistry and the level of dopamine and its metabolites after a HPLC analysis can also been determined. The 6OHDA can be used to ascertain the efficacy of GCSF and GMCSF in a PD rat model. Adult Sprague-Dawley rats (body weight 250g) are unilateral lesioned after one stereotaxic injection of 8 µg in 2µl 6OHDA in the substantia nigra or in the striatum. Different doses of GCSF (0.03mg/kg; 0.1mg/kg, or others) can be administrated subcutaneously daily immediately after the lesioning for 2 weeks. Other groups of treated animals receive a single dose of intrastriatal or intranigral GCSF, or GMCSF (300µg/kg) immediately after the injection of 6OHDA. As for the MPTP model study minocycline can be used as a neuroprotective reference compound (45mg/kg once daily s.c.). Sham animals and lesioned animals treated with buffer are used as control groups. Two weeks after lesioning animals are subjected to rotational behaviour testing. Rats are injected s.c. with apomorphine, placed in a bowl cage and the number of contralateral rotations over a 1h period are recorded. Numbers of rotations for each animal group are compared using standard statistical tests. After the behavioural testing, animals are killed and the brains are processed to for immunochemistry to assay the total number of TH-positive neurons and for HPLC for determining the level of dopamine.

### Example 37: GMCSF acts anti-apoptotically in neurons by activating stat3 pathways

As already exemplified above (Example 22), GCSF had potent activities by inhibiting apoptosis in primary neurons. We suspected that GMCSF might also have a strong anti-apoptotic mechanism-of-action. Therefore, the same type of experiment as exemplified in example 22 was conducted for GMCSF. Here, the concentration applied to the neurons was 50 ng GMCSF (Leukine, Immunex) /ml medium.

Figure 40, part I shows that GMCSF inhibits PARP cleavage, that is specifically occurring as a sign of apoptosis. Cell death was induced in primary neurons by using the NO-donor NOR-3.

Figure 40, part II shows that GMCSF does not lead to increased phosphorylation of STAT1 ("pSTAT1") or STAT5 ("pSTAT5"), although the proteins themselves are expressed in neurons.

Figure 40, part III A shows that GMCSF leads to a time-dependent activation of STAT3, that is maximal at 5 min following the GMCSF stimulus. At 60 min, the level of pSTAT3 drops below the initial level, a response kinetic known from cells of the hematopoeitic system. B, quantification of three independent experiments. C. Activation of STAT3 by phosphorylation can be inhibited by the JAK2 inhibitor AG490. 5 min after giving GMCSF the levels of pSTAT3 are quite different in presenc eof the inhibitor. Figure 40, part IV shows that GMCSF strongly induces the expression of the stat3 target genes Bcl2, and BclX1. These genes are known as being antiapoptotic.

This experiment demonstrates that 1.) GMCSF acts antiapoptotically on neurons 2.) this is mediated by the stat3 pathway. Therefore this also demonstrates the possibility to use the stat3 system in neurons as a screening tool for finding novel neuroprotective durgs, including novel mimetics of gcsf or gmcsf.

### Example 38: GMCSF reduces infarct volume in rodent stroke models - (not part of the invention)

We conducted experiments with GMCSF in the most accepted model for stroke in the rat, the middle cerebral artery occlusion model (MCAO).

In principle, the experiment was conducted as exemplified in example 1 and 19. In brief, male Wistar rats were anesthesized using inhalation anesthesia (halothane/N₂O/O₂). The carotid artery was exposed, and a coated nylon filament inserted into the common carotid artery, and advanced until it blocked blood flow to the MCA. Correct positioning of the filament was monitored by Laser Doppler flowmetry. After 90 min occlusion, the filament was withdrawn allowing reperfusion. GMCSF (Leukine, Immunex) was applied at a dose of 250 µg/ kg body weight to the rats via an intravenous catheter (femoral vein) over a period of app. 20 min by an infusion pump at either 30 min or 3 h following onset of ischemia. Infarct volumes were determined 24 h later with the standard method of TTC-staining, and a computer-based volumetry.

Figure 41 demonstrates that there is a significant reduction of infarct volume in the GMCSF-treated animals, both at the early (Fig 41, part I), and at the late time window of 3 h (Fig 41, part II).

This exemplifies the applicability to GMCSF to neurodegenerative disorders in general, and to stroke treatment in particular.

Obviously, numerous modifications and variations of the present invention are possible in light of the above teachings. It is therefore to be understood that within the scope of the appended claims, the invention may be practiced otherwise than as specifically described herein.

## Claims

1. G-CSF or a functional active variant thereof having at least 90 % sequence identity to human G-CSF as shown in SEQ ID NO:28 for use in a method for the treatment of amyotrophic lateral sclerosis.

2. The G-CSF of claim 1, wherein the G-CSF is human G-CSF.

## Patentansprüche

1. G-CSF oder eine funktionell aktive Variante davon mit mindestens 90 % Sequenzidentität zu humanem G-CSF, wie in SEQ ID NO: 28 gezeigt, zur Verwendung in einem Verfahren für die Behandlung von Amyotropher Lateralsklerose.

2. G-CSF nach Anspruch 1, wobei das G-CSF humanes G-CSF ist.

## Revendications

1. G-CSF ou un variant fonctionnellement actif en dérivant présentant au moins 90 % d'identité de séquence avec le G-CSF humain tel que représenté dans SEQ ID No : 28, destiné à être utilisé dans un procédé de traitement de la sclérose latérale amyotrophique.

2. Le G-CSF selon la revendication 1, dans lequel le G-CSF est le G-CSF humain.


### SEQUENCE LISTING

<110> SCHNEIDER, ARMIN
   SCHAEBITZ, WOLF-RUEDIGER
   KOLLMAR, RAINER
   SCHWAB, STEFAN
<120> METHODS OF TREATING NEUROLOGICAL CONDITIONS WITH HEMATOPOEITIC
   GROWTH FACTORS
<130> 229530US
<160> 65
<170> PatentIn version 3.2
<210> 1
   <211> 70
   <212> PRT
   <213> Artificial Sequence
<220>
   <223> computer generated consensus sequence
<220>
   <221> misc_feature
   <222> (2)..(5)
   <223> Xaa is any amino acid
<220>
   <221> misc_feature
   <222> (6)..(41)
   <223> Xaa is any amino acid
<220>
   <221> misc_feature
   <222> (33)..(41)
   <223>..may be present or absent
<220>
   <221> misc_feature
   <222> (46)..(46)
   <223> Xaa may be any amino acid
<220>
   <221> misc_feature
   <222> (47)..(49)
   <223> Xaa is any amino acid
<220>
   <221> misc_feature
   <222> (47)..(49)
   <223> amino acids may be present or absent
<220>
   <221> misc_feature
<222> (50)..(60)
   <223> Xaa is any amino acid
<220>
   <221> misc_feature
   <222> (57)..(60)
   <223> may be present or absent
<220>
   <221> misc feature
   <222> (63)..(64)
   <223> Xaa is any amino acid
<220>
   <221> misc_feature
   <222> (64)..(64)
   <223> may be present or absent
<220>
   <221> misc_teature
   <222> (69)..(69)
   <223> Xaa is any amino acid
<400> 1
   Asn Xaa Xaa Xaa Xaa Ser Xaa Xaa Xaa Xaa Xaa Xaa Xaa Xaa Xaa Xaa
   1               5                   10                  15
   Xaa Xaa Xaa Xaa Xaa Xaa Xaa Xaa Xaa Xaa Xaa Xaa Xaa Xaa Xaa Xaa
               20                  25                  30
   Xaa Xaa Xaa Xaa Xaa Xaa Xaa Xaa Xaa Leu Val Ile Met Xaa Trp Xaa
           35                  40                  45
   Xaa Xaa Pro Xaa Xaa Xaa Xaa Xaa Xaa Xaa Xaa Xaa Tyr Phe Xaa Xaa
       50                  55                  60
   Val Ile Leu Met Xaa Trp
   65                  70
<210> 2
   <211> 21
   <212> DNA
   <213> Artificial Sequence
<220>
   <223> synthetic DNA
<400> 2
   gcgggcaaat caggatotca c 21
<210> 3
   <211> 21
   <212> DNA
   <213> Artificial Sequence
<220>
   <223> synthetic DNA
<400> 3
   cgaagctcag cttgatccag g 21
<210> 4
   <211> 280
   <212> DNA
   <213> Rattus rattus
<400> 4
   gcgggcaaat caggatctca ccccccattg tccatcttgg ggatcctgtc ctggcctcct 60
   gcaccatcag cccaaactgc agcaaactgg accgacagcc aaagatccta tggagactgc 120
   aagatgaacc aaaccagcct ggggacagac agcatcacct gcctgacggg tcccaggagt 180
   ccatcatcac tctgcctcat ctgaactaca ctcaggcctt cctcttctgc ttggtgccat 240
   ggaacaacag cttccaggtc ctggatcaag ctgagcttcg 280
<210> 5
   <211> 22
   <212> DNA
   <213> Artificial Sequence
<220>
   <223> synthetic DNA
<400> 5
   cccctcaaac ctatcctgcc tc 22
<210> 6
   <211> 22
   <212> DNA
   <213> Artificial Sequence
<220>
   <223> synthetic DNA
<400> 6
   tccaggcaga gatcagcgaa tg 22
<210> 7
   <211> 21
   <212> DNA
   <213> Artificial Sequence
<220>
   <223> synthetic DNA
<400> 7
   ccattgtcca tcttggggat c 21
<210> 8
   <211> 21
   <212> DNA
   <213> Artificial, Sequence
<220>
   <223> synthetic DNA
<400> 8
   cctggaagct gttgttccat g 21
<210> 9
   <211> 20
   <212> DNA
   <213> Artificial Sequence
<220>
   <223> synthetic DNA
<400> 9
   accccaccgt gttcttcgac 20
<210> 10
   <211> 20
   <212> DNA
   <213> Artificial Sequence
<220>
   <223> synthetic DNA
<400> 10
   catttgccat ggacaagatg 20
<210> 11
   <211> 7
   <212> PRT
   <213> Artificial Sequence
<220>
   <223> synthetic peptide sequence
<400> 11
   Leu Gly His Ser Leu Gly Ile
   1 5
<210> 12
   <211> 39
   <212> DNA
   <213> Artificial Sequence
<220>
   <223> synthetic DNA
<400> 12
   cgggatccgg gaccgcgtat ctgatgacga gcgtgtcaa 39
<210> 13
   <211> 25
   <212> DNA
   <213> Artificial Sequence
<220>
   <223> synthetic DNA
<400> 13
   ctcggagacg ctgaggaagg acctg 25
<210> 14
   <211> 40
   <212> DNA
   <213> Artificial Sequence
<220>
   <223> synthetic DNA
<400> 14
   ctgcggccct agaccacgcc caccgctccc cgtgacgtcg 40
<210> 15
   <211> 22
   <212> DNA
   <213> Artificial Sequence
<220>
   <223> synthetic DNA
<400> 15
   acgtcgttgg ctcagttatg tc 22
<210> 16
   <211> 24
   <212> DNA
   <213> Artificial Sequence
<220>
   <223> synthetic DNA
<400> 16
   atttatgtca gagatggagg atgg 24
<210> 17
   <211> 200
   <212> DNA
   <213> Artificial Sequence
<220>
   <223> synthetic DNA
<400> 17
   accccaccgt gttcttcgac 20
<210> 18
   <211> 20
   <212> DNA
   <213> Artificial Sequence
<220>
   <223> synthetic DNA
<400> 18
   catttgccat ggacaagatg 20
<210> 19
   <211> 22
   <212> DNA
   <213> Artificial Sequence
<220>
   <223> synthetic DNA
<400> 19
   acgtcgttgg ctcagttatg tc 22
<210> 20
   <211> 24
   <212> DNA
   <213> Artificial Sequence
<220>
   <223> synthetic DNA
<400> 20
   atttatgtca gagatggagg atgg 24
<210> 21
   <211> 177
   <212> DNA
   <213> Rattus rattus
<400> 21
   acgtcgttgg ctcagttatg tcagacagga aatctcacca tcccacaatg attgacagct 60
   ctcacaggga atcccgcctc cgctgggacc aattgacatc acggacagga atacccgccc 120
   ctgtggccct gatgggcagg tcctgcctgg ctcccatcct ccatctctga cataaat 177
<210> 22
   <211> 400
   <212> PRT
   <213> Homo sapiens
<400> 22
   Met Leu Leu Leu Val Thr Ser Leu Leu Leu Cys Glu Leu Pro His Pro
   1               5                   10                  15
   Ala Phe Leu Leu Ile Pro Glu Lys Ser Asp Leu Arg Thr Val Ala Pro
               20                  25                  30
   Ala Ser Ser Leu Asn Val Arg Phe Asp Ser Arg Thr Met Asn Leu Ser
           35                  40                  45
   Trp Asp Cys Gln Glu Asn Thr Thr Phe Ser Lys Cys Phe Leu Thr Asp
       50                  55                  60
   Lys Lys Asn Arg Val Val Glu Pro Arg Leu Ser Asn Asn Glu Cys Ser
   65                  70                  75                  80
   Cys Thr Phe Arg Glu Ile Cys Leu His Glu Gly Val Thr Phe Glu Val
                   85                  90                  95
   His Val Asn Thr Ser Gln Arg Gly Phe Gln Gln Lys Leu Leu Tyr Pro
               100                 105                 110
   Asn Ser Gly Arg Glu Gly Thr Ala Ala Gln Asn Phe Ser Cys Phe Ile
           115                 120                 125
   Tyr Asn Ala Asp Leu Met Asn Cys Thr Trp Ala Arg Gly Pro Thr Ala
       130                 135                 140
   Pro Arg Asp Val Gln Tyr Phe Leu Tyr Ile Arg Asn Ser Lys Arg Arg
   145                 150                 155                 160
   Arg Glu Ile Arg Cys Pro Tyr Tyr Ile Gln Asp Ser Gly Thr His Val
                   165                 170                 175
   Gly Cys His Leu Asp Asn Leu Ser Gly Leu Thr Ser Arg Asn Tyr Phe
               180                 185                 190
   Leu Val Asn Gly Thr Ser Arg Glu Ile Gly Ile Gln Phe Phe Asp Ser
           195                 200                 205
   Leu Leu Asp Thr Lys Lys Ile Glu Arg Phe Asn Pro Pro Ser Asn Val
       210                 215                 220
   Thr Val Arg Cys Asn Thr Thr His Cys Leu Val Arg Trp Lys Gln Pro
   225                 230                 235                 240
   Arg Thr Tyr Gln Lys Leu Ser Tyr Leu Asp Phe Gln Tyr Gln Leu Asp
                   245                 250                 255
   Val His Arg Lys Asn Thr Gln Pro Gly Thr Glu Asn Leu Leu Ile Asn
               260                 265                 270
   Val Ser Gly Asp Leu Glu Asn Arg Tyr Asn Phe Pro Ser Ser Glu Pro
           275                 280                 285
   Arg Ala Lys His Ser Val Lys Ile Arg Ala Ala Asp Val Arg Ile Leu
       290                 295                 300
   Asn Trp Ser Ser Trp Ser Glu Ala Ile Glu Phe Gly Ser Asp Asp Gly
   305                 310                 315                 320
   Asn Leu Gly Ser Val Tyr Ile Tyr Val Leu Leu Ile Val Gly Thr Leu
                   325                 330                 335
   Val Cys Gly Ile Val Leu Gly Phe Leu Phe Lys Arg Phe Leu Arg Ile
               340                 345                 350
   Gln Arg Leu Phe Pro Pro Val Pro Gln Ile Lys Asp Lys Leu Asn Asp
           355                 360                 365
   Asn His Glu Val Glu Asp Glu Ile Ile Trp Glu Glu Phe Thr Pro Glu
       370                 375                 380
   Glu Gly Lys Gly Tyr Arg Glu Glu Val Leu Ile Val Lys Glu Ile Thr
   385                 390                 395                 400
<210> 23
   <211> 388
   <212> PRT
   <213> Mus musculus
<400> 23
   Met Thr Ser Ser His Ala Met Asn Ile Thr Pro Leu Ala Gln Leu Ala
   1               5                   10                  15
   Leu Leu Phe Ser Thr Leu Leu Leu Pro Gly Thr Gln Ala Leu Leu Ala
               20                  25                  30
   Pro Thr Thr Pro Asp Ala Gly Ser Ala Leu Asn Leu Thr Phe Asp Pro
           35                  40                  45
   Trp Thr Arg Thr Leu Thr Trp Ala Cys Asp Thr Ala Ala Gly Asn Val
       50                  55                  60
   Thr Val Thr Ser Cys Thr Val Thr Ser Arg Glu Ala Gly Ile His Arg
   65                  70                  75                  80
   Arg Val Ser Pro Phe Gly Cys Arg Cys Trp Phe Arg Arg Met Met Ala
                   85                  90                  95
   Leu His His Gly Val Thr Leu Asp Val Asn Gly Thr Val Gly Gly Ala
               100                 105                 110
   Ala Ala His Trp Arg Leu Ser Phe Val Asn Glu Ser Ala Ala Gly Ser
           115                 120                 125
   Gly Ala Glu Asn Leu Thr Cys Glu Ile Arg Ala Ala Arg Phe Leu Ser
       130                 135                 140
   Cys Ala Trp Arg Glu Gly Pro Ala Ala Pro Ala Asp Val Arg Tyr Ser
   145                 150                 155                 160
   Leu Arg Val Leu Asn Ser Thr Gly His Asp Val Ala Arg Cys Met Ala
                   165                 170                 175
   Asp Pro Gly Asp Asp Val Ile Thr Gln Cys Ile Ala Asn Asp Leu Ser
               180                 185                 190
   Leu Leu Gly Ser Glu Ala Tyr Leu Val Val Thr Gly Arg Ser Gly Ala
   195 200 205
   Gly Pro Val Arg Phe Leu Asp Asp Val Val Ala Thr Lys Ala Leu Glu
   210 215 220
   Arg Leu Gly Pro Pro Arg Asp Val Thr Ala Ser Cys Asn Ser Ser His
   225 230 235 240
   Cys Thr Val Ser Trp Ala Pro Pro Ser Thr Trp Ala Ser Leu Thr Ala
   245 250 255
   Arg Asp Phe Gln Phe Glu Val Gln Trp Gln Ser Ala Glu Pro Gly Ser
   260 265 270
   Thr Pro Arg Lys Val Leu Val Val Glu Glu Thr Arg Leu Ala Phe Pro
   275 280 285
   Ser Pro Ala Pro His Gly Gly His Lys Val Lys Val Arg Ala Gly Asp
   290 295 300
   Thr Arg Met Lys His Trp Gly Glu Trp Ser Pro Ala His Pro Leu Glu
   305 310 315 320
   Ala Glu Asp Thr Arg Val Pro Gly Ala Leu Leu Tyr Ala Val Thr Ala
   325 330 335
   Cys Ala Val Leu Leu Cys Ala Leu Ala Leu Gly Val Thr Cys Arg Arg
   340 345 350
   Phe Glu Val Thr Arg Arg Leu Phe Pro Pro Ile Pro Gly Ile Arg Asp
   355 360 365
   Lys Val Ser Asp Asp Val Arg Val Asn Pro Glu Thr Leu Arg Lys Asp
   370 375 380
   Leu Leu Gln Pro
   385
<210> 24
   <211> 9
   <212> PRT
   <213> Rattus rattus
<400> 24
   Ile Asn Ser Glu Arg Thr Ser Glu Gln
   1 5
<210> 25
   <211> 127
   <212> PRT
   <213> Rattus rattus
<400> 25
   Ala Pro Thr Arg Ser Pro Asn Pro Val Thr Arg Pro Trp Lys His Val
   1               5                   10                  15
   Asp Ala Ile Lys Glu Ala Leu Ser Leu Leu Asn Asp Met Arg Ala Leu
               20                  25                  30
   Glu Asn Glu Lys Asn Glu Asp Val Asp Ile Ile Ser Asn Glu Phe Ser
           35                  40                  45
   Ile Gln Arg Pro Thr Cys Val Gln Thr Arg Leu Lys Leu Tyr Lys Gln
       50                  55                  60
   Gly Leu Arg Gly Asn Leu Thr Lys Leu Asn Gly Ala Leu Thr Met Ile
   65                  70                  75                  80
   Ala Ser His Tyr Gln Thr Asn Cys Pro Pro Thr Pro Glu Thr Asp Cys
                   85                  90                  95
   Glu Ile Asp Val Thr Thr Phe Glu Asp Phe Ile Lys Asn Leu Lys Gly
               100                 105                 110
   Phe Leu Phe Asp Ile Pro Phe Asp Cys Trp Lys Pro Val Gln Lys
           115                 120                 125
<210> 26
   <211> 141
   <212> PRT
   <213> Mus musculus
<400> 26
   Met Trp Leu Gln Asn Leu Arg Leu Lys Ile Phe Glu Gln Gly Leu Arg
   1               5                   10                  15
   Gly Asn Phe Thr Lys Leu Lys Gly Ala Leu Asn Met Thr Ala Ser Tyr
               20                  25                  30
   Tyr Gln Thr Tyr Cys Pro Pro Thr Pro Glu Thr Asp Cys Glu Thr Gln
   35 40 45
   Val Thr Thr Tyr Ala Asp Phe Ile Asp Ser Leu Lys Thr Leu Phe Leu
   50 55 60
   Gly Ile Val Val Tyr Ser Leu Ser Ala Pro Thr Arg Ser Pro Ile Phe
   65 70 75 80
   Leu Thr Asp Ile Pro Phe Glu Cys Lys Lys Pro Gly Gln Lys Thr Val
   85 90 95
   Thr Arg Pro Trp Lys His Val Glu Ala Ile Lys Glu Ala Leu Asn Leu
   100 105 110
   Leu Asp Asp Met Pro Val Thr Leu Asn Glu Glu Val Glu Val Val Ser
   115 120 125
   Asn Glu Phe Ser Phe Lys Lys Leu Thr Cys Val Gln Thr
   130 135 140
<210> 27
   <211> 144
   <212> PRT
   <213> Homo sapiens
<400> 27
   Met Trp Leu Gln Ser Leu Leu Leu Leu Gly Thr Val Ala Cys Ser Ile
   1 5 10 15
   Ser Ala Pro Ala Arg Ser Pro Ser Pro Ser Thr Gln Pro Trp Glu His
   20 25 30
   Val Asn Ala Ile Gln Glu Ala Arg Arg Leu Leu Asn Leu Ser Arg Asp
   35 40 45
   Thr Ala Ala Glu Met Asn Glu Thr Val Glu Val Ile Ser Glu Met Phe
   50 55 60
   Asp Leu Gln Glu Pro Thr Cys Leu Gln Thr Arg Leu Glu Leu Tyr Lys
   65 70 75 80
   Gln Gly Leu Arg Gly Ser Leu Thr Lys Leu Lys Gly Pro Leu Thr Met
   85 90 95
   Met Ala Ser His Tyr Lys Gln His Cys Pro Pro Thr Pro Glu Thr Ser
   100 105 110
   Cys Ala Thr Gln Ile Ile Thr Phe Glu Ser Phe Lys Glu Asn Leu Lys
   115 120 125
   Asp Phe Leu Leu Val Ile Pro Phe Asp Cys Trp Glu Pro Val Gln Glu
   130 135 140
<210> 28
   <211> 207
   <212> PRT
   <213> Homo sapiens
<400> 28
   Met Ala Gly Pro Ala Thr Gln Ser Pro Met Lys Leu Met Ala Leu Gln
   1 5 10 15
   Leu Leu Leu Trp His Ser Ala Leu Trp Thr Val Gln Glu Ala Thr Pro
   20 25 30
   Leu Gly Pro Ala Ser Ser Leu Pro Gln Ser Phe Leu Leu Lys Cys Leu
   35 40 45
   Glu Gln Val Arg Lys Ile Gln Gly Asp Gly Ala Ala Leu Gln Glu Lys
   50 55 60
   Leu Val Ser Glu Cys Ala Thr Tyr Lys Leu Cys His Pro Glu Glu Leu
   65 70 75 80
   Val Leu Leu Gly His Ser Leu Gly Ile Pro Trp Ala Pro Leu Ser Ser
   85 90 95
   Cys Pro Ser Gln Ala Leu Gln Leu Ala Gly Cys Leu Ser Gln Leu His
   100 105 110
   Ser Gly Leu Phe Leu Tyr Gln Gly Leu Leu Gln Ala Leu Glu Gly Ile
   115 120 125
   Ser Pro Glu Leu Gly Pro Thr Leu Asp Thr Leu Gln Leu Asp Val Ala
   130 135 140
   Asp Phe Ala Thr Thr Ile Trp Gln Gln Met Glu Glu Leu Gly Met Ala
   145 150 155 160
   Pro Ala Leu Gln Pro Thr Gln Gly Ala Met Pro Ala Phe Ala Ser Ala
   165 170 175
   Phe Gln Arg Arg Ala Gly Gly Val Leu Val Ala Ser His Leu Gln Ser
   180 185 190
   Phe Leu Glu Val Ser Tyr Arg Val Leu Arg His Leu Ala Gln Pro
   195 200 205
<210> 29
   <211> 208
   <212> PRT
   <213> Mus musculu
<400> 29
   Met Ala Gln Leu Ser Ala Gln Arg Arg Met Lys Leu Met Ala Leu Gln
   1 5 10 15
   Leu Leu Leu Trp Gln Ser Ala Leu Trp Ser Gly Arg Glu Ala Val Pro
   20 25 30
   Leu Val Thr Val Ser Ala Leu Pro Pro Ser Leu Pro Leu Pro Arg Ser
   35 40 45
   Phe Leu Leu Lys Ser Leu Glu Gln Val Arg Lys Ile Gln Ala Ser Gly
   50 55 60
   Ser Val Leu Leu Glu Gln Leu Cys Ala Thr Tyr Lys Leu Cys His Pro
   65 70 75 80
   Glu Glu Leu Val Leu Leu Gly His Ser Leu Gly Ile Pro Lys Ala Ser
   85 90 95
   Leu Ser Gly Cys Ser Ser Gln Ala Leu Gln Gln Thr Gln Cys Leu Ser
   100 105 110
   Gln Leu His Ser Gly Leu Cys Leu Tyr Gln Gly Leu Leu Gln Ala Leu
   115 120 125
   Ser Gly Ile Ser Pro Ala Leu Ala Pro Thr Leu Asp Leu Leu Gln Leu
   130 135 140
   Asp Val Ala Asn Phe Ala Thr Thr Ile Trp Gln Gln Met Glu Asn Leu
   145 150 155 160
   Gly Val Ala Pro Thr Val Gln Pro Thr Gln Ser Ala Met Pro Ala Phe
   165 170 175
   Thr Ser Ala Phe Gln Arg Arg Ala Gly Gly Val Leu Ala Ile Ser Tyr
   180 185 190
   Leu Gln Gly Phe Leu Glu Thr Ala Arg Leu Ala Leu His His Leu Ala
   195 200 205
<210> 30
   <211> 214
   <212> PRT
   <213> Rattus norvegicus
<400> 30
   Met Lys Leu Met Ala Leu Gln Leu Leu Leu Trp His Ser Ala Leu Trp
   1 5 10 15
   Ser Gly Gln Glu Ala Ile Pro Leu Leu Thr Val Ser Ser Leu Pro Pro
   20 25 30
   Ser Leu Pro Leu Pro Arg Ser Phe Leu Leu Lys Ser Leu Glu Gln Val
   35 40 45
   Arg Lys Ile Gln Ala Arg Asn Thr Glu Leu Leu Glu Gln Leu Cys Ala
   50 55 60
   Thr Tyr Lys Leu Cys His Pro Glu Glu Leu Val Leu Phe Gly His Ser
   65 70 75 80
   Leu Gly Ile Pro Lys Ala Ser Leu Ser Ser Cys Ser Ser Gln Ala Leu
   85 90 95
   Gln Gln Thr Lys Cys Leu Ser Gln Leu His Ser Gly Leu Phe Leu Tyr
   100 105 110
   Gln Gly Leu Leu Gln Ala Leu Ala Gly Ile Ser Ser Glu Leu Ala Pro
   115 120 125
   Thr Leu Asp Met Leu His Leu Asp Val Asp Asn Phe Ala Thr Thr Ile
   130 135 140
   Trp Gln Gln Met Glu Ser Leu Gly Val Ala Pro Thr Val Gln Pro Thr
   145 150 155 160
   Gln Ser Thr Met Pro Ile Phe Thr Ser Ala Phe Gln Arg Arg Ala Gly
   165 170 175
   Gly Val Leu Val Thr Ser Tyr Leu Gln Ser Phe Leu Glu Thr Ala His
   180 185 190
   His Ala Leu His His Leu Pro Arg Pro Ala Gln Lys His Phe Pro Glu
   195 200 205
   Ser Leu Phe Ile Ser Ile
   210
<210> 31
   <211> 195
   <212> PRT
   <213> Felis catus
<400> 31
   Met Lys Leu Thr Ala Leu Gln Leu Leu Leu Trp His Ser Ala Leu Trp
   1 5 10 15
   Met Val Gln Glu Ala Thr Pro Leu Gly Pro Thr Ser Ser Leu Pro Gln
   20 25 30
   Ser Phe Leu Leu Lys Cys Leu Glu Gln Val Arg Lys Val Gln Ala Asp
   35 40 45
   Gly Thr Ala Leu Gln Glu Arg Leu Cys Ala Ala His Lys Leu Cys His
   50 55 60
   Pro Glu Glu Leu Val Leu Leu Gly His Ala Leu Gly Ile Pro Gln Ala
   65 70 75 80
   Pro Leu Ser Ser Cys Ser Ser Gln Ala Leu Gln Leu Thr Gly Cys Leu
   85 90 95
   Arg Gln Leu His Ser Gly Leu Phe Leu Tyr Gln Gly Leu Leu Gln Ala
   100 105 110
   Leu Ala Gly Ile Ser Pro Glu Leu Ala Pro Thr Leu Asp Met Leu Gln
   115 120 125
   Leu Asp Ile Thr Asp Phe Ala Ile Asn Ile Trp Gln Gln Met Glu Asp
   130 135 140
   Val Gly Met Ala Pro Ala Val Pro Pro Thr Gln Gly Thr Met Pro Thr
   145 150 155 160
   Phe Thr Ser Ala Phe Gln Arg Arg Ala Gly Gly Thr Leu Val Ala Ser
   165 170 175
   Asn Leu Gln Ser Phe Leu Glu Val Ala Tyr Arg Ala Leu Arg His Phe
   180 185 190
   Thr Lys Pro
   195
<210> 32.
   <211> 195
   <212> PRT
   <213> Bos taurus
<400> 32
   Met Lys Leu Met Val Leu Gln Leu Leu Leu Trp His Ser Ala Leu Trp
   1 5 10 15
   Thr Val His Glu Ala Thr Pro Leu Gly Pro Ala Arg Ser Leu Pro Gln
   20 25 30
   Ser Phe Leu Leu Lys Cys Leu Glu Gln Val Arg Lys Ile Gln Ala Asp
   35 40 45
   Gly Ala Glu Leu Gln Glu Arg Leu Cys Ala Ala His Lys Leu Cys His
   50 55 60
   Pro Glu Glu Leu Met Leu Leu Arg His Ser Leu Gly Ile Pro Gln Ala
   65 70 75 80
   Pro Leu Ser Ser Cys Ser Ser Gln Ser Leu Gln Leu Thr Ser Cys Leu
   85 90 95
   Asn Gln Leu His Gly Gly Leu Phe Leu Tyr Gln Gly Leu Leu Gln Ala
   100 105 110
   Leu Ala Gly Ile Ser Pro Glu Leu Ala Pro Thr Leu Asp Thr Leu Gln
   115 120 125
   Leu Asp Val Thr Asp Phe Ala Thr Asn Ile Trp Leu Gln Met Glu Asp
   130 135 140
   Leu Gly Ala Ala Pro Ala Val Gln Pro Thr Gln Gly Ala Met Pro Thr
   145 150 155 160
   Phe Thr Ser Ala Phe Gln Arg Arg Ala Gly Gly Val Leu Val Ala Ser
   165 170 175
   Gln Leu His Arg Phe Leu Glu Leu Ala Tyr Arg Gly Leu Arg Tyr Leu
   180 185 190
   Ala Glu Pro
   195
<210> 33
   <211> 195
   <212> PRT
   <213> Sus scrofa
<400> 33
   Met Lys Leu Met Ala Leu Gln Leu Leu Leu Trp His Ile Ala Leu Trp
   1 5 10 15
   Met Val Pro Glu Ala Ala Pro Leu Ser Pro Ala Ser Ser Leu Pro Gln
   20 25 30
   Ser Phe Leu Leu Lys Cys Leu Glu Gln Val Arg Lys Ile Gln Ala Asp
   35 40 45
   Gly Ala Glu Leu Gln Glu Arg Leu Cys Ala Thr His Lys Leu Cys His
   50 55 60
   Pro Gln Glu Leu Val Leu Leu Gly His Ser Leu Gly Leu Pro Gln Ala
   65 70 75 80
   Ser Leu Ser Ser Cys Ser Ser Gln Ala Leu Gln Leu Thr Gly Cys Leu
   85 90 95
   Asn Gln Leu His Gly Gly Leu Val Leu Tyr Gln Gly Leu Leu Gln Ala
   100 105 110
   Leu Ala Gly Ile Ser Pro Glu Leu Ala Pro Ala Leu Asp Ile Leu Gln
   115 120 125
   Leu Asp Val Thr Asp Leu Ala Thr Asn Ile Trp Leu Gln Met Glu Asp
   130 135 140
   Leu Arg Met Ala Pro Ala Ser Leu Pro Thr Gln Gly Thr Val Pro Thr
   145 150 155 160
   Phe Thr Ser Ala Phe Gln Arg Arg Ala Gly Gly Val Leu Val Val Ser
   165 170 175
   Gln Leu Gln Ser Phe Leu Glu Leu Ala Tyr Arg Val Leu Arg Tyr Leu
   180 185 190
   Ala Glu Pro
   195
<210> 34
   <211> 836
   <212> PRT
   <213> Homo sapiens
<400> 34
<210> 35
   <211> 837
   <212> PRT
   <213> Mus musculus
<400> 35
   Met Val Gly Leu Gly Ala Cys Thr Leu Thr Gly Val Thr Leu Ile Phe
   1 5 10 15
   Leu Leu Leu Pro Arg Ser Leu Glu Ser Cys Gly His Ile Glu Ile Ser
   20 25 30
   Pro Pro Val Val Arg Leu Gly Asp Pro Val Leu Ala Ser Cys Thr Ile
   35 40 45
   Ser Pro Asn Cys Ser Lys Leu Asp Gln Gln Ala Lys Ile Leu Trp Arg
   50 55 60
   Leu Gln Asp Glu Pro Ile Gln Pro Gly Asp Arg Gln His His Leu Pro
   65 70 75 80
   Asp Gly Thr Gln Glu Ser Leu Ile Thr Leu Pro His Leu Asn Tyr Thr
   85 90 95
   Gln Ala Phe Leu Phe Cys Leu Val Pro Trp Glu Asp Ser Val Gln Leu
   100 105 110
   Leu Asp Gln Ala Glu Leu His Ala Gly Tyr Pro Pro Ala Ser Pro Ser
           115                 120                 125
   Asn Leu Ser Cys Leu Met His Leu Thr Thr Asn Ser Leu Val Cys Gln
       130                 135                 140
   Trp Glu Pro Gly Pro Glu Thr His Leu Pro Thr Ser Phe Ile Leu Lys
   145                 150                 155                 160
   Ser Phe Arg Ser Arg Ala Asp Cys Gln Tyr Gln Gly Asp Thr Ile Pro
                   165                 170                 175
   Asp Cys Val Ala Lys Lys Arg Gln Asn Asn Cys Ser Ile Pro Arg Lys
               180                 185                 190
   Asn Leu Leu Leu Tyr Gln Tyr Met Ala Ile Trp Val Gln Ala Glu Asn
           195                 200                 205
   Met Leu Gly Ser Ser Glu Ser Pro Lys Leu Cys Leu Asp Pro Met Asp
       210                 215                 220
   Val Val Lys Leu Glu Pro Pro Met Leu Gln Ala Leu Asp Ile Gly Pro
   225                 230                 235                 240
   Asp Val Val Ser His Gln Pro Gly Cys Leu Trp Leu Ser Trp Lys Pro
                   245                 250                 255
   Trp Lys Pro Ser Glu Tyr Met Glu Gln Glu Cys Glu Leu Arg Tyr Gln
               260                 265                 270
   Pro Gln Leu Lys Gly Ala Asn Trp Thr Leu Val Phe His Leu Pro Ser
           275                 280                 285
   Ser Lys Asp Gln Phe Glu Leu Cys Gly Leu His Gln Ala Pro Val Tyr
       290                 295                 300
   Thr Leu Gln Met Arg Cys Ile Arg Ser Ser Leu Pro Gly Phe Trp Ser
   305                 310                 315                 320
   Pro Trp Ser Pro Gly Leu Gln Leu Arg Pro Thr Met Lys Ala Pro Thr
                   325                 330                 335
   Ile Arg Leu Asp Thr Trp Cys Gln Lys Lys Gln Leu Asp Pro Gly Thr
   340 345 350
   Val Ser Val Gln Leu Phe Trp Lys Pro Thr Pro Leu Gln Glu Asp Ser
   355 360 365
   Gly Gln Ile Gln Gly Tyr Leu Leu Ser Trp Asn Ser Pro Asp His Gln
   370 375 380
   Gly Gln Asp Ile His Leu Cys Asn Thr Thr Gln Leu Ser Cys Ile Phe
   385 390 395 400
   Leu Leu Pro Ser Glu Ala Gln Asn Val Thr Leu Val Ala Tyr Asn Lys
   405 410 415
   Ala Gly Thr Ser Ser Pro Thr Thr Val Val Phe Leu Glu Asn Glu Gly
   420 425 430
   Pro Ala Val Thr Gly Leu His Ala Met Ala Gln Asp Leu Asn Thr Ile
   435 440 445
   Trp Val Asp Trp Glu Ala Pro Ser Leu Leu Pro Gln Gly Tyr Leu Ile
   450 455 460
   Glu Trp Glu Met Ser Ser Pro Ser Tyr Asn Asn Ser Tyr Lys Ser Trp
   465 470 475 480
   Met Ile Glu Pro Asn Gly Asn Ile Thr Gly Ile Leu Leu Lys Asp Asn
   485 490 495
   Ile Asn Pro Phe Gln Leu Tyr Arg Ile Thr Val Ala Pro Leu Tyr Pro
   500 505 510
   Gly Ile Val Gly Pro Pro Val Asn Val Tyr Thr Phe Ala Gly Glu Arg
   515 520 525
   Ala Pro Pro His Ala Pro Ala Leu His Leu Lys His Val Gly Thr Thr
   530 535 540
   Trp Ala Gln Leu Glu Trp Val Pro Glu Ala Pro Arg Leu Gly Met Ile
   545 550 555 560
   Pro Leu Thr His Tyr Thr Ile Phe Trp Ala Asp Ala Gly Asp His Ser
   565 570 575
   Phe Ser Val Thr Leu Asn Ile Ser Leu His Asp Phe Val Leu Lys His
   580 585 590
   Leu Glu Pro Ala Ser Leu Tyr His Val Tyr Leu Met Ala Thr Ser Arg
   595 600 605
   Ala Gly Ser Thr Asn Ser Thr Gly Leu Thr Leu Arg Thr Leu Asp Pro
   610 615 620
   Ser Asp Leu Asn Ile Phe Leu Gly Ile Leu Cys Leu Val Leu Leu Ser
   625 630 635 640
   Thr Thr Cys Val Val Thr Trp Leu Cys Cys Lys Arg Arg Gly Lys Thr
   645 650 655
   Ser Phe Trp Ser Asp Val Pro Asp Pro Ala His Ser Ser Leu Ser Ser
   660 665 670
   Trp Leu Pro Thr Ile Met Thr Glu Glu Thr Phe Gln Leu Pro Ser Phe
   675 680 685
   Trp Asp Ser Ser Val Pro Ser Ile Thr Lys Ile Thr Glu Leu Glu Glu
   690 695 700
   Asp Lys Lys Pro Thr His Trp Asp Ser Glu Ser Ser Gly Asn Gly Ser
   705 710 715 720
   Leu Pro Ala Leu Val Gln Ala Tyr Val Leu Gln Gly Asp Pro Arg Glu
   725 730 735
   Ile Ser Asn Gln Ser Gln Pro Pro Ser Arg Thr Gly Asp Gln Val Leu
   740 745 750
   Tyr Gly Gln Val Leu Glu Ser Pro Thr Ser Pro Gly Val Met Gln Tyr
   755 760 765
   Ile Arg Ser Asp Ser Thr Gln Pro Leu Leu Gly Gly Pro Thr Pro Ser
   770 775 780
   Pro Lys Ser Tyr Glu Asn Ile Trp Phe His Ser Arg Pro Gln Glu Thr
   785 790 795 800
   Phe Val Pro Gln Pro Pro Asn Gln Glu Asp Asp Cys Val Phe Gly Pro
   805 810 815
   Pro Phe Asp Phe Pro Leu Phe Gln Gly Leu Gln Val His Gly Val Glu
   820 825 830
   Glu Gln Gly Gly Phe
   835
<210> 36
   <211> 112
   <212> PRT
   <213> Rattus rattus
<400> 36
   Leu Glu Gly Cys Gly Gln Ile Arg Ile Ser Pro Pro Ile Val His Leu
   1 5 10 15
   Gly Asp Pro Val Leu Ala Ser Cys Thr Ile Ser Pro Asn Cys Ser Lys
   20 25 30
   Leu Asp Arg Gln Pro Lys Ile Leu Trp Arg Leu Gln Asp Glu Pro Asn
   35 40 45
   Gln Pro Gly Asp Arg Gln His His Leu Pro Asp Gly Ser Gln Glu Ser
   50 55 60
   Ile Ile Thr Leu Pro His Leu Asn Tyr Thr Gln Ala Phe Leu Phe Cys
   65 70 75 80
   Leu Val Pro Trp Asn Asn Ser Phe Gln Val Leu Asp Gln Ala Glu Leu
   85 90 95
   Arg Ala Gly Cys Lys Ser Leu Gln Pro Pro Thr His Leu Leu Gln Cys
   100 105 110
<210> 37
   <211> 174
   <212> PRT
   <213> Homo sapiens
<400> 37
   Thr Pro Leu Gly Pro Ala Ser Ser Leu Pro Gln Ser Phe Leu Leu Lys
   1 5 10 15
   Cys Leu Glu Gln Val Arg Lys Ile Gln Gly Asp Gly Ala Ala Leu Gln
   20 25 30
   Glu Lys Leu Cys Ala Thr Tyr Lys Leu Cys His Pro Glu Glu Leu Val
   35 40 45
   Leu Leu Gly His Ser Leu Gly Ile Pro Trp Ala Pro Leu Ser Ser Cys
   50 55 60
   Pro Ser Gln Ala Leu Gln Leu Ala Gly Cys Leu Ser Gln Leu His Ser
   65 70 75 80
   Gly Leu Phe Leu Tyr Gln Gly Leu Leu Gln Ala Leu Glu Gly Ile Ser
   85 90 95
   Pro Glu Leu Gly Pro Thr Leu Asp Thr Leu Gln Leu Asp Val Ala Asp
   100 105 110
   Phe Ala Thr Thr Ile Trp Gln Gln Met Glu Glu Leu Gly Met Ala Pro
   115 120 125
   Ala Leu Gln Pro Thr Gln Gly Ala Met Pro Ala Phe Ala Ser Ala Phe
   130 135 140
   Gln Arg Arg Ala Gly Gly Val Leu Val Ala Ser His Leu Gln Ser Phe
   145 150 155 160
   Leu Glu Val Ser Tyr Arg Val Leu Arg His Leu Ala Gln Pro
   165 170
<210> 38
   <211> 175
   <212> PRT
   <213> Homo sapiens
<400> 38
   Met Thr Pro Leu Gly Pro Ala Ser Ser Leu Pro Gln Ser Phe Leu Leu
   1 5 10 15
   Lys Cys Leu Glu Gln Val Arg Lys Ile Gln Gly Asp Gly Ala Ala Leu
   20 25 30
   Gln Glu Lys Leu Cys Ala Thr Tyr Lys Leu Cys His Pro Glu Glu Leu
   35 40 45
   Val Leu Leu Gly His Ser Leu Gly Ile Pro Trp Ala Pro Leu Ser Ser
   50 55 60
   Cys Pro Ser Gln Ala Leu Gln Leu Ala Gly Cys Leu Ser Gln Leu His
   65 70 75 80
   Ser Gly Leu Phe Leu Tyr Gln Gly Leu Leu Gln Ala Leu Glu Gly Ile
   85 90 95
   Ser Pro Glu Leu Gly Pro Thr Leu Asp Thr Leu Gln Leu Asp Val Ala
   100 105 110
   Asp Phe Ala Thr Thr Ile Trp Gln Gln Met Glu Glu Leu Gly Met Ala
   115 120 125
   Pro Ala Leu Gln Pro Thr Gln Gly Ala Met Pro Ala Phe Ala Ser Ala
   130 135 140
   Phe Gln Arg Arg Ala Gly Gly Val Leu Val Ala Ser His Leu Gln Ser
   145 150 155 160
   Phe Leu Glu Val Ser Tyr Arg Val Leu Arg His Leu Ala Gln Pro
   165 170 175
<210> 39
   <211> 177
   <212> PRT
   <213> Homo sapiens
<400> 39
   Thr Pro Leu Gly Pro Ala Ser Ser Leu Pro Gln Ser Phe Leu Leu Lys
   1 5 10 15
   Cys Leu Glu Gln Val Arg Lys Ile Gln Gly Asp Gly Ala Ala Leu Gln
   20 25 30
   Glu Lys Leu Val Ser Glu Cys Ala Thr Tyr Lys Leu Cys His Pro Glu
   35 40 45
   Glu Leu Val Leu Leu Gly His Ser Leu Gly Ile Pro Trp Ala Pro Leu
   50 55 60
   Ser Ser Cys Pro Ser Gln Ala Leu Gln Leu Ala Gly Cys Leu Ser Gln
   65 70 75 80
   Leu His Ser Gly Leu Phe Leu Tyr Gln Gly Leu Leu Gln Ala Leu Glu
   85 90 95
   Gly Ile Ser Pro Glu Leu Gly Pro Thr Leu Asp Thr Leu Gln Leu Asp
   100 105 110
   Val Ala Asp Phe Ala Thr Thr Ile Trp Gln Gln Met Glu Glu Leu Gly
   115 120 125
   Met Ala Pro Ala Leu Gln Pro Thr Gln Gly Ala Met Pro Ala Phe Ala
   130 135 140
   Ser Ala Phe Gln Arg Arg Ala Gly Gly Val Leu Val Ala Ser His Leu
   145 150 155 160
   Gln Ser Phe Leu Glu Val Ser Tyr Arg Val Leu Arg His Leu Ala Gln
   165 170 175
   Pro
<210> 40
   <211> 1155
   <212> DNA
   <213> Rattus rattus
<400> 40
   atgagcatca ttcccctgcc tcagctcctc gccctgctct gctgctgcgg acttgctgct 60
   gctactcagg gccccacaga cccgtccacg ccccctaacc tgggcctcgc ccacttccac 120
   aacctgacct tcgaccccgg gacctggaca ctgagctggg cctgtggcgg ccatgatggg 180
   gcagtgatgt cgtgcacggt gattgaccag gaggcaggga tccggcgcag agtgcggtcc 240
   cggggctgcc gctgccggtt tcagccaatg gagttacacc gcggggtcga cctggaggtt 300
   gcgggggaca aaggccatgc ccaagtccat cagactctgc gcttcgagaa tgaaggtgcc 360
   ccaggctccg gggcagagaa cctgacctgt gagatccttg ctgcccactt cctgtgctgt 420
   tattgggcgg tggggccggc tgcacccgat gacatcagat actcactgcg cgtgctcaac 480
   gccactggtc atgaggtcgc cagctgctcc gctgcccccg gaaccccacc cacgcgttgc   540
   caggctgatg atctcacaca tctgccccgc ctcgcataca tcgtcgtcac tgggcagagc   600
   cggacggggc tggtgcggtt cctggatgcc gtggtcaaca ccaagggcat tgagcgcctg   660
   ggtcccccag ataacgtctc tgcctcctgt aacttctccc actgcaccat cacctgggct   720
   ccgcccccta cctgggcgcc tatgacggaa caggatttcc gctttgagat cgagtggaag   780
   aaggcggagc ccagcagcat tgcccagaag gtggttatcg cagggcgcga ggacaacgcc   840
   ttcgccttcc ccagccccgc cccccgtggc cgcctctggg tcagagttcg tgcaggggac   900
   acacgcagtg atcggtggag cgactggagc cccgccctgg agctcggctc ggaggccaca   960
   accccgccgc gggccctggt gttggcggcg tcgagctgtg cagccctgct gtgtgcgctg  1020
   gcactggggg cggcctgcag gagactcgcg ctctcacgcc gcctcctccc ccccatcccc  1080
   gggatccggg accgcgtatc tgatgacgag cgtgtcaact cggagacgct gaggaaggac  1140
   ctgctgcggc cctag                                                   1155
<210> 41
   <211> 384
   <212> PRT
   <213> Rattus rattus
<400> 41
   Met Ser Ile Ile Pro Leu Pro Gln Leu Leu Ala Leu Leu Cys Cys Cys
   1               5                   10                  15
   Gly Leu Ala Ala Ala Thr Gln Gly Pro Thr Asp Pro Ser Thr Pro Pro
               20                  25                  30
   Asn Leu Gly Leu Ala His Phe His Asn Leu Thr Phe Asp Pro Gly Thr
           35                  40                  45
   Trp Thr Leu Ser Trp Ala Cys Gly Gly His Asp Gly Ala Val Met Ser
       50                  55                  60
   Cys Thr Val Ile Asp Gln Glu Ala Gly Ile Arg Arg Arg Val Arg Ser
   65                  70                  75                  80
   Arg Gly Cys Arg Cys Arg Phe Gln Pro Met Glu Leu His Arg Gly Val
                   85                  90                  95
   Asp Leu Glu Val Ala Gly Asp Lys Gly His Ala Gln Val His Gln Thr
   100 105 110
   Leu Arg Phe Glu Asn Glu Gly Ala Pro Gly Ser Gly Ala Glu Asn Leu
   115 120 125
   Thr Cys Glu Ile Leu Ala Ala His Phe Leu Cys Cys Tyr Trp Ala Val
   130 135 140
   Gly Pro Ala Ala Pro Asp Asp Ile Arg Tyr Ser Leu Arg Val Leu Asn
   145 150 155 160
   Ala Thr Gly His Glu Val Ala Ser Cys Ser Ala Ala Pro Gly Thr Pro
   165 170 175
   Pro Thr Arg Cys Gln Ala Asp Asp Leu Thr His Leu Pro Arg Leu Ala
   180 185 190
   Tyr Ile Val Val Thr Gly Gln Ser Arg Thr Gly Leu Val Arg Phe Leu
   195 200 205
   Asp Ala Val Val Asn Thr Lys Gly Ile Glu Arg Leu Gly Pro Pro Asp
   210 215 220
   Asn Val Ser Ala Ser Cys Asn Phe Ser His Cys Thr Ile Thr Trp Ala
   225 230 235 240
   Pro Pro Pro Thr Trp Ala Pro Met Thr Glu Gln Asp Phe Arg Phe Glu
   245 250 255
   Ile Glu Trp Lys Lys Ala Glu Pro Ser Ser Ile Ala Gln Lys Val Val
   260 265 270
   Ile Ala Gly Arg Glu Asp Asn Ala Phe Ala Phe Pro Ser Pro Ala Pro
   275 280 285
   Arg Gly Arg Leu Trp Val Arg Val Arg Ala Gly Asp Thr Arg Ser Asp
   290 295 300
   Arg Trp Ser Asp Trp Ser Pro Ala Leu Glu Leu Gly Ser Glu Ala Thr
   305 310 315 320
   Thr Pro Pro Arg Ala Leu Val Leu Ala Ala Ser Ser Cys Ala Ala Leu
   325 330 335
   Leu Cys Ala Leu Ala Leu Gly Ala Ala Cys Arg Arg Leu Ala Leu Ser
   340 345 350
   Arg Arg Leu Leu Pro Pro Ile Pro Gly Ile Arg Asp Arg Val Ser Asp
   355 360 365
   Asp Glu Arg Val Asn Ser Glu Thr Leu Arg Lys Asp Leu Leu Arg Pro
   370 375 380
<210> 42
   <211> 21
   <212> DNA
   <213> Artificial Sequence
<220>
   <223> synthetic oligonucleotide
<400> 42
   ccattgtcca tcttggggat c 21
<210> 43
   <211> 21
   <212> DNA
   <213> Artificial Sequence
<220>
   <223> synthetic oligonucleotide
<400> 43
   cctggaagct gttgttccat g 21
<210> 44
   <211> 24
   <212> DNA
   <213> Artificial Sequence
<220>
   <223> synthetic oligonucleotide
<400> 44
   cacagcgggc tcttcctcta ccaa 24
<210> 45
   <211> 24
   <212> DNA
   <213> Artificial Sequence
<220>
   <223> synthetic oligonucleotide
<400> 45
   agcagcggca ggaatcaata ctcg 24
<210> 46
   <211> 20
   <212> DNA
   <213> Artificial Sequence
<220>
   <223> synthetic oligonucleotide
<400> 46
   accccaccgt gttcttcgac 20
<210> 47
   <211> 20
   <212> DNA
   <213> Artificial Sequence
<220>
   <223> synthetic oligonucleotide
<400> 47
   catttgccat ggacaagatg 20
<210> 48
   <211> 20
   <212> DNA
   <213> Artificial Sequence
<220>
   <223> synthetic oligonucleotide
<400> 48
   aggaagaagc tgcagcagag 20
<210> 49
   <211> 20
   <212> DNA
   <213> Artificial Sequence
<220>
   <223> synthetic oligonucleotide
<400> 49
   ttcacctgct tgggctctat 20
<210> 50
   <211> 20
   <212> DNA
   <213> Artificial Sequence
<220>
   <223> synthetic oligonucleotide
<400> 50
   ggcaaggatg ccactaatgt 20
<210> 51
   <211> 20
   <212> DNA
   <213> Artificial Sequence
<220>
   <223> synthetic oligonucleotide
<400> 51
   agggtcagca ggagacttga 20
<210> 52
   <211> 23
   <212> DNA
   <213> Artificial Sequence
<220>
   <223> synthetic oligonucleotide
<400> 52
   ccacctacgg ggacctcaac cac 23
<210> 53
   <211> 21
   <212> DNA
   <213> Artificial Sequence
<220>
   <223> synthetic oligonucleotide
<400> 53
   gacatgcgcc cacggaagac g 21
<210> 54
   <211> 21
   <212> DNA
   <213> Artificial Sequence
<220>
   <223> synthetic oligonucleotide
<400> 54
   tcattctttg gagcgggtgt g 21
<210> 55
   <211> 24
   <212> DNA
   <213> Artificial Sequence
<220>
   <223> synthetic oligonucleotide
<400> 55
   taaggacggc aaagttgtaa gtgg 24
<210> 56
   <211> 22
   <212> DNA
   <213> Artificial Sequence
<220>
   <223> synthetic oligonucleotide
<400> 56
   cctttcttat gcatgtacgg ag 22
<210> 57
   <211> 22
   <212> DNA
   <213> Artificial Sequence
<220>
   <223> synthetic oligonucleotide
<400> 57
   gtacactaat acgaaggcac tc 22
<210> 58
   <211> 20
   <212> DNA
   <213> Artificial Sequence
<220>
   <223> synthetic oligonucleotide
<400> 58
   accccaccgt gttcttcgac 20
<210>. 59
   <211> 20
   <212> DNA
   <213> Artificial Sequence
<220>
   <223> synthetic oligonucleotide
<400> 59
   catttgccat ggacaagatg 20
<210> 60
   <211> 22
   <212> DNA
   <213> Artificial Sequence
<220>
   <223> synthetic oligonucleotide
<400> 60
   acgtcgttgg ctcagttatg tc 22
<210> 61
   <211> 24
   <212> DNA
   <213> Artificial Sequence
<220>
   <223> synthetic oligonucleotide
<400> 61
   atttatgtca gagatggagg atgg 24
<210> 62
   <211> 21
   <212> DNA
   <213> artificial Sequence
<220>
   <223> synthetic oligonucleotide
<400> 62
   ggagctctaa gcttctagat c 21
<210> 63
   <211> 21
   <212> DNA
   <213> Artificial Sequence
<220>
   <223> synthetic oligonucleotide
<400> 63
   ggctcaatgt gatttcttgg g 21
<210> 64
   <211> 175
   <212> PRT
   <213> Homo sapiens
<400> 64
   Met Thr Pro Leu Gly Pro Ala Ser Ser Leu Pro Gln Ser Phe Leu Leu
   1 5 10 15
   Lys Cys Leu Glu Gln Val Arg Lys Ile Gln Gly Asp Gly Ala Ala Leu
   20 25 30
   Gln Glu Lys Leu Cys Ala Thr Tyr Lys Leu Cys His Pro Glu Glu Leu
   35 40 45
   Val Leu Leu Gly His Ser Leu Gly Ile Pro Trp Ala Pro Leu Ser Ser
   50 55 60
   Cys Pro Ser Gln Ala Leu Gln Leu Ala Gly Cys Leu Ser Gln Leu His
   65 70 75 80
   Ser Gly Leu Phe Leu Tyr Gln Gly Leu Leu Gln Ala Leu Glu Gly Ile
   85 90 95
   Ser Pro Glu Leu Gly Pro Thr Leu Asp Thr Leu Gln Leu Asp Val Ala
   100 105 110
   Asp Phe Ala Thr Thr Ile Trp Gln Gln Met Glu Glu Leu Gly Met Ala
   115 120 125
   Pro Ala Leu Gln Pro Thr Gln Gly Ala Met Pro Ala Phe Ala Ser Ala
   130 135 140
   Phe Gln Arg Arg Ala Gly Gly Val Leu Val Ala Ser His Leu Gln Ser
   145 150 155 160
   Phe Leu Glu Val Ser Tyr Arg Val Leu Arg His Leu Ala Gln Pro
   165 170 175
<210> 65
   <211> 174
   <212> PRT
   <213> Homo sapiens
<400> 65
   Ala Pro Thr Thr Arg Ala Ser Ser Leu Pro Gln Ser Phe Leu Leu Lys
   1 5 10 15
   Ser Leu Glu Gln Val Arg Lys Ile Gln Gly Asp Gly Ala Ala Leu Gln
   20 25 30
   Glu Lys Leu Cys Ala Thr Tyr Lys Leu Cys His Pro Glu Glu Leu Val
           35                  40                  45
   Leu Leu Gly His Ser Leu Gly Ile Pro Trp Ala Pro Leu Ser Ser Cys
       50                  55                  60
   Pro Ser Gln Ala Leu Gln Leu Ala Gly Cys Leu Ser Gln Leu His Ser
   65                  70                  75                  80
   Gly Leu Phe Leu Tyr Gln Gly Leu Leu Gln Ala Leu Glu Gly Ile Ser
                   85                  90                  95
   Pro Glu Leu Gly Pro Thr Leu Asp Thr Leu Gln Leu Asp Val Ala Asp
               100                 105                 110
   Phe Ala Thr Thr Ile Trp Gln Gln Met Glu Glu Leu Gly Met Ala Pro
           115                 120                 125
   Ala Leu Gln Pro Thr Gln Gly Ala Met Pro Ala Phe Ala Ser Ala Phe
       130                 135                 140
   Gln Arg Arg Ala Gly Gly Val Leu Val Ala Ser His Leu Gln Ser Phe
   145                 150                 155                 160
   Leu Glu Val Ser Tyr Arg Val Leu Arg His Leu Ala Gln Pro
                   165                 170